# EUROPEAN PATENT SPECIFICATION

(11) **EP 2 046 950 B1**
(45) Date of publication and mention of the grant of the patent: **04.06.2014**
(21) Application number: 07805007.7
(22) Date of filing: 25.06.2007
(51) Int. Cl.: C12N 9/22, C12N 15/55, A61K 48/00, C12N 15/85, C12N 15/82, C12N 15/63, G01N 33/48, C12N 5/10, A01K 67/027, C12N 15/09

(54) **Meganuclease variants cleaving a DNA target sequence from a RAG1 gene and uses thereof**
DNA-Zielsequenz aus einem RAG1-Gen spaltende Meganukleasevarianten und ihre Verwendung
Variants de méganucléases clivant une séquence d'ADN cible d'un gène RAG1 et leurs utilisations

(30) Priority: 18.07.2006 WO PCT/IB2006/002816
(43) Date of publication of application: 15.04.2009
(73) Proprietor: CELLECTIS, 93230 Romainville (FR)
(72) Inventor: ARNOULD, Sylvain, 93200 Saint-Denis (FR); GRIZOT, Sylvestre, 92250 La Garenne Colombes (FR)
(74) Representative: Leblois-Préhaud, Hélène Marthe Georgette
(86) International application number: PCT/IB2007/002891
(87) International publication number: WO 2008/010093

(56) References cited:
- WO-A-03/078619
- WO-A-2004/067753
- WO-A-2006/097784
- WO-A-2006/097853
- ARNOULD ET AL: "Engineering of Large Numbers of Highly Specific Homing Endonucleases that Induce Recombination on Novel DNA Targets" JOURNAL OF MOLECULAR BIOLOGY, LONDON, GB, vol. 355, no. 3, 20 January 2006 (2006-01-20), pages 443-458, XP005206991 ISSN: 0022-2836 cited in the application
- CHEVALIER B ET AL: "Flexible DNA Target Site Recognition by Divergent Homing Endonuclease Isoschizomers I-CreI and I-MsoI" JOURNAL OF MOLECULAR BIOLOGY, LONDON, GB, vol. 329, no. 2, 30 May 2003 (2003-05-30), pages 253-269, XP004454255 ISSN: 0022-2836 cited in the application
- SELIGMAN L M ET AL: "Mutations altering the cleavage specificity of a homing endonuclease" NUCLEIC ACIDS RESEARCH, OXFORD UNIVERSITY PRESS, SURREY, GB, vol. 30, no. 17, 1 September 2002 (2002-09-01), pages 3870-3879, XP002282592 ISSN: 0305-1048
- AGGARWAL A K ET AL: "Novel site-specific DNA endonucleases" CURRENT OPINION IN STRUCTURAL BIOLOGY, CURRENT BIOLOGY LTD., LONDON, GB, vol. 8, 1998, pages 19-25, XP002236854 ISSN: 0959-440X
- CHAMES P. ET AL: "In vivo selection of engineered homing endonucleases using double-strand break induced homologous recombination" NUCLEIC ACIDS RESEARCH, OXFORD UNIVERSITY PRESS, SURREY, GB, vol. 33, no. 20, 2005, XP002378874 ISSN: 0305-1048
- GOUBLE AGNÈS ET AL: "Efficient in toto targeted recombination in mouse liver by meganuclease-induced double-strand break." THE JOURNAL OF GENE MEDICINE MAY 2006, vol. 8, no. 5, May 2006 (2006-05), pages 616-622, XP002427714 ISSN: 1099-498X
- ROSEN LAURA E ET AL: "Homing endonuclease I-CreI derivatives with novel DNA target specificities." NUCLEIC ACIDS RESEARCH 2006, vol. 34, no. 17, 2006, pages 4791-4800, XP002427715 ISSN: 1362-4962

## Description

The invention relates to a meganuclease variant cleaving a DNA target sequence from a RAG gene, to a vector encoding said variant, to a cell, an animal or a plant modified by said vector and to the use of said meganuclease variant and derived products for genome therapy, *in vivo* and *ex* vivo (gene cell therapy), and genome engineering.

Severe Immune Combined Deficiency (SCID) results from a defect in lymphocytes T maturation, always associated with a functional defect in lymphocytes B (Cavazzana-Calvo et al., Annu. Rev. Med., 2005, 56, 585-602; Fischer et al., Immunol. Rev., 2005, 203, 98-109). Overall incidence is estimated to 1 in 75 000 births. Patients with untreated SCID are subject to multiple opportunist microorganism infections, and do generally not live beyond one year. SCID can be treated by allogenic hematopoietic stem cell transfer, from a familial donor. Histocompatibility with the donor can vary widely. In the case of Adenosine Deaminase (ADA) deficiency, one of the SCID forms, patients can be treated by injection of recombinant Adenosine Deaminase enzyme.

Since the ADA gene has been shown to be mutated in SCID patients (Giblett et al., Lancet, 1972, 2, 1067-1069), several other genes involved in SCID have been identified (Cavazzana-Calvo et al., Annu. Rev. Med., 2005, 56, 585-602; Fischer et al., Immunol. Rev., 2005, 203, 98-109). There are four major causes for SCID: (i) mutation in the ADA gene results in a defect in purine metabolism that is lethal for lymphocyte precursors, which in turn results in the absence of B, T and NK cells. (ii) The most frequent form of SCID, SCID-X1, is caused by mutation in the gene coding for γC (Noguchi, et al., Cell, 1993, 73, 147-157), a component of the T, B and NK cells cytokine receptor. This receptor activates several targets through the JAK3 kinase (Macchi et al., Nature, 1995, 377, 65-68), which inactivation results in the same syndrome as γC inactivation. (iii) Defective V(D)J recombination is an essential step in the maturation of immunoglobulins and T lymphocytes receptors (TCRs). Mutations in Recombination Activating Gene 1 and 2 (RAG1 and RAG2) and Artemis, three genes involved in this process, result in the absence of T and B lymphocytes. RAG1 and RAG2, are two proteins responsible for the initiation of V(D)J recombination (Schatz et al., Cell, 1989, 59, 1035-1048; Oettinger et al., Science, 1990, 248, 1517-1523). These proteins bind recombination sequences (RS) adjacent to the V, D and J coding segments in the immunoglobulin and TCR loci, and catalyze a complex cleavage reaction. The outcome of the cleavage is DNA double strand break (DSB) occurring between the RS and the coding segment, with a blunt end on one side of the break (the side of the RS), and a hairpin on the other side (Dudley et al., Adv. Immunol., 2005, 86, 43-112). This hairpin is cleaved by the Artemis protein, and then processed by Non-Homologous End Joining (NHEJ) factors such as Lig4 and XRCC4. In addition to the absence of B and T cells, mutations in the Artemis gene are also associated with an increased cellular radiosensitivity (Moshous et al., Cell, 2001, 105, 177-186). This particular phenotype, called RS-SCID is probably due to a role of Artemis in both immunoglobulin maturation and DNA maintenance. (iv) Mutations in other genes such as CD45, involved in T cell specific signalling have also been reported, although they represent a minority of cases (Cavazzana-Calvo et al., Annu. Rev. Med., 2005, 56, 585-602; Fischer et al., Immunol. Rev., 2005, 203, 98-109).

Since when their genetic bases have been identified, the different SCID forms have become a paradigm for gene therapy approaches (Fischer et al., Immunol. Rev., 2005, 203, 98-109) for two major reasons.

First, as in all blood diseases, an *ex vivo* treatment can be envisioned. Hematopoietic Stem Cells (HSCs) can be recovered from bone marrow, and keep their pluripotent properties for a few cell divisions. Therefore, they can be treated in *vitro,* and then reinjected into the patient, where they repopulate the bone marrow.

Second, since the maturation of T and B cells and precursors is impaired in SCID patients, corrected cells have a selective advantage. Therefore, a small number of corrected cells can restore a functional immune system. This hypothesis was validated several times by (i) the partial restoration of immune functions associated with the reversion of mutations in SCID patients (Hirschhorn et al., Nat. Genet., 1996, 13, 290-295; Stephan et al., N. Engl. J. Med., 1996, 335, 1563-1567; Bousso et al., Proc. Natl., Acad. Sci. USA, 2000, 97, 274-278; Wada et al., Proc. Natl. Acad. Sci. USA, 2001, 98, 8697-8702; Nishikomori et al., Blood, 2004, 103, 4565-4572), (ii) the correction of SCID-X1 deficiencies *in vitro* in hematopoietic cells (Candotti et al., Blood, 1996, 87, 3097-3102; Cavazzana-Calvo et al., Blood, 1996, Blood, 88, 3901-3909; Taylor et al., Blood, 1996, 87, 3103-3107; Hacein-Bey et al., Blood, 1998, 92, 4090-4097), (iii) the correction of SCID-X1 (Soudais et al., Blood, 2000, 95, 3071-3077; Tsai et al., Blood, 2002, 100, 72-79), JAK-3 (Bunting et al., Nat. Med., 1998, 4, 58-64; Bunting et al., Hum. Gene Ther., 2000, 11,2353-2364) and RAG2 (Yates et al., Blood, 2002, 100, 3942-3949) deficiencies *in vivo* in animal models and (iv) by the result of gene therapy clinical trials (Cavazzana-Calvo et al., Science, 2000, 288, 669-672; Aiuti et al., Nat. Med., 2002, 8, 423-425; Gaspar et al., Lancet, 2004, 364, 2181-2187).

Since the nineties, several gene therapy clinical trials have generated a large body of very useful information. These studies are all based on the complementation of the mutated gene with a functional gene introduced into the genome with a viral vector. Clinical trial for SCID-X1 (γC deficiency) resulted in the restoration of a functional immune system in nine out of ten patients treated by gene therapy (Cavazzana-Calvo et al., Science, 2000, 288, 669-672). Other successful clinical trials were conducted with four SCID-X1 patients (Gaspar et al., Lancet, 2004, 364, 2181-2187) and four ADA patients (Aiuti et al., Science, 2002, 296, 2410-2413), confirming the benefits of the gene therapy approach. However, the first trials have also illustrated the risks associated with this approach. Later, three patients developed a monoclonal lymphoproliferation, closely mimicking acute leukemia. These lymphoproliferations are associated with the activation of cellular oncogenes by insertional mutagenesis. In all three cases, proliferating cells are characterized by the insertion of the retroviral vector in the same locus, resulting in overexpression of the LMO2 gene (Hacein-Bey et al., Science, 2003, 302, 415-419; Fischer et al., N. Engl. J. Med., 2004, 350, 2526-2527).

Thus, these results have demonstrated both the extraordinary potential of a «genomic therapy » in the treatment of inherited diseases, and the limits of the integrative retroviral vectors (Kohn et al., Nat. Rev. Cancer, 2003, 3, 477-488). Despite the development of novel electroporation methods (Nucleofector® technology from AMAXA GmbH; PCT/EP01/07348, PCT/DE02/01489 and PCT/DE02/01483), viral vectors have so far given the most promising results in HSCs. Retrovirus derived from the MoMLV (Moloney Murine Leukemia Virus) have been used to transduce HSCs efficiently, including for clinical trials (see above). However, classical retroviral vectors transduce only cycling cells, and transduction of HSCs with Moloney vectors requires their stimulation and the induction of mitosis with growth factors, thus strongly compromising their pluripotent properties *ex* vivo. In contrast, lentiviral vectors derived from HIV-1, can efficiently transduce non mitotic cells, and are perfectly adapted to HSCs transduction (Logan et al., Curr. Opin. Biotechnol., 2002, 13, 429-436). With such vectors, the insertion of flap DNA strongly stimulate entry into the nucleus, and thereby the rate of HSC transduction (Sirven et al., Blood, 2000, 96, 4103-4110; Zennou et al., Cell, 2000, 101, 173-185). However, lentivirial vectors are also integrative, with same potential risks as Moloney vectors: following insertion into the genome, the virus LTRs promoters and enhancers can stimulate the expression of adjacent genes (see above). Deletion of enhancer and promoter of the U3 region from LTR3' can be an option. After retrotranscription, this deletion will be duplicated into the LTR5', and these vectors, called «delta U3» or «Self Inactivating», can circumvent the risks of insertional mutagenesis resulting from the activation of adjacent genes. However, they do not abolish the risks of gene inactivation by insertion, or of transcription readthrough.

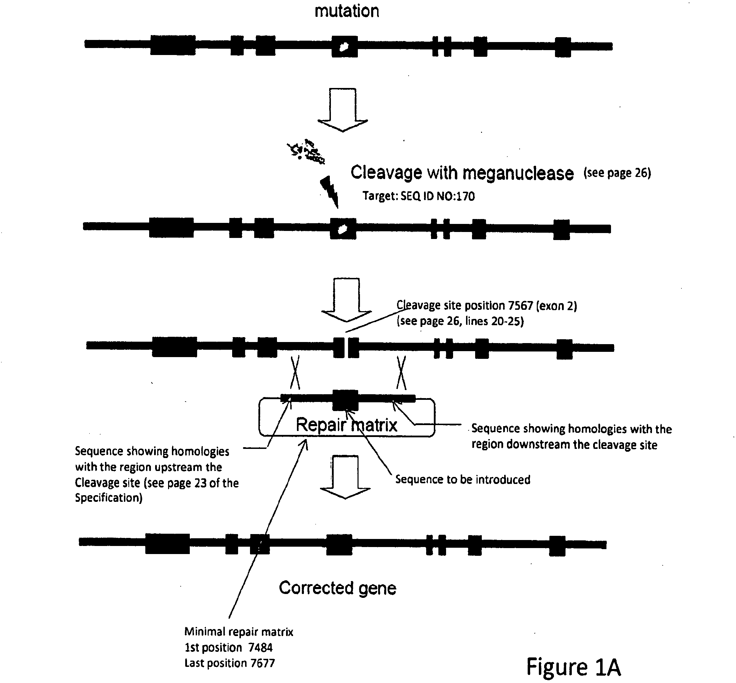

Targeted homologous recombination is another alternative that should bypass the problems raised by current approaches. Current gene therapy strategies are based on a complementation approach, wherein randomly inserted but functional extra copy of the gene provide for the function of the mutated endogenous copy. In contrast, homologous recombination should allow for the precise correction of mutations *in situ* (Figure 1A).

Homologous gene targeting strategies have been used to knock out endogenous genes (Capecchi, M. R., Science, 1989, 244, 1288-1292; Smithies, O., Nat. Med., 2001, 7, 1083-1086) or knock-in exogenous sequences in the chromosome. It can as well be used for gene correction, and in principle, for the correction of mutations linked with monogenic diseases. However, this application is in fact difficult, due to the low efficiency of the process (10⁻⁶ to 10⁻⁹ of transfected cells). In the last decade, several methods have been developed to enhance this yield. For example, chimeraplasty (De Semir et al. J. Gene Med., 2003, 5, 625-639) and Small Fragment Homologous Replacement (Goncz et al., Gene Ther, 2001, 8, 961-965; Bruscia et al., Gene Ther., 2002, 9, 683-685; Sangiuolo et al., BMC Med. Genet., 2002, 3, 8; De Semir, D. and J.M. Aran, Oligonucleotides, 2003, 13, 261-269) have both been used to try to correct CFTR mutations with various levels of success.

Another strategy to enhance the efficiency of recombination is to deliver a DNA double-strand break (DSB) in the targeted locus, using meganucleases. Meganucleases are by definition sequence-specific endonucleases recognizing large sequences (Chevalier, B.S. and B.L. Stoddard, Nucleic Acids Res., 2001, 29, 3757-3774). They can cleave unique sites in living cells, thereby enhancing gene targeting by 1000-fold or more in the vicinity of the cleavage site (Puchta et al., Nucleic Acids Res., 1993, 21, 5034-5040; Rouet et al., Mol. Cell. Biol., 1994, 14, 8096-8106; Choulika et al., Mol. Cell. Biol., 1995, 15, 1968-1973; Puchta et al., Proc. Natl. Acad. Sci. USA, 1996, 93, 5055-5060 ; Sargent et al., Mol. Cell. Biol., 1997, 17, 267-77; Donoho et al., Mol. Cell. Biol, 1998, 18, 4070-4078; Elliott et al., Mol. Cell. Biol., 1998, 18, 93-101; Cohen-Tannoudji et al., Mol. Cell. Biol., 1998, 18, 1444-1448). Such meganucleases could be used to correct mutation responsible for monogenic inherited diseases, such as SCID.

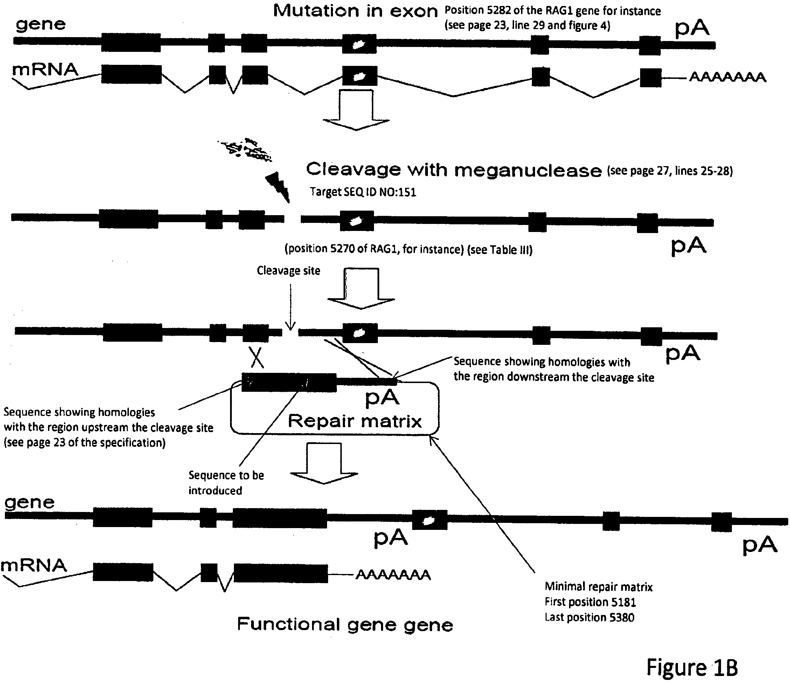

The most accurate way to correct a genetic defect is to use a repair matrix with a non mutated copy of the gene, resulting in a reversion of the mutation (Figure 1A). However, the efficiency of gene correction decreases as the distance between the mutation and the DSB grows, with a five-fold decrease by 200 bp of distance. Therefore, a given meganuclease can be used to correct only mutations in the vicinity of its DNA target. An alternative, termed "exon knock-in" is featured in Figure 1B. In this case, a meganuclease cleaving in the 5' part of the gene can be used to knock-in functional exonic sequences upstream of the deleterious mutation. Although this method places the transgene in its regular location, it also results in exons duplication, which impact on the long range remains to be evaluated. In addition, should naturally cis-acting elements be placed in an intron downstream of the cleavage, their immediate environment would be modified and their proper function would also need to be explored. However, this method has a tremendous advantage: a single meganuclease could be used for many different patients.

However, the use of this technology is limited by the repertoire of natural meganucleases. For example, there is no cleavage site for a known natural meganuclease in human *SCID* genes. Therefore, the making of meganucleases with tailored specificities is under intense investigation and several laboratories have tried to alter the specificity of natural meganucleases or to make artificial endonuclease.

Recently, fusion of Zinc-Finger Proteins with the catalytic domain of the *Fok*I, a class IIS restriction endonuclease, were used to make functional sequence-specific endonucleases (Smith et al., Nucleic Acids Res., 1999, 27, 674-681; Bibikova et al., Mol. Cell. Biol., 2001, 21, 289-297; Bibikova et al., Genetics, 2002, 161, 1169-1175; Bibikova et al., Science, 2003, 300, 764-; Porteus, M.H. and D. Baltimore, Science, 2003, 300, 763-; Alwin et al., Mol. Ther., 2005, 12, 610-617; Urnov et al., Nature, 2005, 435, 646-651; Porteus, M.H., Mol. Ther., 2006, 13, 438-446). Such nucleases were recently used for the engineering of the ILR2G gene in human cells from the lymphoid lineage (Urnov et al., Nature, 2005, 435, 646-651).

The Cys2-His2 type Zinc-Finger Proteins (ZFP), represent a simple and modular system that is easy to manipulate since the ZFP specificity is driven by essentially four residues per finger (Pabo et al., Annu. Rev. Biochem., 2001, 70, 313-340; Jamieson et al., Nat. Rev. Drug Discov., 2003, 2, 361-368). Studies from the Pabo (Rebar, E.J. and C.O. Pabo, Science, 1994, 263, 671-673; Kim J.S. and C.O. Pabo, Proc. Natl. Acad. Sci USA, 1998, 95, 2812-2817), Klug (Choo, Y. and A. Klug, Proc. Natl. Acad. Sci. USA, 1994, 91, 11163-11167; Isalan et al., Nat. Biotechnol., 2001, 19, 656-660) and Barbas (Choo, Y. and A. Klug, Proc. Natl. Acad. Sci. USA, 1994, 91, 11163-11167; Isalan et al., Nat. Biotechnol., 2001, 19, 656-660) laboratories resulted in a large repertoire of novel artificial ZFP, able to bind most G/ANNG/ANNG/ANN sequences.

Nevertheless, ZFP might have their limitations, especially for applications requiring a very high level of specificity, such as therapeutic applications. It was recently shown that *Fok*I nuclease activity in fusion acts with either one recognition site or with two sites separated by varied distances via a DNA loop including in the presence of some DNA-binding defective mutants of *Fok*I (Catto et al., Nucleic Acids Res., 2006, 34, 1711-1720). Thus, specificity might be very degenerate, as illustrated by toxicity in mammalian cells (Porteus, M.H. and D. Baltimore, Science, 2003, 300, 763-) and Drosophila (Bibikova et al., Genetics, 2002, 161, 1169-1175; Bibikova et al., Science, 2003, 300, 764).

In the wild, meganucleases are essentially represented by Homing Endonucleases (HEs). Homing Endonucleases are a widespread family of natural meganucleases including hundreds of proteins families (Chevalier B.S. and B.L. Stoddard, Nucleic Acids Res., 2001, 29, 3757-3774). These proteins are encoded by mobile genetic elements which propagate by a process called "homing": the endonuclease cleaves a cognate allele from which the mobile element is absent, thereby stimulating a homologous recombination event that duplicates the mobile DNA into the recipient locus. Given their exceptional cleavage properties in terms of efficacy and specificity, they could represent ideal scaffold to derive novel, highly specific endonucleases.

HEs belong to four major families. The LAGLIDADG family, named after a conserved peptidic motif involved in the catalytic center, is the most widespread and the best characterized group. Seven structures are now available. Whereas most proteins from this family are monomeric and display two LAGLIDADG motifs, a few ones have only one motif, but dimerize to cleave palindromic or pseudo-palidromic target sequences.

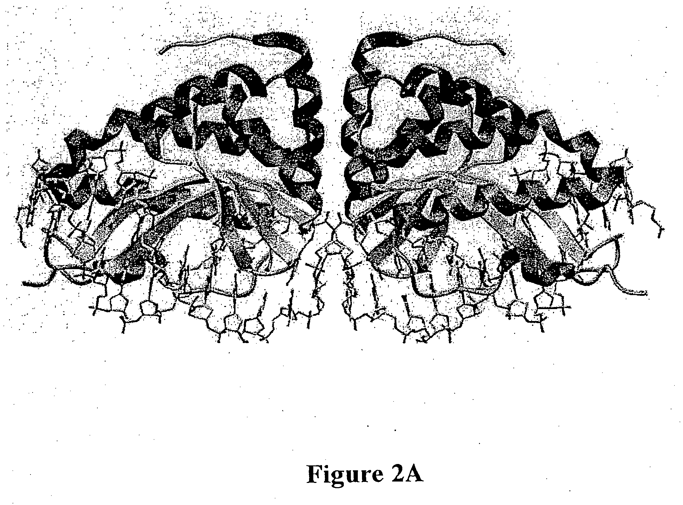

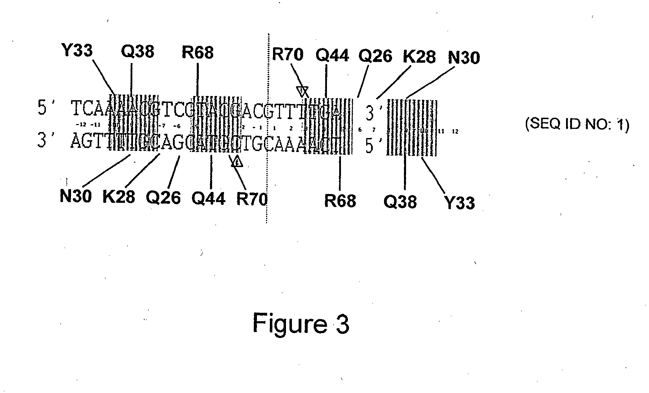

Although the LAGLIDADG peptide is the only conserved region among members of the family, these proteins share a very similar architecture (Figure 2A). The catalytic core is flanked by two DNA-binding domains with a perfect twofold symmetry for homodimers such as I-*Cre*I (Chevalier et al., Nat. Struct. Biol., 2001, 8, 312-316) and I*-Mso*I (Chevalier et al., J. Mol. Biol., 2003, 329, 253-269), and with a pseudo symmetry for monomers such as I-*Sce*I (Moure et al., J. Mol. Biol., 2003, 334, 685-695), I-*Dmo*I (Silva et al., J. Mol. Biol., 1999, 286, 1123-1136) or I-*Ani*I (Bolduc et al., Genes Dev., 2003, 17, 2875-2888). Both monomers, or both domains (for monomeric proteins) contribute to the catalytic core, organized around divalent cations. Just above the catalytic core, the two LAGLIDADG peptides play also an essential role in the dimerization interface. DNA binding depends on two typical saddle-shaped ββαββ folds, sitting on the DNA major groove (Figure 2A). Analysis of I-*Cre*I structure bound to its natural target shows that in each monomer, eight residues (Y33, Q38, N30, K28, Q26, Q44, R68 and R70) establish direct interactions with seven bases at positions ± 3, 4, 5, 6, 7, 9 and 10 (Figure 3). In addition, some residues establish water-mediated contact with several bases; for example S40 and N30 with the base pair at position +8 and -8 (Chevalier et al., 2003, precited). Other domains can be found, for example in inteins such as PI-*Pfu*I (Ichiyanagi et al., J. Mol. Biol., 2000, 300, 889-901) and PI-*Sce*I (Moure et al., Nat. Struct. Biol., 2002, 9, 764-770), which protein splicing domain is also involved in DNA binding.

The making of functional chimeric meganucleases has demonstrasted the plasticity of LAGLIDADG proteins. New meganucleases could be obtained by swapping LAGLIDADG Homing Endonuclease Core Domains of different monomers (Epinat et al., Nucleic Acids Res., 2003, 31, 2952-62; Chevalier et al., Mol. Cell., 2002, 10, 895-905; Steuer et al., Chembiochem., 2004, 5, 206-13; International PCT Applications WO 03/078619 and WO 2004/031346). These single-chain chimeric meganucleases wherein the two LAGLIDADG Homing Endonuclease Core Domains from different meganucleases are linked by a spacer, are able to cleave the hybrid target corresponding to the fusion of the two half parent DNA target sequences.

Besides different groups have used a rational approach to locally alter the specificity of the I-*Cre*I, I-*Sce*I, I*-Mso*I and PI-*Sce*I HEs (Sussman et al., J. Mol. Biol., 2004, 342, 31-41; Seligman et al., Genetics, 1997, 147, 1653-1664; Arnould et al., J. Mol. Biol., 2006, 355, 443-458; Doyon et al., J. Am. Chem. Soc., 2006, 128, 2477-2484; Ashworth et al., Nature, 2006, 441, 656-659; Gimble et al., J. Mol. Biol., 2003, 334, 993-1008).

The construction of chimeric and single chain artificial HEs has suggested that a combinatorial approach could be used to obtain novel meganucleases cleaving novel (non-palindromic) target sequences: different monomers or core domains could be fused in a single protein, to achieve novel specificities. These results mean that the two DNA binding domains of an I-*Cre*I dimer behave independently; each DNA binding domain binds a different half of the DNA target site.

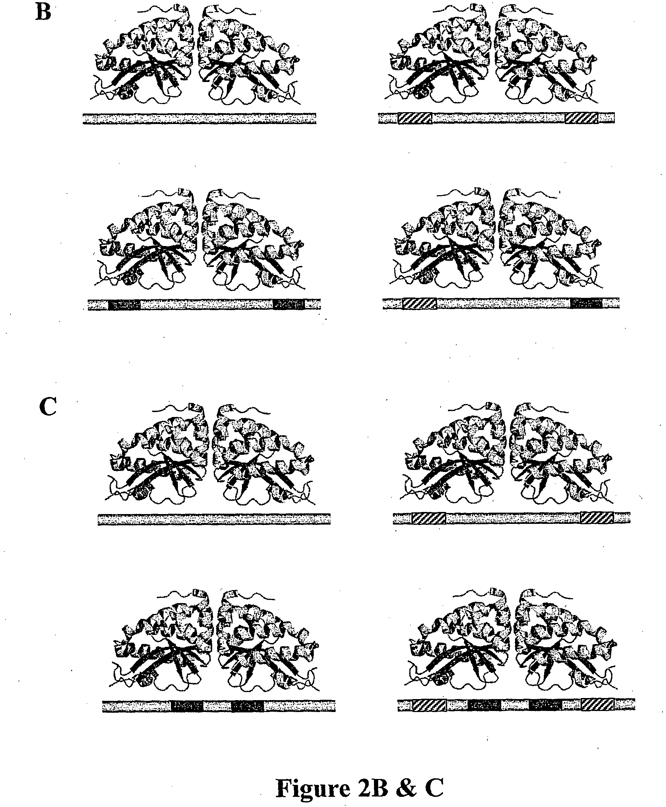

Combining the semi-ration approach and High Throughput Screening (HTS), Arnould *et al.* could derive hundreds of I-*Cre*I derivatives with altered specificity (Arnould et al., J. Mol. Biol., 2006, 355, 443-458). Residues Q44, R68 and R70 of I-*Cre*I were mutagenized, and a collection of variants with altered specificity in positions ± 3 to 5 were identified by screening. Then, two different variants were combined and assembled in a functional heterodimeric endonuclease able to cleave a chimeric target resulting from the fusion of a different half of each variant DNA target sequence. Interestingly, the novel proteins had kept proper folding and stability, high activity, and a narrow specificity. Therefore, a two step strategy may be used to tailor the specificity of a natural LAGLIDADG meganuclease. The first step is to locally mutagenize a natural LAGLIDADG meganuclease such as I-*Cre*I and to identify collections of variants with altered specificity by screening. The second step is to rely on the modularity of these proteins, and use a combinatorial approach to make novel meganucleases, that cleave the site of choice (Figure 2B).

The generation of collections of novel meganucleases, and the ability to combine them by assembling two different monomers/core domains considerably enriches the number of DNA sequences that can be targeted, but does not yet saturate all potential sequences.

To reach a larger number of sequences, it would be extremely valuable to be able to identify smaller independent subdomains that could be combined (Figure 2C).

However, a combinatorial approach is much more difficult to apply within a single monomer or domain than between monomers since the structure of the binding interface is very compact and the two different ββ hairpins which are responsible for virtually all base-specific interactions do not constitute separate subdomains, but are part of a single fold. For example, in the internal part of the DNA binding regions of I-*Cre*I, the gtc triplet is bound by one residue from the first hairpin (Q44), and two residues from the second hairpin (R68 and R70; see Figure 1B of Chevalier et al., 2003, precited).

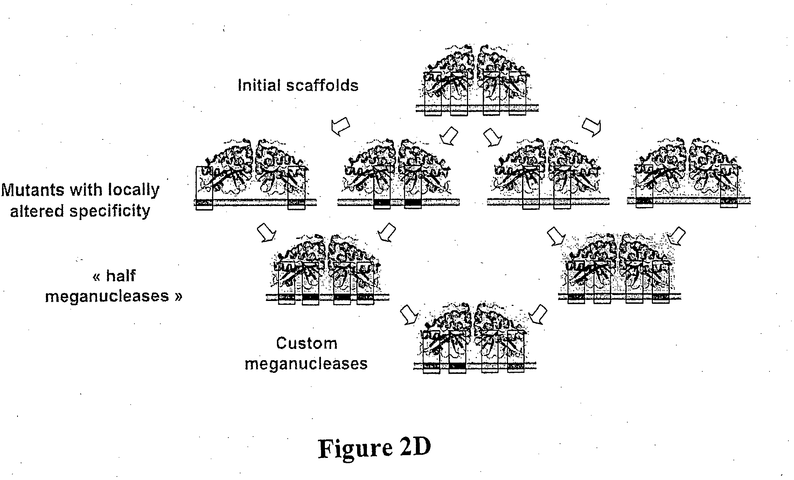

In spite of this lack of apparent modularity at the structural level, the Inventors have identified separable functional subdomains, able to bind distinct parts of a homing endonuclease half-site. By assembling two subdomains from different monomers or core domains within the same monomer, the Inventors have engineered functional homing endonuclease (homodimeric) variants, which are able to cleave palindromic chimeric targets (Figure 2C). Furthermore, a larger combinatorial approach is allowed by assembling four different subdomains to form new heterodimeric molecules which are able to cleave non-palindromic chimeric targets (Figure 2D). The different subdomains can be modified separately and combine in one meganuclease variant (heterodimer or single-chain molecule) which is able to cleave a target from a gene of interest.

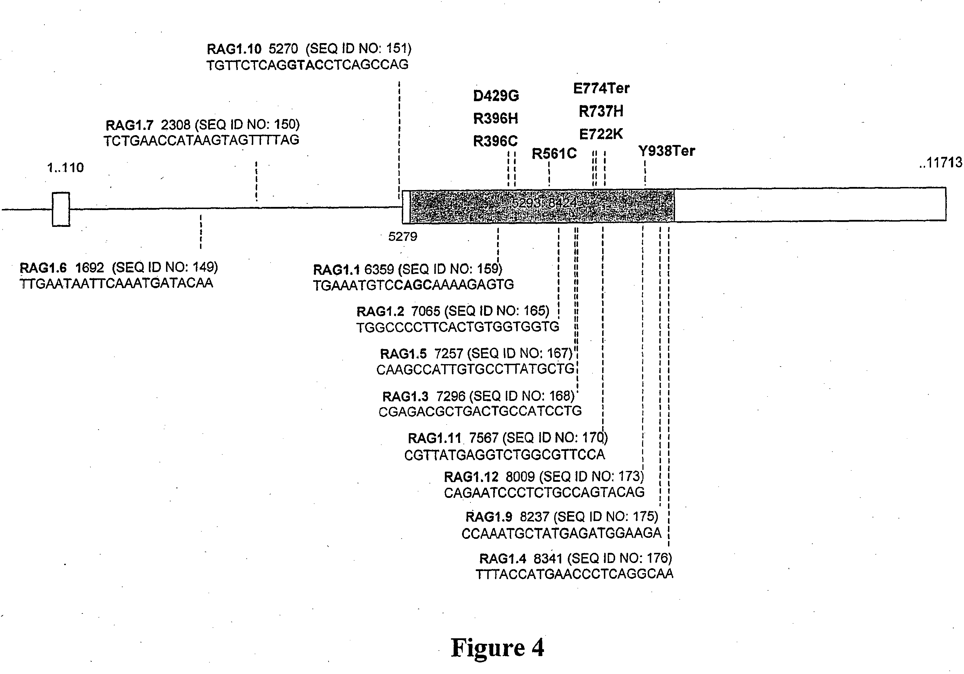

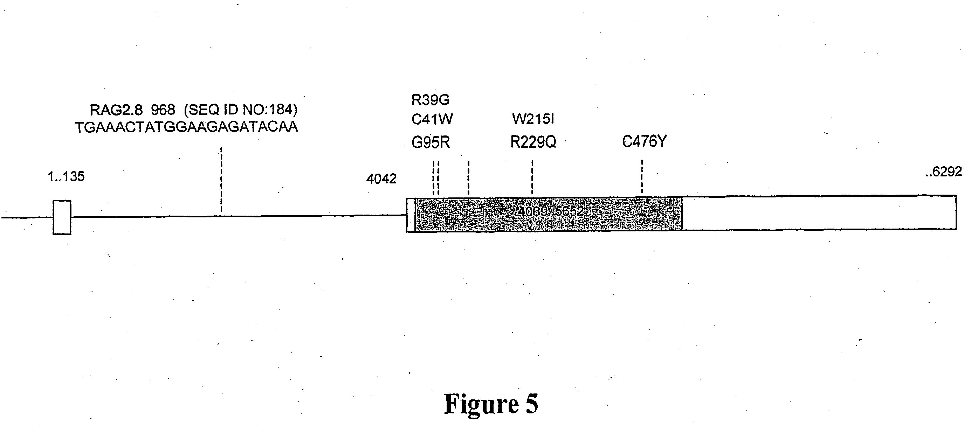

The Inventors have used this strategy to engineer I-*Cre*I variants which are able to cleave a DNA target sequence from a RAG gene and thus can be used for repairing the RAG1 and RAG2 mutations associated with a SCID syndrome (Figures 4 and 5). Other potential applications include genome engineering at the RAG genes loci.

The engineered variant can be used for gene correction via double-strand break induced recombination (Figure 1A and 1B).

The invention relates to an I-*Cre*I variant wherein at least one of the two I-*Cre*I monomers has at least two substitutions, one in each of the two functional subdomains of the LAGLIDADG core domain situated respectively from positions 26 to 40 and 44 to 77 of I-*Cre*I, and is able to cleave a DNA target sequence from a RAG gene.The cleavage activity of the variant according to the invention may be measured by any well-known*, in vitro* or *in* vivo cleavage assay, such as those described in the International PCT Application WO 2004/067736 or in Arnould et al., J. Mol. Biol., 2006, 355, 443-458. For example, the cleavage activity of the variant of the invention may be measured by a direct repeat recombination assay, in yeast or mammalian cells, using a reporter vector. The reporter vector comprises two truncated, non-functional copies of a reporter gene (direct repeats) and the genomic DNA target sequence within the intervening sequence, cloned in a yeast or a mammalian expression vector. Expression of the variant results in a functional endonuclease which is able to cleave the genomic DNA target sequence. This cleavage induces homologous recombination between the direct repeats, resulting in a functional reporter gene, whose expression can be monitored by appropriate assay.

### Definitions

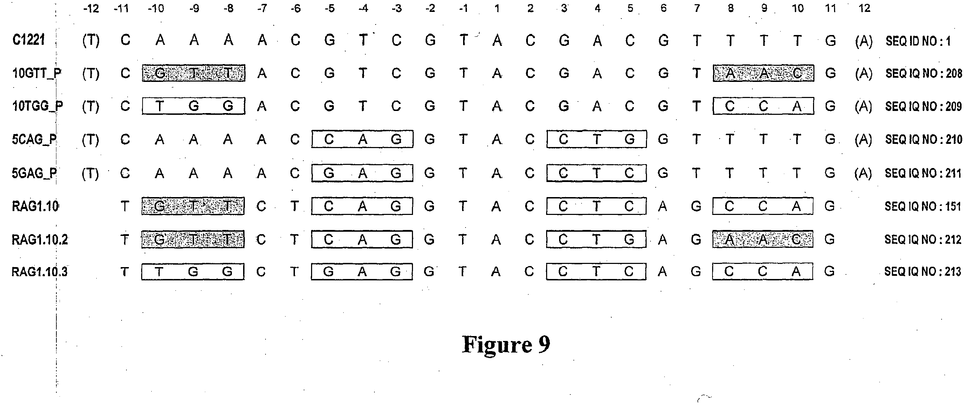

- Amino acid residues in a polypeptide sequence are designated herein according to the one-letter code, in which, for example, Q means Gln or Glutamine residue, R means Arg or Arginine residue and D means Asp or Aspartic acid residue.
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- by "meganuclease", is intended an endonuclease having a double-stranded DNA target sequence of 14 to 40 pb. Said meganuclease is either a dimeric enzyme, wherein each domain is on a monomer or a monomeric enzyme comprising the two domains on a single polypeptide.
- by "meganuclease domain" is intended the region which interacts with one half of the DNA target of a meganuclease and is able to associate with the other domain of the same meganuclease which interacts with the other half of the DNA target to form a functional meganuclease able to cleave said DNA target.
- by "meganuclease variant" or "variant" is intented a meganuclease obtained by replacement of at least one residue in the amino acid sequence of the wild-type meganuclease (natural meganuclease) with a different amino acid.
- by "functional variant" is intended a variant which is able to cleave a DNA target sequence, preferably said target is a new target which is not cleaved by the parent meganuclease. For example, such variants have amino acid variation at positions contacting the DNA target sequence or interacting directly or indirectly with said DNA target.
- by "I-*Cre*I" is intended the wild-type I-*Cre*I having the sequence SWISSPROT P05725 (SEQ ID NO: 234) or pdb accession code 1g9y.
- by "I-*Cre*I variant with novel specificity" is intended a variant having a pattern of cleaved targets different from that of the parent meganuclease. The terms "novel specificity", "modified specificity", "novel cleavage specificity", "novel substrate specificity" which are equivalent and used indifferently, refer to the specificity of the variant towards the nucleotides of the DNA target sequence.
- by *"*I-*Cre*I site" is intended a 22 to 24 bp double-stranded DNA sequence which is cleaved by I-*Cre*I. I-*Cre*I sites include the wild-type (natural) non-palindromic I-*Cre*I homing site and the derived palindromic sequences such as the sequence 5'- t₋₁₂c₋₁₁a₋₁₀a₋₉a₋₈a₋₇c₋₆g₋₅t₋₄c₋₃g₋₂t₋₁a₊₁c₊₂g₊₃a₊₄c₊₅g₊₆t₊₇t₊₈t₊₉t₊₁₀g₊₁₁a₊₁₂ (SEQ ID NO:1), also called C1221 (figures 3 and 9).
- by "domain" or "core domain" is intended the "LAGLIDADG Homing Endonuclease Core Domain" which is the characteristic α₁β₁β₂α₂β₃β₄α₃ fold of the homing endonucleases of the LAGLIDADG family, corresponding to a sequence of about one hundred amino acid residues. Said domain comprises four beta-strands (β₁, β₂, β₃, β₄) folded in an antiparallel beta-sheet which interacts with one half of the DNA target. This domain is able to associate with another LAGLIDADG Homing Endonuclease Core Domain which interacts with the other half of the DNA target to form a functional endonuclease able to cleave said DNA target. For example, in the case of the dimeric homing endonuclease I-*Cre*I (163 amino acids), the LAGLIDADG Homing Endonuclease Core Domain corresponds to the residues 6 to 94.
- by "subdomain" is intended the region of a LAGLIDADG Homing Endonuclease Core Domain which interacts with a distinct part of a homing endonuclease DNA target half-site. Two different subdomains behave independently and the mutation in one subdomain does not alter the binding and cleavage properties of the other subdomain. Therefore, two subdomains bind distinct part of a homing endonuclease DNA target half-site.
- by "beta-hairpin" is intended two consecutive beta-strands of the antiparallel beta-sheet of a LAGLIDADG homing endonuclease core domain (β₁β₂ or β₃β₄) which are connected by a loop or a turn.
- by "single-chain meganuclease", "single-chain chimeric meganuclease", "single-chain meganuclease derivative", "single-chain chimeric meganuclease derivative" or "single-chain derivative", is intended a meganuclease comprising two LAGLIDADG homing endonuclease domains or core domains linked by a peptidic spacer. The single-chain meganuclease is able to cleave a chimeric DNA target sequence comprising one different half of each parent meganuclease target sequence.
- by "DNA target", "DNA target sequence", "target sequence" , "target-site", "target", "site"; "site of interest"; "recognition site", "recognition sequence", "homing recognition site", "homing site", "cleavage site" is intended a 20 to 24 bp double-stranded palindromic, partially palindromic (pseudo-palindromic) or non-palindromic polynucleotide sequence that is recognized and cleaved by a LAGLIDADG homing endonuclease. These terms refer to a distinct DNA location, preferably a genomic location, at which a double stranded break (cleavage) is to be induced by the endonuclease. The DNA target is defined by the 5' to 3' sequence of one strand of the double-stranded polynucleotide, as indicated above for C1221. Cleavage of the DNA target occurs at the nucleotides in positions +2 and -2, respectively for the sense and the antisense strand (Figure 3). Unless otherwise indicated, the position at which cleavage of the DNA target by an I-*Cre* I meganuclease variant occurs, corresponds to the cleavage site on the sense strand of the DNA target.
- by "DNA target half-site", "half cleavage site" or half-site" is intended the portion of the DNA target which is bound by each LAGLIDADG homing endonuclease core domain.
- by "chimeric DNA target" or "hybrid DNA target" is intended the fusion of a different half of two parent meganucleases target sequences. In addition, at least one half of said target may comprise the combination of nucleotides which are bound by at least two separate subdomains (combined DNA target).
- by "DNA target sequence from a RAG gene", genomic DNA target sequence", "genomic DNA cleavage site", "genomic DNA target" or "genomic target" is intended a 20 to 24 bp sequence of a RAG gene which is recognized and cleaved by a meganuclease variant or a single-chain chimeric meganuclease derivative.
- by "RAG gene" is intended the RAG1 or RAG2 gene of a mammal. For example, the human RAG genes are available in the NCBI database, under the accession number NC_000011.8: the RAG1 (GeneID:5896) and RAG2 (GeneID:5897) sequences are situated from positions 36546139 to 36557877 and 36570071 to 36576362 (minus strand), respectively. Both genes have a short untranslated exon 1 and an exon 2 comprising the ORF coding for the RAG protein, flanked by a short and a long untranslated region, respectively at its 5' and 3' ends (Figures 4 and 5).
- by "vector" is intended a nucleic acid molecule capable of transporting another nucleic acid to which it has been linked.
- by "homologous" is intended a sequence with enough identity to another one to lead to a homologous recombination between sequences, more particularly having at least 95 % identity, preferably 97 % identity and more preferably 99 %.
- "identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default settings.
- "individual" includes mammals, as well as other vertebrates (e.g., birds, fish and reptiles). The terms "mammal" and "mammalian", as used herein, refer to any vertebrate animal, including monotremes, marsupials and placental, that suckle their young and either give birth to living young (eutharian or placental mammals) or are egg-laying (metatharian or nonplacental mammals). Examples of mammalian species include humans and other primates (e.g., monkeys, chimpanzees), rodents (e.g., rats, mice, guinea pigs) and others such as for example: cows, pigs and horses.
- by mutation is intended the substitution, deletion, addition of one or more nucleotides/amino acids in a polynucleotide (cDNA, gene) or a polypeptide sequence. Said mutation can affect the coding sequence of a gene or its regulatory sequence. It may also affect the structure of the genomic sequence or the structure/stability of the encoded mRNA.

The instant invention is limited to RAG1 gene.

The variant according to the present invention may be a homodimer which is able to cleave a palindromic or pseudo-palindromic DNA target sequence. Alternatively, said variant is an heterodimer, resulting from the association of a first and a second monomer having different mutations in positions 26 to 40 and/or 44 to 77 of I-*Cre*I, said heterodimer being able to cleave a non-palindromic DNA target sequence from a RAG1 gene. Preferably, both monomers of the heterodimer have different substitutions both in positions 26 to 40 and 44 to 77 of I-*Cre*I of SEQ ID NO:234.

In a preferred embodiment of said variant, said substitution(s) in the subdomain situated from positions 44 to 77 of I-*Cre*I are in positions 44, 68, 70, 75 and/or 77.

The mutations in positions 44, 68, 70, 75 and/or 77 may be advantageously combined with a mutation in position 66.

In another preferred embodiment of said variant, said substitution(s) in the subdomain situated from positions 26 to 40 of I-*Cre*I are in positions 26, 28, 30, 32, 33, 38 and/or 40.

In another preferred embodiment of said variant, said substitutions are replacement of the initial amino acids with amino acids selected from the group consisting of: A, D, E, G, H, K, N, P, Q, R, S, T, Y, C, V, L and W.

For example:
- the lysine (K) in position 28 may be mutated in : N, Q, A or R,
- the asparagine (N) in position 30 may be mutated in: H, G, R, K and D,
- the serine (S) in position 32 may be mutated in : G, T, K, E, H, D and Q,
- the tyrosine (Y) in position 33 may be mutated in: S, R, C, A, N, R, G, T and H,
- the glutamine (Q) in position 38 may be mutated in: R, A, T, Y, E, G, W, D and H,
- the serine (S) in position 40 may be mutated in: R, K, Q, A, D, E and H,
- the glutamine (Q) in position 44 may be mutated in : A, Y, N, K, D, R, T, E and H,
- the arginine (R) in position 68 may be mutated in: H, A, Y, S, N, T, E and G,
- the arginine (R) in position 70 may be mutated in: T, S, N, Q, H and A,
- the aspartic acid (D) in position 75 may be mutated in: R, Y, E, N, Q, K and S, and
- the isoleucine (I) in position 77 may be mutated in: V, L N, R, Y, Q, E, K and D.

In another preferred embodiment of said variant, it comprises one or more substitutions at additional positions.

The additional residues which are mutated may contact the DNA target sequence or interact with the DNA backbone or with the nucleotide bases, directly or via a water molecule; these I-*Cre*I interacting residues are well-known in the art. For example, additional mutations may be introduced at positions interacting indirectly with the phosphate backbone or the nucleotide bases.

Alternatively, said variant may comprise one or more additional mutations that improve the binding and/or the cleavage properties of the variant towards the DNA target sequence from a RAG1 gene. The additional residues which are mutated may be on the entire I-*Cre*I sequence or in the C-terminal half of I-*Cre*I (positions 80 to 163). These mutations are preferably substitutions in positions: 4, 6, 19, 34, 43, 49, 50, 54, 79, 80, 82, 85, 86, 87, 94, 96, 100, 103, 105, 107, 108, 114, 115, 116, 117, 125, 129, 131, 132, 139, 147, 150, 151, 153, 154, 155, 157, 159 and 160 of I-*Cre*I. More preferably, the substitutions are selected in the group consisting of: G19S, G19A, F54L, S79G, F87L, V105A and I132V.

Among these mutations, the G19S mutation is still more preferred since it not only increases the cleavage activity of I-*Cre*I derived heterodimeric meganucleases but also the cleavage specificity of said heterodimeric meganucleases by impairing the formation of a functional homodimer from the monomer carrying the G19S mutation.

The DNA target sequence which is cleaved by said variant may be in an exon or in an intron of the RAG1 gene. Preferably, it is located, either in the vicinity of a mutation, preferably within 500 bp of the mutation, or upstream of a mutation, preferably upstream of all the mutations of said RAG1 gene.

In another preferred embodiment of said variant, said DNA target sequence is from a human RAG1 gene.

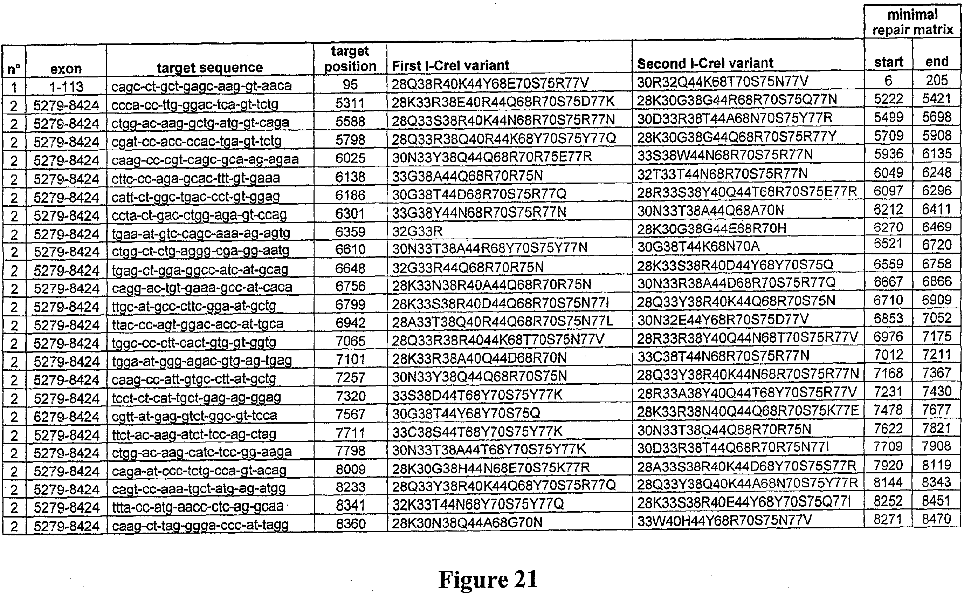

DNA targets from each human RAG1 gene are presented in Table III and Figure 21.

For example, the sequences SEQ ID NO: 148 to 177 are DNA targets from the RAG1 gene ; SEQ ID NO: 152 to 177 are situated in the RAG1 ORF (positions 5293 to 8424) and these sequences cover all the RAG1 ORF (Table III and Figures 4 and 21). The target sequence SEQ ID NO: 151 (RAG 1.10) is situated close to the RAG ORF and upstream of the mutations (Figure 4). The target sequences SEQ ID NO: 148, 149 (RAG1.6), and 150 (RAG1.7) are situated upstream of the mutations (Figure 4).

Thus the variant according to the invention is an heterodimer able to cleave a DNA target sequence from a human RAG1 gene selected from the group consisting of the sequences SEQ ID NO: 148 to 177, said heterodimer consisting of a first and a second monomer having amino acids at positions 28, 30, 32, 33, 38, 40, 44, 68, 70, 75 and 77 of I-*Cre*I which are preferably as indicated in Table I, Figure 21 or in Table XV as specified in claim 2.

**Table I: Sequence of heterodimeric I-CreI variants having a DNA target site in the RAG1 gene**

| First monomer | Second monomer | Target* Position |
|---|---|---|
| 28Q38R40K44Y68E70S75R77V | 30R32Q44K68T70S75N77V | 95 |
| 28K30N32S33S38R40H44A68Y70S75Y77K | 28A30N32S33S38R40K44D68N70S75N77I | 1692 |
| 28K30D32S33R38T40S44Y68S70S75S77D | 28K30N32T33C38Q40S44K68Y70S75Q77N | 2308 |
| 28N30N32S33S38R40R44A68R70T75N77I | 28K30R32S33C38Q40S44A68Y70S75Y77K | 5270 |
| 28N30N32S33S38R40R44Y68R70S75Q77V | 28K30K32S33A38Q40S44A68Y70S75Y77K | 5270 |
| 28N30N32S33S38R40R44Y68R70S75Q77V | 28K30K32S33A38Q40S44A68S70S75D77R | 5270 |
| 28N30N32S33S38R40R44Y68R70S75Q77V | 28K30K32S33A38Q40S44R68Y70S75D77T | 5270 |
| 28N30N32S33S38R40R44Y68R70S75Q77V | 28K30R32S33N38Q40S44T68Y70S75Y77R | 5270 |
| 28N30N32S33S38R40R44Y68R70S75Q77V | 28K30R32S33C38Q40S44A68Y70S75Y77K | 5270 |
| 28N30N32S33S38R40R44Y68R70S75Q77V | 28K30R32S33N38Q40S44K68Y70S75Y77N | 5270 |
| 28N30N32S33S38R40R44Y68R70S75Q77V | 28K30N32S33R38T40S44A68Y70S75Y77K | 5270 |
| 28N30N32S33S38R40R44Y68R70S75Q77V | 28K30K32S33G38Q40S44A68Y70S75Y77K | 5270 |
| 28N30N32S33S38R40R44A68R70N75N77I | 28K30K32S33A38Q40S44A68Y70S75Y77K | 5270 |
| 28N30N32S33S38R40R44A68R70N75N77I | 28K30K32S33A38Q40S44A68S70S75D77R | 5270 |
| 28N30N32S33S38R40R44A68R70N75N77I | 28K30R32S33N38Q40S44T68Y70S75Y77R | 5270 |
| 28N30N32S33S38R40R44A68R70N75N77I | 28K30R32S33C38Q40S44A68Y70S75Y77K | 5270 |
| 28N30N32S33S38R40R44A68R70N75N77I | 28K30R32S33N38Q40S44K68Y70S75Y77N | 5270 |
| 28N30N32S33S38R40R44A68R70N75N77I | 28K30N32S33R38T40S44A68Y70S75Y77K | 5270 |
| 28N30N32S33S38R40R44A68R70N75N77I | 28K30K32S33G38Q40S44A68Y70S75Y77K | 5270 |
| 28Q30N32S33S38R40K44A68H70Q75N77I | 28K30K32S33A38Q40S44A68Y70S75Y77K | 5270 |
| 28Q30N32S33S38R40K44A68H70Q75N77I | 28K30K32S33A38Q40S44A68S70S75D77R | 5270 |
| 28Q30N32S33S38R40K44A68H70Q75N77I | 28K30R32S33N38Q40S44T68Y70S75Y77R | 5270 |
| 28Q30N32S33S38R40K44A68H70Q75N77I | 28K30R32S33C38Q40S44A68Y70S75Y77K | 5270 |
| 28Q30N32S33S38R40K44A68H70Q75N77I | 28K30R32S33N38Q40S44K68Y70S75Y77N | 5270 |
| 28Q30N32S33S38R40K44A68H70Q75N77I | 28K30N32S33R38T40S44A68Y70S75Y77K | 5270 |
| 28Q30N32S33S38R40K44A68H70Q75N77I | 28K30K32S33G38Q40S44A66Y70S75Y77K | 5270 |
| 28K30H32S33M38A40S44A68R70S75Y77T | 28K30K32S33A38Q40S44A68Y70S75Y77K | 5270 |
| 28K30H32S33M38A40S44A68R70S75Y77T | 28K30R32S33N38Q40S44A68Y70S75Y77K117G | 5270 |
| 28K30H32S33M38A40S44A68R70S75Y77T | 28K30R32S33N38Q40S44A68Y70S75Y77K107R153G | 5270 |
| 28K30H32S33M38A40S44A68R70S75Y77T | 28K30R32S33N38Q40S44A68Y70S75D77R | 5270 |
| 28K30H32S33M38A40S44A68R70S75Y77T | 28K30R32S33N34T38Q40S44A68Y70S75Y77K117K | 5270 |
| 28K30H32S33M38A40S44A68R70S75Y77T | 28K30R32S33N38Q40S44A68Y70S75Y77K100R | 5270 |
| 28K30H32S33M38A40S44A68R70S75Y77T | 28K30R32S33N38Q40S44A68Y70S75Y77K150T | 5270 |
| 28N30N32S33S38R40R44Y68R70S75Y77Q | 28K30K32S33A38Q40S44A68Y70S75Y77K | 5270 |
| 28N30N32S33S38R40R44Y68R70S75Y77Q | 28K30K32S33A38Q40S44A68S70S75D77R | 5270 |
| 28N30N32S33S38R40R44Y68R70S75Y77Q | 28K30K32S33A38Q40S44R68Y70S75D77T | 5270 |
| 28N30N32S33S38R40R44Y68R70S75Y77Q | 28K30R32S33N38Q40S44T68Y70S75Y77R | 5270 |
| 28N30N32S33S38R40R44Y68R70S75Y77Q | 28K30R32S33C38Q40S44A68Y70S75Y77K | 5270 |
| 28N30N32S33S38R40R44Y68R70S75Y77Q | 28K30R32S33N38Q40S44K68Y70S75Y77N | 5270 |
| 28N30N32S33S38R40R44Y68R70S75Y77Q | 28K30N32S33R38T40S44A68Y70S75Y77K | 5270 |
| 28N30N32S33S38R40R44Y68R70S75Y77Q | 28K30K32S33G38Q40S44A68Y70S75Y77K | 5270 |
| 28N30N32S33S38R40R44N68R70S75Y77V | 28K30K32S33A38Q40S44A68Y70S75Y77K | 5270 |
| 28N30N32S33S38R40R44N68R70S75Y77V | 28K30K32S33A38Q40S44A68S70S75D77R | 5270 |
| 28N30N32S33S38R40R44N68R70S75Y77V | 28K30R32S33N38Q40S44T68Y70S75Y77R | 5270 |
| 28N30N32S33S38R40R44N68R70S75Y77V | 28K30R32S33C38Q40S44A68Y70S75Y77K | 5270 |
| 28N30N32S33S38R40R44N68R70S75Y77V | 28K30R32S33N38Q40S44K68Y70S75Y77N | 5270 |
| 28N30N32S33S38R40R44N68R70S75Y77V | 28K30N32S33R38T40S44A68Y70S75Y77K | 5270 |
| 28N30N32S33S38R40R44N68R70S75Y77V | 28K30K32S33G38Q40S44A68Y70S75Y77K | 5270 |
| 28Q30N32S33S38R40K44A68A70N75N77I | 28K30K32S33A38Q40S44A68Y70S75Y77K | 5270 |
| 28Q30N32S33S38R40K44A68A70N75N77I | 28K30K32S33A38Q40S44A68S70S75D77R | 5270 |
| 28Q30N32S33S38R40K44A68A70N75N77I | 28K30R32S33N38Q40S44T68Y70S75Y77R | 5270 |
| 28Q30N32S33S38R40K44A68A70N75N77I | 28K30R32S33C38Q40S44A68Y70S75Y77K | 5270 |
| 28Q30N32S33S38R40K44A68A70N75N77I | 28K30R32S33N38Q40S44K68Y70S75Y77N | 5270 |
| 28Q30N32S33S38R40K44A68A70N75N77I | 28K30N32S33R38T40S44A68Y70S75Y77K | 5270 |
| 28K33R38E40R44Q68R70S75D77K | 28K30G38G44R68R70S75Q77N | 5311 |
| 28Q33S38R40K44N68R70S75R77N | 30D33R38T44A68N70S75Y77R | 5588 |
| 28Q33R38Q40R44K68Y70S75Y77Q | 28K30G38G44Q68R70S75R77Y | 5798 |
| 30N33Y38Q44Q68R70R75E77R | 33S38W44N68R70S75R77N | 6025 |
| 33G38A44Q68R70R75N | 32T33T44N68R70S75R77N | 6138 |
| 30G38T44D68R70S75R77Q | 28R33S38Y40Q44T68R70S75E77R | 6186 |
| 33G38Y44N68R70S75R77N | 30N33T38A44Q68A70N | 6301 |
| 32G33R | 28K30G38G44E68R70H | 6359 |
| 30N33T38A44R68Y70S75Y77N | 30G38T44K68N70A | 6610 |
| 32G33R44Q68R70R75N | 28K33S38R40D44Y68Y70S75Q | 6648 |
| 28K33N38R40A44Q68R70R75N | 30N33R38A44D68R70S75R77Q | 6756 |
| 28K33S38R40D44Q68R70S75N77I | 28Q33Y38R40K44Q68R70S75N | 6799 |
| 28A33T38Q40R44Q68R70S75N77L | 30N32E44Y68R70S75D77V | 6942 |
| 28Q33R38R40K44K68T70S75N77V | 28R33R38Y40Q44N68T70S75R77V | 7065 |
| 28K33R38A40Q44D68R70N | 33C38T44N68R70S75R77N | 7101 |
| 30N33Y38Q44Q68R70S75N | 28Q33Y38R40K44N68R70S75R77N | 7257 |
| 28K30R32D33Y38Q40S44D68N70S75N77I | 28K30G32S33Y38H40S44N68R70S75R77D | 7296 |
| 33S38D44T68Y70S75Y77K | 28R33A38Y40Q44T68Y70S75R77V | 7320 |
| 30G38T44Y68Y70S75Q | 28K33R38N40Q44Q68R70S75K77E | 7567 |
| 33C38S44T68Y70S75Y77K | 30N33T38Q44Q68R70R75N | 7711 |
| 30N33T38A44T68Y70S75Y77K | 30D33R38T44Q68R70R75N77I | 7798 |
| 28K30G38H44N68E70S75K77R | 28A33S38R40K44D68Y70S75S77R | 8009 |
| 28Q33Y38R40K44Q68Y70S75R77Q | 28Q33Y38Q40K44A68N70S75Y77R | 8233 |
| 28K30N32S33H38Q40Q44D68N70S75N77I | 28K30D32S33R38Q40S44N68Y70S75R77V | 8238 |
| 32K33T44N68Y70S75Y77Q | 28K33S38R40E44Y68Y70S75Q77I | 8341 |
| 28K30N38Q44A68G70N | 33W40H44Y68R70S75N77V | 8360 |

| | | |
|---|---|---|
| *position of the first base of the target in the human RAG 1 gene. | | |

The sequence of each variant is defined by its amino acid residues at the indicated positions. For example, the first heterodimeric variant of Table I consists of a first monomer having Q, R, K, Y, E, S, R and V in positions 28, 38, 40, 44, 68, 70, 75 and 77, respectively and a second monomer having R, Q, K, T, S, N and V in positions 30, 32, 44, 68, 70, 75 and 77, respectively. The positions are indicated by reference to I-*Cre*I sequence SWISSPROT P05725 or pdb accession code 1g9y (SEQ ID NO: 234) ; I-*Cre*I has K, N, S, Y, Q, S, Q, R, R, D, I, E and K, in positions 28, 30, 32, 33, 38, 40, 44, 68, 70, 75, 77, 80 and 82, respectively.

Preferably, the variant according to the invention is an heterodimer consisting of a first and a second monomer having amino acids at positions 28, 30, 32, 33, 38, 40, 44, 68, 70, 75, 77 and at least one of the positions of I-*Cre*I defined in the instant invention, which are as indicated below in reference to Table XVII of claim 5:

| **First monomer** | **Second monomer** |
|---|---|
| 28K30H32S33M38A40S44A68R70S75Y77T | 28K30R32S33N38Q40S44A68Y70S75Y77K117G |
| 28K30H32S33M38A40S44A68R70S75Y77T | 28K30R32S33N38Q40S44A68Y70S75Y77K107R 153G |
| 28K30H32S33M38A40S44A68R70S75Y77T | 28K30R32S33N34T38Q40S44A68Y70S75Y77K117K |
| 28K30H32S33M38A40S44A68R70S75Y77T | 28K30R32S33N38Q40S44A68Y70S75Y77K100R |
| 28K30H32S33M38A40S44A68R70S75Y77T | 28K30R32S33N38Q40S44A68Y70S75Y77K150T |
| 19S28N30N32S33S38R40R44Y68R70S75Q77V | 28K30R32S33N38Q40S44A68Y70S75D77R |
| 28N30N32S33S38R40R44Y68R70S75Q77V | 19S28K30R32S33N38Q40S44A68Y70S75D77R |

The variant may consist of an I-*Cre*I sequence having the amino acid residues as indicated in Table I. In this case, the positions which are not indicated are not mutated and thus correspond to the wild-type I-*Cre*I sequence (SEQ ID NO: 234).

Examples of such heterodimeric I-*Cre*I variants having a DNA target site in the RAG1 gene are the variants consisting of a first monomer of the sequence SEQ ID NO: 2 to 38 and a second monomer of the sequence SEQ ID NO: 39 to 75, 248 to 253.

For instance, the variant according to the invention is an heterodimer consisting of a first and a second monomer selected from the following pairs of sequences : SEQ ID NO: 2 and 39, SEQ ID NO: 3 and 40, SEQ ID NO: 4 and 41, SEQ ID NO: 5 and 42, SEQ ID NO: 6 or 10 and any of the SEQ ID NO: 42 to 49, SEQ ID NO: 7, 8, 11 and any of the SEQ ID NO: 42 to 44 and 46 to 49, SEQ ID NO: 9 and any of SEQ ID NO: 43, 248 to 253, SEQ ID NO: 12 and any of the SEQ ID NO: 42 to 44 and 46 to 48, SEQ ID NO: 13 and 50, SEQ ID NO: 14 and 51, SEQ ID NO: 15 and 52, SEQ ID NO: 16 and 53, SEQ ID NO: 17 and 54, SEQ ID NO: 18 and 55, SEQ ID NO: 19 and 56, SEQ ID NO: 20 and 57, SEQ ID NO: 21 and 58, SEQ ID NO: 22 and 59, SEQ ID NO: 23 and 60, SEQ ID NO: 24 and 61, SEQ ID NO: 25 and 62, SEQ ID NO: 26 and 63, SEQ ID NO: 27 and 64, SEQ ID NO: 28 and 65, SEQ ID NO: 29 and 66, SEQ ID NO: 30 and 67, SEQ ID NO: 31 and 68, SEQ ID NO: 32 and 69, SEQ ID NO: 33 and 70, SEQ ID NO: 34 and 71, SEQ ID NO: 35 and 72, SEQ ID NO: 36 and 73, SEQ ID NO: 37 and 74, SEQ ID NO: 38 and 75, SEQ ID NO: 6 and the variant of SEQ ID NO:250 further including the G19S mutation, the variant of SEQ ID NO:6 further including the G19S mutation and SEQ ID NO: 250, and wherein said heterodimer is able to cleave a DNA target sequence from a human RAG1 gene selected from the group consisting of the sequences SEQ ID NO: 148 to 177.

Alternatively, the variant may comprise an I-*Cre*I sequence having the amino acid residues as indicated in Table I.

In the latter case, the positions which are not indicated may comprise mutations as defined above, or may not be mutated. For example, the variant may be derived from an I-*Cre*I scaffold protein encoded by SEQ ID NO: 203, said I-*Cre*I scaffold protein (SEQ ID NO: 235) having the insertion of an alanine in position 2, the substitutions A42T, D75N, W110E and R111Q and three additional amino acids (A, A and D) at the C-terminus. In addition, said variant, derived from wild-type I-*Cre*I or an I-*Cre*I scaffold protein, may comprise additional mutations, as defined above.

The position of the first base of the target which is cleaved by each heterodimeric variant is indicated in the last column of Table I.

More generally, the present invention relates to an I-*Cre*I variant comprising two I-*Cre*I monomers, characterized in that at least one of the two I-*Cre*I monomers has at least two substitutions, one in each of the two functional subdomains of the LAGLIDADG core domain situated respectively from positions 26 to 40 and 44 to 77 of I-*Cre*I aminoacid sequence SEQ ID NO:234, said variant being able to cleave a DNA target sequence from a human RAG1 gene selected from the group consisting of the sequences SEQ ID NO: 148 to 177, and being obtainable by a method comprising at least the steps of:
(a) constructing a first series of I-*Cre*I variants having at least one substitution at positions 26, 28, 30, 32, 33, 38 and/or 40 of a first functional subdomain of the LAGLIDADG core domain situated from positions 26 to 40 of I-*Cre*I,
(b) constructing a second series of I-*Cre*I variants having at least one substitution at positions 44, 68, 70, 75, and/or 77 of a second functional subdomain of the LAGLIDADG core domain situated from positions 44 to 77 of I-*Cre*I,
(c) selecting the variants from the first series of step (a) which are able to cleave a mutant I-*Cre*I site wherein (i) the nucleotide triplet in positions -10 to -8 of the I-*Cre*I site has been replaced with the nucleotide triplet which is present in position -10 to -8 of said human RAG1 DNA target and (ii) the nucleotide triplet in positions +8 to +10 has been replaced with the reverse complementary sequence of the nucleotide triplet which is present in position -10 to -8 of said human RAG1 DNA target,
(d) selecting the variants from the second series of step (b) which are able to cleave a mutant I-*Cre*I site wherein (i) the nucleotide triplet in positions -5 to -3 of the I-*Cre*I site has been replaced with the nucleotide triplet which is present in position -5 to -3 of said human RAG1 DNA target and (ii) the nucleotide triplet in positions +3 to +5 has been replaced with the reverse complementary sequence of the nucleotide triplet which is present in position -5 to -3 of said human RAG1 DNA target,
(e) selecting the variants from the first series of step (a) which are able to cleave a mutant I-*Cre*I site wherein (i) the nucleotide triplet in positions +8 to +10 of the I-*Cre*I site has been replaced with the nucleotide triplet which is present in positions +8 to +10 of said human RAG1 DNA target and (ii) the nucleotide triplet in positions -10 to -8 has been replaced with the reverse complementary sequence of the nucleotide triplet which is present in position +8 to +10 of said human RAG1 DNA target,
(f) selecting the variants from the second series of step (b) which are able to cleave a mutant I-*Cre*I site wherein (i) the nucleotide triplet in positions +3 to +5 of the I-*Cre*I site has been replaced with the nucleotide triplet which is present in positions +3 to +5 of said human RAG1 DNA target and (ii) the nucleotide triplet in positions -5 to -3 has been replaced with the reverse complementary sequence of the nucleotide triplet which is present in position +3 to +5 of said human RAG1 DNA target,
(g) combining in a single variant, the mutation(s) in positions 26 to 40 and 44 to 77 of two variants from step (c) and step (d), to obtain a novel homodimeric I-*Cre*I variant which cleaves a sequence wherein (i) the nucleotide triplet in positions -10 to -8 is identical to the nucleotide triplet which is present in positions -10 to -8 of said human RAG1 DNA target, (ii) the nucleotide triplet in positions +8 to +10 is identical to the reverse complementary sequence of the nucleotide triplet which is present in positions -10 to -8 of said human RAG1 DNA target, (iii) the nucleotide triplet in positions -5 to -3 is identical to the nucleotide triplet which is present in positions -5 to -3 of said human RAG1 DNA target and (iv) the nucleotide triplet in positions +3 to +5 is identical to the reverse complementary sequence of the nucleotide triplet which is present in positions -5 to -3 of said human RAG1 DNA target, and/or
(h) combining in a single variant, the mutation(s) in positions 26 to 40 and 44 to 77 of two variants from step (e) and step (f), to obtain a novel homodimeric I-*Cre*I variant which cleaves a sequence wherein (i) the nucleotide triplet in positions +3 to +5 is identical to the nucleotide triplet which is present in positions +3 to +5 of said human RAG1 DNA target, (ii) the nucleotide triplet in positions -5 to -3 is identical to the reverse complementary sequence of the nucleotide triplet which is present in positions +3 to +5 of said human RAG1 DNA target, (iii) the nucleotide triplet in positions +8 to +10 of the I-*Cre*I site has been replaced with the nucleotide triplet which is present in positions +8 to +10 of said human RAG1 DNA target and (iv) the nucleotide triplet in positions -10 to -8 is identical to the reverse complementary sequence of the nucleotide triplet in positions +8 to +10 of said human RAG1 DNA target,
(i) combining the variants obtained in steps (g) and (h) to form heterodimers, and
(j) selecting the heterodimers from step (i) which are able to cleave said DNA target sequences from RAG1 gene.

In addition, the variants of the invention may include one or more residues inserted at the NH₂ terminus and/or COOH terminus of the sequence. For example, a tag (epitope or polyhistidine sequence) is introduced at the NH₂ terminus and/or COOH terminus; said tag is useful for the detection and/or the purification of said variant.

The subject-matter of the present invention is also a single-chain chimeric endonuclease derived from an I-*Cre*I variant as defined above. The single-chain chimeric endonuclease may comprise two I-*Cre*I monomers, two I-*Cre*I core domains (positions 6 to 94 of I-*Cre*I) or a combination of both.

The subject-matter of the present invention is also a polynucleotide fragment encoding a variant or a single-chain chimeric endonuclease as defined above; said polynucleotide may encode one monomer of an homodimeric or heterodimeric variant, or two domains/monomers of a single-chain chimeric endonuclease.

The subject-matter of the present invention is also a recombinant vector for the expression of a variant or a single-chain molecule according to the invention. The recombinant vector comprises at least one polynucleotide fragment encoding a variant or a single-chain molecule, as defined above.

In a preferred embodiment, said vector comprises two different polynucleotide fragments, each encoding one of the monomers of an heterodimeric variant.

A vector which can be used in the present invention includes, but is not limited to, a viral vector, a plasmid, a RNA vector or a linear or circular DNA or RNA molecule which may consist of a chromosomal, non-chromosomal, semi-synthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available.

Viral vectors include retrovirus, adenovirus, parvovirus (e. g. adeno-associated viruses), coronavirus, negative strand RNA viruses such as orthomyxovirus (e. g., influenza virus), rhabdovirus (e. g., rabies and vesicular stomatitis virus), para-myxovirus (e. g. measles and Sendai), positive strand RNA viruses such as picornavirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996).

Preferred vectors include lentiviral vectors, and particularly self inactivacting lentiviral vectors.

Vectors can comprise selectable markers, for example: neomycin phosphotransferase, histidinol dehydrogenase, dihydrofolate reductase, hygromycin phosphotransferase, herpes simplex virus thymidine kinase, adenosine deaminase, glutamine synthetase, and hypoxanthine-guanine phosphoribosyl transferase for eukaryotic cell culture; TRP1 for *S. cerevisiae;* tetracycline, rifampicin or ampicillin resistance in *E. coli.*

Preferably said vectors are expression vectors, wherein the sequence(s) encoding the variant/single-chain molecule of the invention is placed under control of appropriate transcriptional and translational control elements to permit production or synthesis of said variant. Therefore, said polynucleotide is comprised in an expression cassette. More particularly, the vector comprises a replication origin, a promoter operatively linked to said encoding polynucleotide, a ribosome-binding site, an RNA-splicing site (when genomic DNA is used), a polyadenylation site and a transcription termination site. It also can comprise an enhancer. Selection of the promoter will depend upon the cell in which the polypeptide is expressed. Preferably, when said variant is an heterodimer, the two polynucleotides encoding each of the monomers are included in one vector which is able to drive the expression of both polynucleotides, simultaneously. Suitable promoters include tissue specific and/or inducible promoters. Examples of inducible promoters are: eukaryotic metallothionine promoter which is induced by increased levels of heavy metals, prokaryotic lacZ promoter which is induced in response to isopropyl-β-D-thiogalacto-pyranoside (IPTG) and eukaryotic heat shock promoter which is induced by increased temperature. Examples of tissue specific promoters are skeletal muscle creatine kinase, prostate-specific antigen (PSA), α-antitrypsin protease, human surfactant (SP) A and B proteins, β-casein and acidic whey protein genes.

According to another advantageous embodiment of said vector, it includes a targeting construct comprising sequences sharing homologies with the region surrounding the genomic DNA target cleavage site as defined above.

Alternatively, the vector coding for an *I-CreI* variant and the vector comprising the targeting construct are different vectors.

More preferably, the targeting DNA construct comprises:
a) sequences sharing homologies with the region surrounding the genomic DNA cleavage site as defined above, and
b) a sequence to be introduced flanked by sequences as in a).

More precisely, the targeting DNA construct comprises a sequence to be introduced in a RAG1 gene flanked by sequences homologous to the sequences of the RAG1 gene flanking one of the RAG1 gene target sequence selected from the group consisting of the sequences SEQ ID NO: 148 to 177.

Preferably, homologous sequences of at least 50 bp, preferably more than 100 bp and more preferably more than 200 bp are used. Indeed, shared DNA homologies are located in regions flanking upstream and downstream the site of the break and the DNA sequence to be introduced should be located between the two arms. The sequence to be introduced is preferably a sequence which repairs a mutation in the gene of interest (gene correction or recovery of a functional gene), for the purpose of genome therapy. Alternatively, it can be any other sequence used to alter the chromosomal DNA in some specific way including a sequence used to modify a specific sequence, to attenuate or activate the endogenous gene of interest, to inactivate or delete the endogenous gene of interest or part thereof, to introduce a mutation into a site of interest or to introduce an exogenous gene or part thereof. Such chromosomal DNA alterations are used for genome engineering (animal models).

For correcting the RAG1 gene, cleavage of the gene occurs in the vicinity of the mutation, preferably, within 500 bp of the mutation (Figure 1A). The targeting construct comprises a RAG1 gene fragment which has at least 200 bp of homologous sequence flanking the target site (minimal repair matrix) for repairing the cleavage, and includes the correct sequence of the RAG1 gene for repairing the mutation (Figure 1A). Consequently, the targeting construct for gene correction comprises or consists of the minimal repair matrix; it is preferably from 200 pb to 6000 pb, more preferably from 1000 pb to 2000 pb.

Alternatively, for restoring a functional gene (Figure 1B), cleavage of the gene occurs upstream of a mutation, for example at positions 1704, 2320 or 5282 of the RAG1 gene (Figure 4) situated in the RAG1.6, RAG1.7 and RAG1.10 targets, respectively. Preferably said mutation is the first known mutation in the sequence of the gene, so that all the downstream mutations of the gene can be corrected simultaneously. The targeting construct comprises the exons downstream of the cleavage site fused in frame (as in the cDNA) and with a polyadenylation site to stop transcription in 3'. The sequence to be introduced (exon knock-in construct) is flanked by introns or exons sequences surrounding the cleavage site, so as to allow the transcription of the engineered gene (exon knock-in gene) into a mRNA able to code for a functional protein (Figure 1B). For example, the exon knock-in construct is flanked by sequences upstream and downstream of the cleavage site, from a minimal repair matrix as defined above.

For example, the target which is cleaved by each of the variant (Table I or Table XV in reference to claim 2) and the minimal matrix for repairing the cleavage with each variant are indicated in Table III and in Figure 21.

**Table III : RAG1 gene targets cleaved by I-CreI variants**

| **SEQ ID NO:** | **Sequence** | **Position*** | **Location** | **minimal repair matrix** | |
|---|---|---|---|---|---|
| | | | | **start** | **end** |
| **148** | cagcctgctaagcaaggtaaca | 95 | Exon 1 | 6 | 205 |
| **149** | ttgaataattcaaatqatacaa | 1692 | Intron 1 | 1603 | 1802 |
| **150** | tctgaaccataagtagttttag | 2308 | Intron 1 | 2219 | 2418 |
| **151** | tgttctcaggtacctcagccag | 5270 | Intron 1 | 5181 | 5380 |
| **152** | cccaccttggqactcagttctg | 5311 | Exon 2 | 5222 | 5421 |
| **153** | ctggacaaggctgatqgtcaga | 5588 | Exon 2 | 5499 | 5698 |
| **154** | cgatccaccccactgagttctg | 5798 | Exon 2 | 5709 | 5908 |
| **155** | caagcccgtcagcgcaagagaa | 6025 | Exon 2 | 5936 | 6135 |
| **156** | cttcccagagcactttatgaaa | 6138 | Exon 2 | 6049 | 6248 |
| **157** | cattctqqctgaccctgtggag | 6186 | Exon 2 | 6097 | 6296 |
| **158** | cctactgacctggagagtccag | 6301 | Exon 2 | 6212 | 6411 |
| **159** | tgaaatgtccagcaaaagagtg | 6359 | Exon 2 | 6270 | 6469 |
| **160** | ctggctctgagggcgaggaatg | 6610 | Exon 2 | 6521 | 6720 |
| **161** | tgagctggaggccatcatgcag | 6648 | Exon 2 | 6559 | 6758 |
| **162** | cagqactgtgaaagccatcaca | 6756 | Exon 2 | 6667 | 6866 |
| **163** | ttgcatgcccttcggaatgctg | 6799 | Exon 2 | 6710 | 6909 |
| **164** | ttacccagtggacaccattgca | 6942 | Exon 2 | 6853 | 7052 |
| **165** | tggccccttcactgtggtggtg | 7065 | Exon 2 | 6976 | 7175 |
| **166** | tggaatgggagacgtgagtgag | 7101 | Exon 2 | 7012 | 7211 |
| **167** | caagccattgtgccttatgctg | 7257 | Exon 2 | 7168 | 7367 |
| **168** | cgagacgctaactgccatcctg | 7296 | Exon 2 | 7207 | 7406 |
| **169** | tcctctcattgctgaqagggag | 7320 | Exon 2 | 7231 | 7430 |
| **170** | cgttatgaggtctggcgttcca | 7567 | Exon 2 | 7478 | 7677 |
| **171** | ttctacaagatcttccagctag | 7711 | Exon 2 | 7622 | 7821 |
| **172** | ctggacaagcatctccggaaga | 7798 | Exon 2 | 7709 | 7908 |
| **173** | cagaatccctctgccagtacag | 8009 | Exon 2 | 7920 | 8119 |
| **174** | **cagtccaaatgctatgaaatgg** | 8233 | Exon 2 | 8144 | 8343 |
| **175** | **ccaaatgctatgagatggaaga** | 8237 | Exon 2 | 8149 | 8348 |
| **176** | **tttaccatgaaccctcaggcaa** | 8341 | Exon 2 | 8252 | 8451 |
| **177** | **caagcttaggggacccattagg** | 8360 | Exon 2 | 8271 | 8470 |

| | | | | | |
|---|---|---|---|---|---|
| * position of the first base of the target in the human RAG1 gene. | | | | | |

For example, for correcting some of the mutations in the RAG1 gene associated with a SCID syndrome, as indicated in figure 4, the following combinations of variants/targeting constructs may be used:
- R396C, R396H, and D429G :
   * variant: 32G and 33R (first monomer)/ 28K, 30G, 38G, 44E, 68R and 70H (second monomer), and a targeting construct comprising at least positions 6270 to 6469 of the RAG1 gene, for efficient repair of the DNA double-strand break, and all sequences between the meganuclease cleavage site and the mutation site, for efficient repair of the mutation.
- R561C:
   * variant 28Q, 33R, 38R, 40K, 44K, 68T, 70S, 75N and 77V (first monomer)/ 28R, 33R, 38Y, 40Q, 44N, 68T, 70S, 75R and 77V (second monomer) and a targeting construct comprising at least positions 6976 to 7175 of the RAG1 gene, for efficient repair of the DNA double-strand break, and all sequences between the meganuclease cleavage site and the mutation site, for efficient repair of the mutation.
   * variant 30N, 33Y, 38Q, 44Q, 68R, 70S and 75N (first monomer)/ 28Q, 33Y, 38R, 40K, 44N, 68R, 70S, 75R and 77N (second monomer) and a targeting construct comprising at least positions 7168 to 7367 of the RAG1 gene, for efficient repair of the DNA double-strand break, and all sequences between the meganuclease cleavage site and the mutation site, for efficient repair of the mutation.
   * variant: 28K, 30R, 32D, 33Y, 38Q, 40S, 44D, 68N, 70S, 75N, and 77I (first monomer)/ 28K, 30G, 32S, 33Y, 38H, 40S, 44N, 68R, 70S, 75R, and 77D (second monomer), and a targeting construct comprising at least positions 7207 to 7406 of the RAG1 gene, for efficient repair of the DNA double-strand break, and all sequences between the meganuclease cleavage site and the mutation site, for efficient repair of the mutation.
- E774Ter (premature stop codon), R737H, E722K:
   * variant 30G, 38T, 44Y, 68Y, 70S, 75Q (first monomer)/ 28K, 33R, 38N, 40Q, 44Q, 68R, 70S, 75K, and 77E (second monomer) and a targeting construct comprising at least positions 7478 to 7677 of the RAG1 gene, for efficient repair of the DNA double-strand break, and all sequences between the meganuclease cleavage site and the mutation site, for efficient repair of the mutation.
- Y938Ter:
   * variant : 28K, 30N, 32S, 33H, 38Q, 40Q, 44D, 68N, 70S, 75N, and 77I (first monomer)/ 28K, 30D, 32S, 33R, 38Q, 40S, 44N, 68Y, 70S, 75R, and 77V (second monomer), and a targeting construct comprising at least positions 8149 to 8348 of the RAG1 gene, for efficient repair of the DNA double-strand break, and all sequences between the meganuclease cleavage site and the mutation site, for efficient repair of the mutation.
   * variant: 32K, 33T 44N, 68Y, 70S, 75Y and 77Q (first monomer)/ 28K, 33S, 38R, 40E, 44Y, 68Y, 70S, 75Q and 77I (second monomer), and a targeting construct comprising at least positions 8252 to 8451 of the RAG1 gene, for efficient repair of the DNA double-strand break, and all sequences between the meganuclease cleavage site and the mutation site, for efficient repair of the mutation.
   * variant : 28K, 30G, 38H, 44N, 68E, 70S, 75K, and 77R (first monomer)/ 28A, 33S, 38R, 40K, 44D, 68Y, 70S, 75S, and 77R (second monomer), and a targeting construct comprising at least positions 8149 to 8348 of the RAG1 gene, for efficient repair of the DNA double-strand break, and all sequences between the meganuclease cleavage site and the mutation site, for efficient repair of the mutation.

Alternatively, for restoring a functional RAG1 gene (Figure 1B), the following combinations of variants may be used in combination with an exon knock-in construct comprising a cDNA sequence coding for the RAG1 protein and a downstream polyadenylation site, flanked by sequences upstream and downstream of the cleavage site, from a minimal repair matrix as defined above (Table III):
* variant : 28K, 30N, 32S, 33S, 38R, 40H, 44A, 68Y, 70S, 75Y, and 77K (first monomer)/ 28A, 30N, 32S, 33S, 38R, 40K, 44D, 68N, 70S, 75N, and 77I (second monomer), and an exon knock-in construct flanked by sequences comprising at least positions 1608 to 1802 of the RAG1 gene for efficient repair of the DNA double-strand break.
* variant : 28K, 30D, 32S, 33R, 38T, 40S, 44Y, 68S, 70S, 75S, 77D (first monomer)/ 28K, 30N, 32T, 33C, 38Q, 40S, 44K, 68Y, 70S, 75Q, and 77N (second monomer), and an exon knock-in construct flanked by sequences comprising at least positions 2219 to 2418 of the RAG1 gene for efficient repair of the DNA double-strand break.
* variant : SEQ ID NO: 5 to 12 (first monomer)/ SEQ ID NO: 42 to 49, 248 to 253 (second monomer), and an exon knock-in construct flanked by sequences comprising at least positions 5181 to 5380 of the RAG1 gene for efficient repair of the DNA double-strand break.

The subject-matter of the present invention is also a composition characterized in that it comprises at least one variant, one single-chain chimeric endonuclease and/or at least one expression vector encoding said variant/single-chain molecule, as defined above.

In a preferred embodiment of said composition, it comprises a targeting DNA construct comprising a sequence which repairs a mutation in the RAG gene, flanked by sequences sharing homologies with the genomic DNA cleavage site of said variant, as defined above. The sequence which repairs the mutation is either a fragment of the gene with the correct sequence or an exon knock-in construct, as defined above.

Preferably, said targeting DNA construct is either included in a recombinant vector or it is included in an expression vector comprising the polynucleotide(s) encoding the variant/single-chain molecule according to the invention.

In the case where two vectors may be used, the subject-matter of the present invention is also products containing a I-*Cre*I variant expression vector as defined above and a vector which includes a targeting construct as defined above as a combined preparation for simultaneous, separate or sequential use in the treatment of a SCID syndrome associated with a mutation in a RAG gene.

The subject-matter of the present invention is also the use of at least one meganuclease variant and/or one expression vector, as defined above, for the preparation of a medicament for preventing, improving or curing a SCID syndrome associated with a mutation in a RAG gene, in an individual in need thereof, said medicament being administrated by any means to said individual.

In this case, the use of the meganuclease variant comprises at least the step of (a) inducing in somatic tissue(s) of the individual a double stranded cleavage at a site of interest comprising at least one recognition and cleavage site of said variant, and (b) introducing into the individual a targeting DNA, wherein said targeting DNA comprises (1) DNA sharing homologies to the region surrounding the cleavage site and (2) DNA which repairs the site of interest upon recombination between the targeting DNA and the chromosomal DNA. The targeting DNA is introduced into the individual under conditions appropriate for introduction of the targeting DNA into the site of interest.

According to the present invention, said double-stranded cleavage is induced, either *in toto* by administration of said meganuclease to an individual, or *ex vivo* by introduction of said meganuclease into somatic cells (hematopoietic stem cells) removed from an individual and returned into the individual after modification.

The subject-matter of the present invention is also a method for preventing, improving or curing a SCID syndrome in an individual in need thereof, said method comprising at least the step of administering to said individual a composition as defined above, by any means.

The meganuclease variant can be used either as a polypeptide or as a polynucleotide construct encoding said polypeptide. It is introduced into somatic cells of an individual, by any convenient means well-known to those in the art, which are appropriate for the particular cell type, alone or in association with either at least an appropriate vehicle or carrier and/or with the targeting DNA.

According to an advantageous embodiment of the uses according to the invention, the meganuclease variant (polypeptide) is associated with:
- liposomes, polyethyleneimine (PEI); in such a case said association is administered and therefore introduced into somatic target cells.
- membrane translocating peptides (Bonetta, The Scientist, 2002, 16, 38; Ford et al., Gene Ther., 2001, 8, 1-4 ; Wadia and Dowdy, Curr. Opin. Biotechnol., 2002, 13, 52-56); in such a case, the sequence of the variant/single-chain molecule is fused with the sequence of a membrane translocating peptide (fusion protein).

According to another advantageous embodiment of the uses according to the invention, the meganuclease (polynucleotide encoding said meganuclease) and/or the targeting DNA is inserted in a vector. Vectors comprising targeting DNA and/or nucleic acid encoding a meganuclease can be introduced into a cell by a variety of methods (e.g., injection, direct uptake, projectile bombardment, liposomes, electroporation). Meganucleases can be stably or transiently expressed into cells using expression vectors. Techniques of expression in eukaryotic cells are well known to those in the art. (See Current Protocols in Human Genetics: Chapter 12 "Vectors For Gene Therapy" & Chapter 13 "Delivery Systems for Gene Therapy"). Optionally, it may be preferable to incorporate a nuclear localization signal into the recombinant protein to be sure that it is expressed within the nucleus.

Once in a cell, the meganuclease and if present, the vector comprising targeting DNA and/or nucleic acid encoding a meganuclease are imported or translocated by the cell from the cytoplasm to the site of action in the nucleus.

For purposes of therapy, the meganucleases and a pharmaceutically acceptable excipient are administered in a therapeutically effective amount. Such a combination is said to be administered in a "therapeutically effective amount" if the amount administered is physiologically significant. An agent is physiologically significant if its presence results in a detectable change in the physiology of the recipient. In the present context, an agent is physiologically significant if its presence results in a decrease in the severity of one or more symptoms of the targeted disease and in a genome correction of the lesion or abnormality.

In one embodiment of the uses according to the present invention, the meganuclease is substantially non-immunogenic, i.e., engender little or no adverse immunological response. A variety of methods for ameliorating or eliminating deleterious immunological reactions of this sort can be used in accordance with the invention.

In a preferred embodiment, the meganuclease is substantially free of N-formyl methionine.

Another way to avoid unwanted immunological reactions is to conjugate meganucleases to polyethylene glycol ("PEG") or polypropylene glycol ("PPG") (preferably of 500 to 20,000 daltons average molecular weight (MW)). Conjugation with PEG or PPG, as described by Davis et al. (US 4,179,337) for example, can provide non-immunogenic, physiologically active, water soluble endonuclease conjugates with anti-viral activity. Similar methods also using a polyethylene--polypropylene glycol copolymer are described in Saifer et al. (US 5,006,333).

The invention also concerns a prokaryotic or eukaryotic host cell which is modified by a polynucleotide or a vector as defined above, preferably an expression vector.

The invention also concerns a non-human transgenic animal or a transgenic plant, characterized in that all or part of their cells are modified by a polynucleotide or a vector as defined above.

As used herein, a cell refers to a prokaryotic cell, such as a bacterial cell, or an eukaryotic cell, such as an animal, plant or yeast cell.

The subject-matter of the present invention is further the use of a meganuclease variant as defined above, one or two polynucleotide(s), preferably included in expression vector(s), for genome engineering (animal models generation: knock-in or knock-out), for non-therapeutic purposes.

According to an advantageous embodiment of said use, it is for inducing a double-strand break in the gene of interest, thereby inducing a DNA recombination event, a DNA loss or cell death.

According to the invention, said double-strand break is for: repairing a specific sequence, modifying a specific sequence, restoring a functional gene in place of a mutated one, attenuating or activating an endogenous gene of interest, introducing a mutation into a site of interest, introducing an exogenous gene or a part thereof, inactivating or deleting an endogenous gene or a part thereof, translocating a chromosomal arm, or leaving the DNA unrepaired and degraded.

According to another advantageous embodiment of said use, said variant, polynucleotide(s), vector are associated with a targeting DNA construct as defined above.

In a first embodiment of the use of the meganuclease variant according to the present invention, it comprises at least the following steps: 1) introducing a double-strand break at the genomic locus comprising at least one recognition and cleavage site of said meganuclease variant; 2) providing a targeting DNA construct comprising the sequence to be introduced flanked by sequences sharing homologies to the targeted locus. Said meganuclease variant can be provided directly to the cell or through an expression vector comprising the polynucleotide sequence encoding said meganuclease and suitable for its expression in the used cell. This strategy is used to introduce a DNA sequence at the target site, for example to generate knock-in or knock-out animal models or cell lines that can be used for drug testing.

The subject-matter of the present invention is also the use of at least one homing endonuclease variant, as defined above, as a scaffold for making other meganucleases. For example a third round of mutagenesis and selection/screening can be performed on said variants, for the purpose of making novel, third generation homing endonucleases.

The different uses of the homing endonuclease variant and the methods of using said homing endonuclease variant according to the present invention include also the use of the single-chain chimeric endonuclease derived from said variant, the polynucleotide(s), vector, cell, transgenic plant or non-human transgenic mammal encoding said variant or single-chain chimeric endonuclease, as defined above.

The I-*Cre*I variant according to the invention may be obtained as specified above by a method for engineering I-*Cre*I variants able to cleave a genomic DNA target sequence of interest, such as for example a DNA target sequence from a mammalian gene, comprising at least the steps of:
(a) constructing a first series of I-*Cre*I variants having at least one substitution in a first functional subdomain of the LAGLIDADG core domain situated from positions 26 to 40 of I-*Cre*I,
(b) constructing a second series of I-Crel variants having at least one substitution in a second functional subdomain of the LAGLIDADG core domain situated from positions 44 to 77 of I-*Cre*I,
(c) selecting and/or screening the variants from the first series of step (a) which are able to cleave a mutant I-*Cre*I site wherein (i) the nucleotide triplet in positions -10 to -8 of the I-*Cre*I site has been replaced with the nucleotide triplet which is present in positions -10 to -8 of said genomic target and (ii) the nucleotide triplet in positions +8 to +10 has been replaced with the reverse complementary sequence of the nucleotide triplet which is present in positions -10 to -8 of said genomic target,
(d) selecting and/or screening the variants from the second series of step (b) which are able to cleave a mutant I-*Cre*I site wherein (i) the nucleotide triplet in positions -5 to -3 of the I-*Cre*I site has been replaced with the nucleotide triplet which is present in positions -5 to -3 of said genomic target and (ii) the nucleotide triplet in positions +3 to +5 has been replaced with the reverse complementary sequence of the nucleotide triplet which is present in positions -5 to -3 of said genomic target,
(e) selecting and/or screening the variants from the first series of step (a) which are able to cleave a mutant I-*Cre*I site wherein (i) the nucleotide triplet in positions +8 to +10 of the I-*Cre*I site has been replaced with the nucleotide triplet which is present in positions +8 to +10 of said genomic target and (ii) the nucleotide triplet in positions -10 to -8 has been replaced with the reverse complementary sequence of the nucleotide triplet which is present in positions +8 to +10 of said genomic target,
(f) selecting and/or screening the variants from the second series of step (b) which are able to cleave a mutant I-*Cre*I site wherein (i) the nucleotide triplet in positions +3 to +5 of the I-*Cre*I site has been replaced with the nucleotide triplet which is present in positions +3 to +5 of said genomic target and (ii) the nucleotide triplet in positions -5 to -3 has been replaced with the reverse complementary sequence of the nucleotide triplet which is present in positions +3 to +5 of said genomic target,
(g) combining in a single variant, the mutation(s) in positions 26 to 40 and 44 to 77 of two variants from step (c) and step (d), to obtain a novel homodimeric I-*Cre*I variant which cleaves a sequence wherein (i) the nucleotide triplet in positions -10 to -8 is identical to the nucleotide triplet which is present in positions -10 to -8 of said genomic target, (ii) the nucleotide triplet in positions +8 to +10 is identical to the reverse complementary sequence of the nucleotide triplet which is present in positions -10 to -8 of said genomic target, (iii) the nucleotide triplet in positions -5 to -3 is identical to the nucleotide triplet which is present in positions -5 to -3 of said genomic target and (iv) the nucleotide triplet in positions +3 to +5 is identical to the reverse complementary sequence of the nucleotide triplet which is present in positions -5 to -3 of said genomic target, and/or
(h) combining in a single variant, the mutation(s) in positions 26 to 40 and 44 to 77 of two variants from step (e) and step (f), to obtain a novel homodimeric I-*Cre*I variant which cleaves a sequence wherein (i) the nucleotide triplet in positions +3 to +5 is identical to the nucleotide triplet which is present in positions +3 to +5 of said genomic target, (ii) the nucleotide triplet in positions -5 to - 3 is identical to the reverse complementary sequence of the nucleotide triplet which is present in positions +3 to +5 of said genomic target, (iii) the nucleotide triplet in positions +8 to +10 of the I-*Cre*I site has been replaced with the nucleotide triplet which is present in positions +8 to +10 of said genomic target and (iv) the nucleotide triplet in positions -10 to -8 is identical to the reverse complementary sequence of the nucleotide triplet in positions +8 to +10 of said genomic target,
(i) combining the variants obtained in steps (g) and (h) to form heterodimers, and
(j) selecting and/or screening the heterodimers from step (i) which are able to cleave said genomic DNA target situated in a mammalian gene.

One of the step(s) (c), (d), (e) or (f) may be omitted. For example, if step (c) is omitted, step (d) is performed with a mutant I-*Cre*I site wherein both nucleotide triplets in positions -10 to -8 and -5 to -3 have been replaced with the nucleotide triplets which are present in positions -10 to -8 and -5 to -3, respectively of said genomic target, and the nucleotide triplets in positions +3 to +5 and +8 to +10 have been replaced with the reverse complementary sequence of the nucleotide triplets which are present in positions -5 to -3 and -10 to -8, respectively of said genomic target.

Steps (a), (b), (g), and (h) may further comprise the introduction of additional mutations at other positions contacting the DNA target sequence or interacting directly or indirectly with said DNA target, at positions which improve the binding and/or cleavage properties of the mutants, or at positions which prevent the formation of functional homodimers, as defined above.

This may be performed by generating a combinatorial library as described in the International PCT Application WO 2004/067736.

The method for engineering I-*Cre*I variants of the invention advantageously comprise the introduction of random mutations on the whole variant or in a part of the variant, in particular the C-terminal half of the variant (positions 80 to 163) to improve the binding and/or cleavage properties of the mutants towards the DNA target from the gene of interest. The mutagenesis may be performed by generating random mutagenesis libraries on a pool of variants, according to standard mutagenesis methods which are well-known in the art and commercially available. Preferably, the mutagenesis is performed on the entire sequence of one monomer of the heterodimer formed in step (i) or obtained in step (j), advantageously on a pool of monomers, preferably on both monomers of the heterodimer of step (i) or (j).

Preferably, two rounds of selection/screening are performed according to the process illustrated by figure 4 of Arnould et al., J. Mol. Biol., Epub 10 May 2007. In the first round, one of the monomers of the heterodimer is mutagenised (monomer Y in figure 4), co-expressed with the other monomer (monomer X in figure 4) to form heterodimers, and the improved monomers Y⁺ are selected against the target from the gene of interest. In the second round, the other monomer (monomer X) is mutagenised, co-expressed with the improved monomers Y⁺ to form heterodimers, and selected against the target from the gene of interest to obtain meganucleases (X⁺ Y⁺) with improved activity.

The (intramolecular) combination of mutations in steps (g) and (h) may be performed by amplifying overlapping fragments comprising each of the two subdomains, according to well-known overlapping PCR techniques.

The (intermolecular) combination of the variants in step (i) is performed by co-expressing one variant from step (g) with one variant from step (h), so as to allow the formation of heterodimers. For example, host cells may be modified by one or two recombinant expression vector(s) encoding said variant(s). The cells are then cultured under conditions allowing the expression of the variant(s), so that heterodimers are formed in the host cells.

The selection and/or screening in steps (c), (d), (e), (f) and/or (j) may be performed by using a cleavage assay *in vitro* or *in vivo,* as described in the International PCT Application WO 2004/067736 or in Arnould et al., J. Mol. Biol., 2006, 355, 443-458.

According to another advantageous embodiment of said method, steps (c), (d), (e), (f) and/or (j) are performed *in vivo,* under conditions where the double-strand break in the mutated DNA target sequence which is generated by said variant leads to the activation of a positive selection marker or a reporter gene, or the inactivation of a negative selection marker or a reporter gene, by recombination-mediated repair of said DNA double-strand break.

The subject matter of the present invention is also an I-*Cre*I variant having mutations in positions 26 to 40 and/or 44 to 77 of I-*Cre*I that is useful for engineering the variants able to cleave a DNA target from a RAG gene, according to the present invention. In particular, the invention encompasses the I-*Cre*I variants as defined in step (c) to (f) of the method for engineering I-*Cre*I variants, as defined above, including the variants of Tables V, VI, VIII, IX. The invention encompasses also the I-*Cre*I variants as defined in step (g) and (h) of the method for engineering I-*Cre*I variants, as defined above, including the combined variants of Table VII and X.

Single-chain chimeric meganucleases able to cleave a DNA target from the gene of interest are derived from the variants according to the invention by methods well-known in the art (Epinat et al., Nucleic Acids Res., 2003, 31, 2952-62; Chevalier et al., Mol. Cell., 2002, 10, 895-905; Steuer et al., Chembiochem., 2004, 5, 206-13; International PCT Applications WO 03/078619 and WO 2004/031346). Any of such methods, may be applied for constructing single-chain chimeric. meganucleases derived from the variants as defined in the present invention.

The polynucleotide sequence(s) encoding the variant as defined in the present invention may be prepared by any method known by the man skilled in the art. For example, they are amplified from a cDNA template, by polymerase chain reaction with specific primers. Preferably the codons of said cDNA are chosen to favour the expression of said protein in the desired expression system.

The recombinant vector comprising said polynucleotides may be obtained and introduced in a host cell by the well-known recombinant DNA and genetic engineering techniques.

The I-*Cre*I variant or single-chain derivative as defined in the present invention is produced by expressing the polypeptide(s) as defined above; preferably said polypeptide(s) are expressed or co-expressed (in the case of the variant only) in a host cell or a transgenic animal/plant modified by one or two expression vector(s) (in the case of the variant only), under conditions suitable for the expression or co-expression of the polypeptides, and the variant or single-chain derivative is recovered from the host cell culture or from the transgenic animal/plant.

In addition to the preceding features, the invention further comprises other features which will emerge from the description which follows, which refers to examples illustrating the I-*Cre*I meganuclease variants and their uses according to the invention, as well as to the appended drawings in which:
- figure 1 represents two different strategies for restoring a functional gene by meganuclease-induced recombination. **A.** Gene correction. A mutation occurs within a known gene. Upon cleavage by a meganuclease and recombination with a repair matrix the deleterious mutation is corrected. **B.** Exonic sequences knock-in. A mutation occurs within a known gene. The mutated mRNA transcript is featured below the gene. In the repair matrix, exons located downstream of the cleavage site are fused in frame (as in a cDNA), with a polyadenylation site to stop transcription in 3'. Introns and exons sequences can be used as homologous regions. Exonic sequences knock-in results into an engineered gene, transcribed into a mRNA able to code for a functional protein.
- figure 2 illustrates the modular structure of homing endonucleases and the combinatorial approach for custom meganucleases design **A.** Tridimensional structure of the I-*Cre*I homing endonuclease bound to its DNA target. The catalytic core is surrounded by two αββαββα folds forming a saddle-shaped interaction interface above the DNA major groove. **B.** Given the separability of the two DNA binding subdomain (top left), one can combine different I-*Cre*I monomers binding different sequences derived from the I-*Cre*I target sequence (top right and bottom left) to obtain heterodimers or single chain fusion molecules cleaving non-palindromic chimeric targets (bottom right). **C.** The identification of smaller independent subunit, i.e. subunit within a single monomer or αββαββα fold (top right and bottom left) would allow for the design of novel chimeric molecules (bottom right), by combination of mutations within a same monomer. Such molecules would cleave palindromic chimeric targets (bottom right). **D.** The combination of the two former steps would allow a larger combinatorial approach, involving four different subdomains. In a first step, couples of novel meganucleases could be combined in new molecules ("half-meganucleases") cleaving palindromic targets derived from the target one wants to cleave. Then, the combination of such "half-meganuclease" can result in an heterodimeric species cleaving the target of interest. Thus, the identification of a small number of new cleavers for each subdomain would allow for the design of a very large number of novel endonucleases.
- figure 3 represents the map of the base specific interactions of I-*Cre*I with its DNA target C1221 (SEQ ID NO:1; Chevalier and Stoddard, Nucleic Acids Res., 2001, 29, 3757-74 ; Chevalier et al. J. Mol. Biol., 2003, 329, 253-69). The inventors have identified novel I-*Cre*I derived endonucleases able to bind DNA targets modified in regions -10 to -8 and +8 to +10, or -5 to -3 and +3 to +5. These DNA regions are indicated in grey boxes.
- figure 4 represents the human RAG1 gene (GeneID 5896, accession number NC_000011.8, positions 36546139 to 36557877). CDS sequences are boxed, and the CDS junctions are indicated. ORF is indicated as a grey box. The RAG1.10 site (SEQ ID NO: 151) as well as various potential meganuclease sites (RAG1.6: SEQ ID NO: 149; RAG1.7: SEQ ID NO: 150; RAG1.1: SEQ ID NO: 159, 207; RAG1.2: SEQ ID NO: 165; RAG1.5: SEQ ID NO: 167; RAG1.3: SEQ ID NO: 168; RAG1.11: SEQ ID NO: 170; RAG1.12: SEQ ID NO: 173; RAG1.9: SEQ ID NO: 175, and RAG1.4: SEQ ID NO: 176) are indicated with their sequences and positions. Examples of known deletorious mutations are indicated above the ORF.
- figure 5 represents the human RAG2 gene (GeneID 5897, accession number NC_000011.8, 36570071 to 36576362 (minus strand)). CDS sequences are boxed, and the CDS junctions are indicated. ORF is indicated as a grey box. The RAG2.8 meganuclease site is indicated with its sequence (SEQ ID NO: 184) and position. Examples of known deletorious mutations are indicated above the ORF.

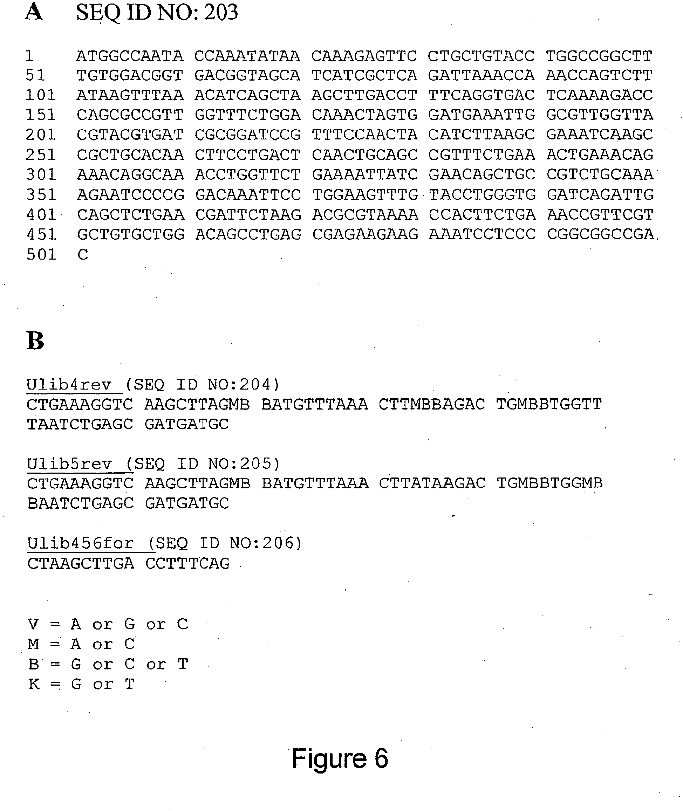

- figure 6 represents the sequences of the I-*Cre*I N75 scaffold protein and degenerated primers used for the Ulib4 and Ulib5 libraries construction. A. The scaffold (SEQ ID NO: 203) is the I-*Cre*I ORF including the D75N codon substitution and three additional codons (AAD) at the 3' end. B. Primers (SEQ ID NO: 204, 205, 206),

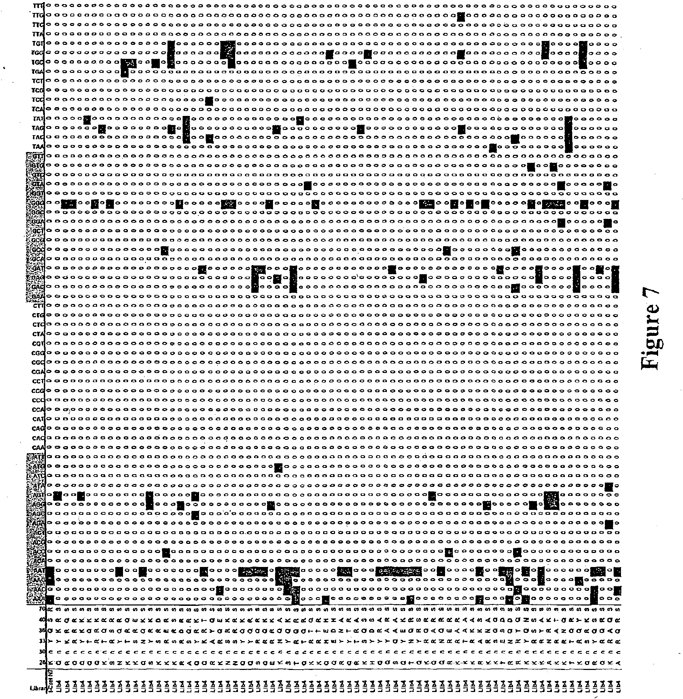

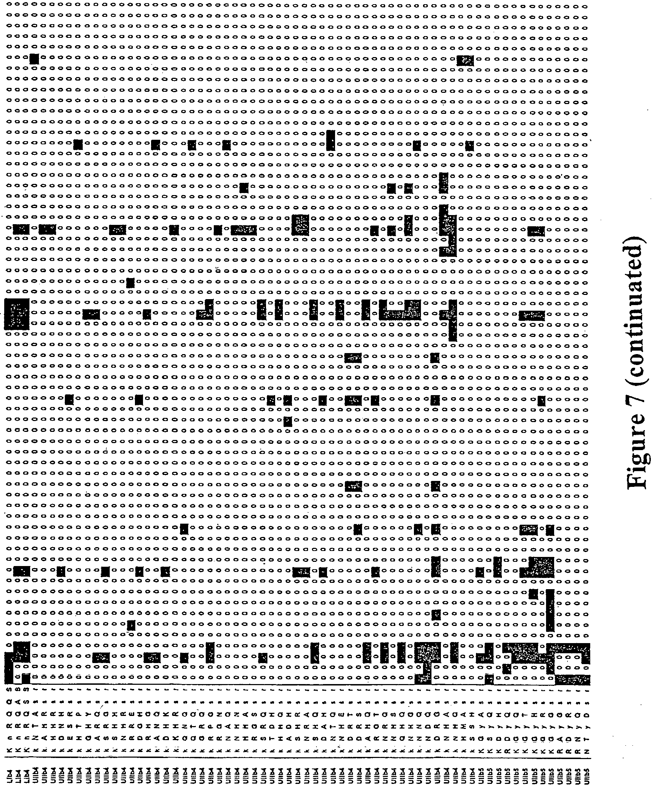

- figure 7 represents the cleavage patterns of the I-*Cre*I variants in positions 28, 30, 33, 38 and/or 40. For each of the 141 I-*Cre*I variants obtained after screening, and defined by residues in position 28, 30, 33, 38, 40, 70 and 75, cleavage was monitored in yeast with the 64 targets derived from the C1221 palindromic target cleaved by I-*Cre*I, by substitution of the nucleotides in positions ± 8 to 10.Targets are designated by three letters, corresponding to the nucleotides in position -10, -9 and -8. For example GGG corresponds to the tcgggacgtcgtacgacgtcccga target (SEQ ID NO: 207). Values correspond to the intensity of the cleavage, evaluated by an appropriate software after scanning of the filter. For each protein, observed cleavage (black box) or non observed cleavage (0) is shown for each one of the 64 targets. All the variants are mutated in position 75: D75N.

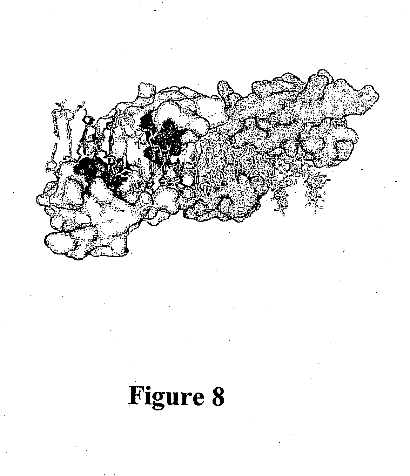

- figure 8 represents the localisation of the mutations in the protein and DNA target, on a I-*Cre*I homodimer bound to its target. The two set of mutations (residues 44, 68 and 70; residues 28, 30, 33, 38 and 40) are shown in black on the monomer on the left. The two sets of mutations are clearly distinct spatially. However, there is no structural evidence for distinct subdomains. Cognate regions in the DNA target site (region -5 to -3; region -10 to -8) are shown in grey on one half site.
- figure 9 represents the RAG1.10 series of targets and close derivatives. C1221 (SEQ ID NO: 1) is one of the I-*Cre*I palindromic target sequences. 10GTT_P, 10TGG_P, 5CAG_P and 5GAG_P (SEQ ID NO: 208 to 211) are close derivatives found to be cleaved by I-*Cre*I mutants. They differ from C1221 by the boxed motives. C1221, 10GTT_P, 10TGG_P, 5CAG_P and 5GAG_P were first described as 24 bp sequences, but structural data suggest that only the 22 bp are relevant for protein/DNA interaction. However, positions ±12 are indicated in parenthesis. RAG1.10 (SEQ ID NO: 151) is the DNA sequence located in the human RAG1 gene at position 5270. RAG1.10.2 (SEQ ID NO; 212) is the palindromic sequence derived from the left part of RAG1.10, and RAG1.10.3 (SEQ ID NO: 213) is the palindromic sequence derived from the right part of RAG1.10. The boxed motives from 10GTT_P, 10TGG_P, 5CAG_P and 5GAG_P are found in the RAG1.10 series of targets.

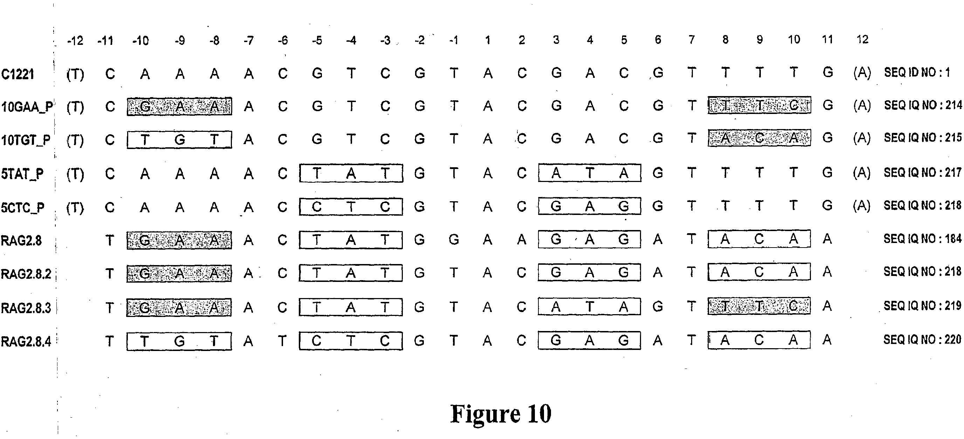

- figure 10 represents the RAG2.8 series of targets and close derivatives. C1221 (SEQ ID NO: 1) is one of the I-*Cre*I palindromic target sequences. 10GAA_P, 10TGT_P, 5TAT_P and 5CTC_P (SEQ ID NO: 214 to 217) are close derivatives found to be cleaved by I-*Cre*I mutants. They differ from C1221 by the boxed motives. C1221, 10GAA_P, 10TGT_P, 5TAT_P and 5CTC_P were first described as 24 bp sequences, but structural data suggest that only the 22 bp are relevant for protein/DNA interaction. However, positions ±12 are indicated in parenthesis. RAG2.8 (SEQ ID NO: 184) is the DNA sequence located in the human RAG2 gene at position 968. In the RAG2.8.2 target (SEQ ID NO: 218), the ttga sequence in the middle of the target is replaced with gtac, the bases found in C1221. RAG2.8.3 (SEQ ID NO: 219) is the palindromic sequence derived from the left part of RAG2.8.2, and RAG2.8.4 (SEQ ID NO: 220) is the palindromic sequence derived from the right part of RAG2.8.2. The boxed motives from 10GAA_P, 10TGT_P, 5TAT_P and 5CTC_P are found in the RAG2.8 series of targets.

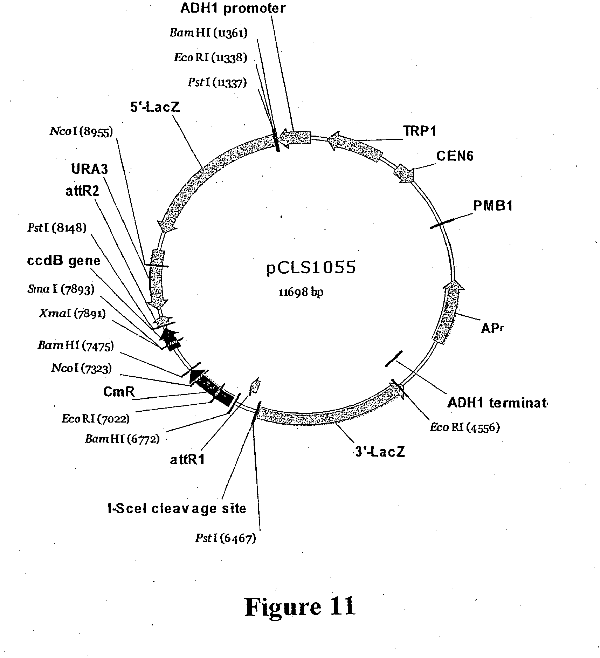

- figure 11 represents the pCLS1055 plasmid vector map.

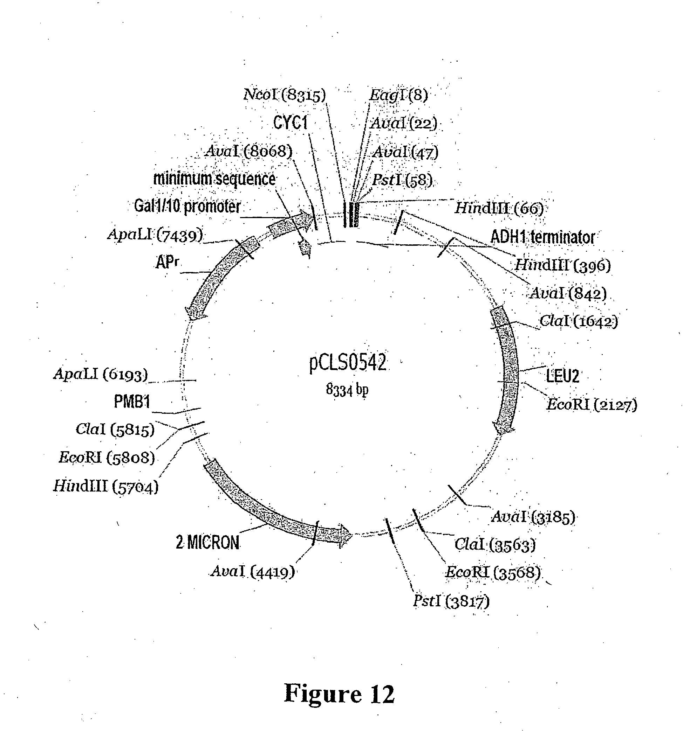

- figure 12 represents the pCLS 10542 plasmid vector map.

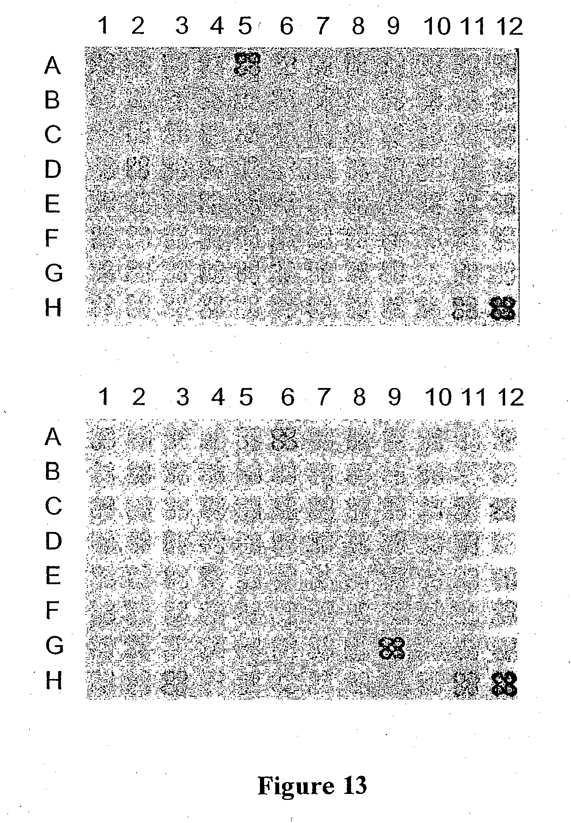

- figure 13 illustrates the cleavage of the RAG1.10.2 target by combinatorial mutants. The figure displays an example of primary screening of I-*Cre*I combinatorial mutants with the RAG1.10.2 target. H11 and H12 are positive controls of different strength. In the first filter, the sequences of positive mutants at positions A5 and D2 are KKSAQS/ASSDR and KKSSQS/AYSYK, respectively (same nomenclature as for Table V). In the second filter, the sequences of positive mutants at positions A6, G9 and H3 are respectively KRDYQS/AYSYK, KRSNQS/AYSYK and KKSGQS/AYSYK.

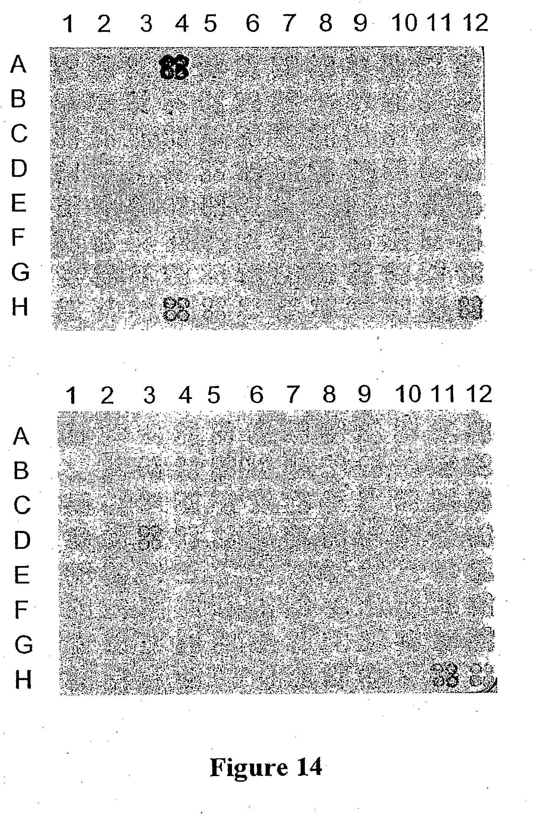

- figure 14 illustrates the cleavage of the RAG1.10.3 target by combinatorial mutants. The figure displays an example of primary screening of I-*Cre*I combinatorial mutants with the RAG1.10.3 target. H12 is a positive control. In the first filter, the sequences of positive mutants at positions A4 and H4 are KNSTAK/NYSYN and QNSSRK/AHQNI, respectively (same nomenclature as for Table VI). In the second filter, the sequences of positive mutants at position D3 and H11 are respectively NNSSRRS/TRSYI and NNSSRR/NRSYV.

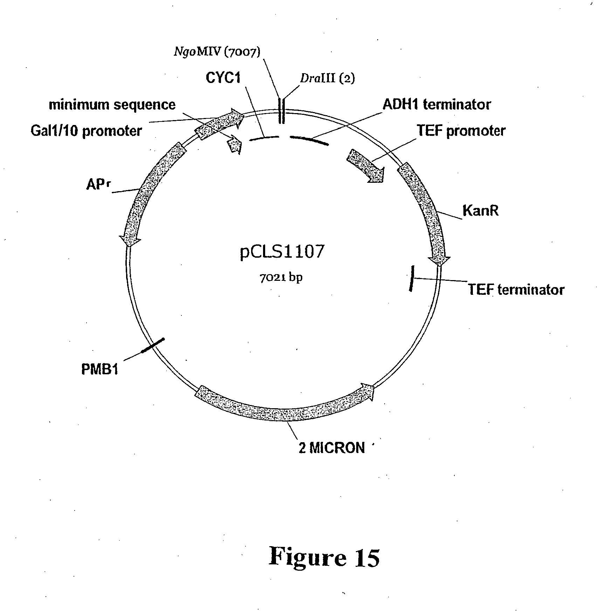

- figure 15 represents the pCLS1107 vector map.

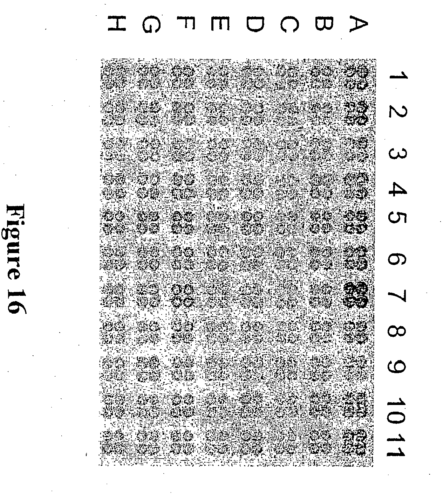

- figure 16 illustrates the cleavage of the RAG1.10 target by heterodimeric combinatorial mutants. The figure displays secondary screening of a series of combinatorial mutants among those described in Table VII.

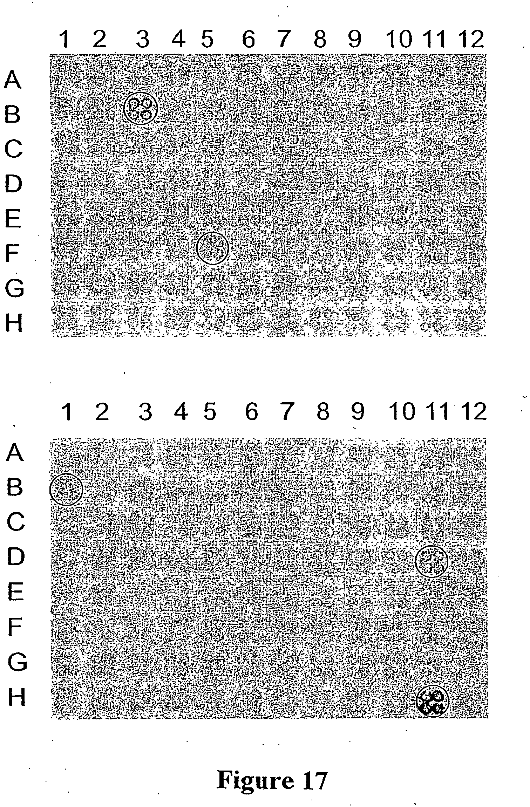

- figure 17 illustrates the cleavage of the RAG2.8.3 target by combinatorial mutants. The figure displays an example of primary screening of I-*Cre*I combinatorial mutants with the RAG2.8.3 target. In the first filter, the sequences of positive mutants at positions B3 and F5 are KNSRQQ/ATQNI and KNSRQQ/NRNNI, respectively (same nomenclature as for Table VIII). In the second filter, the sequences of positive mutants at positions B1, D11 and H11 are respectively KNSRQA/RHTNI, KRSRQQ/AKGNI and KNRSQQ/ARHNI.

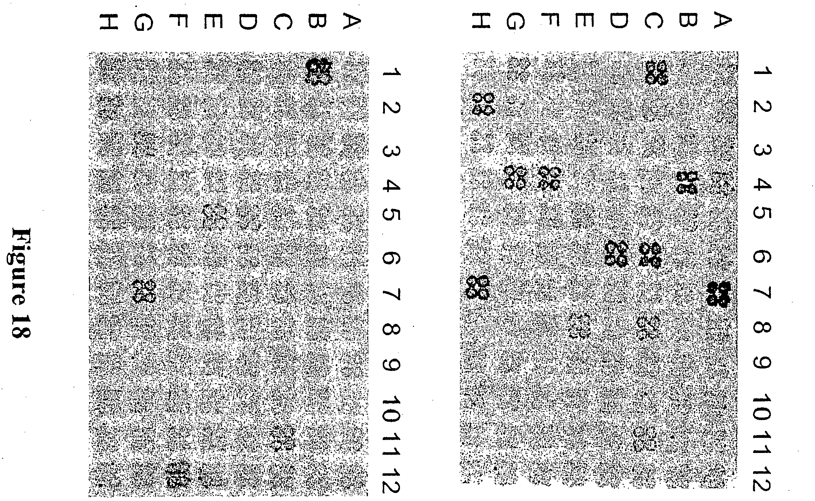

- figure 18 illustrates the cleavage of the RAG2.8.4 target by combinatorial mutants. The figure displays an example of primary screening of I-*Cre*I combinatorial mutants with the RAG2.8.4 target. In the first filter, positive mutants are NNSSRR/RYSNN (A7), NNSSRK/TRSRY 83S (B4), NNSSRR/TYSRA (C1 and H2), QNSSRK/KYSYN (C6, F4, G4 and H7), NNSSRR/TYSRV 140A (C8 and E8), NNSSRR/KYSYN (C11), NNSSRK/TYSRA (D6), and NNSSRR/TYSRA (H10), or non identified (A4 and G1) (same nomenclature as for Table IX). In the second filter, the positive mutants are KNSYQS/RYSNN (A5), NNSSRR/KYSYN 54L (B1), NNSSRR/RYSNT (C11 and G3), NNSSRR/RYSNN (D5 and G7), KNSSRS/QYSYN (E5), QNSSRK/KYSYN (F12), NNSSRK/TYSRA (H2).

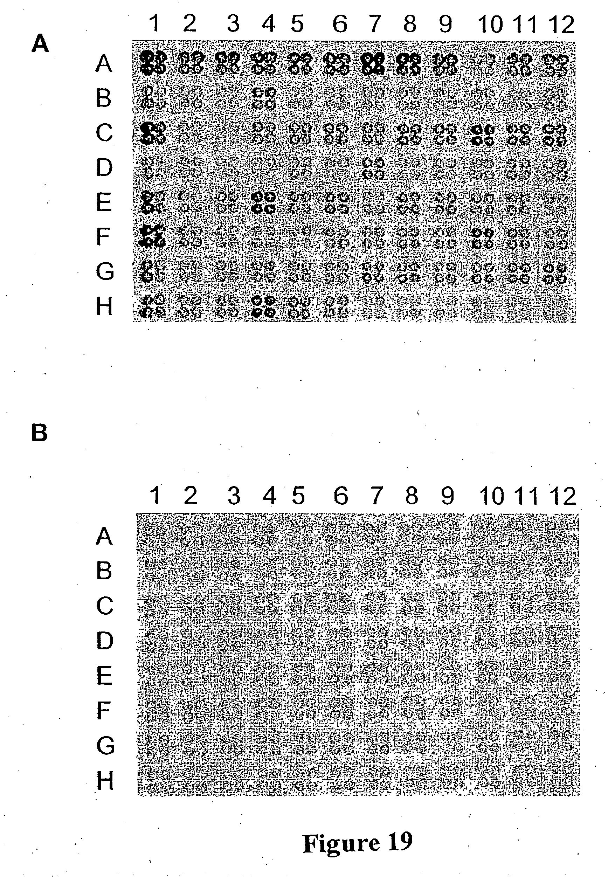

- figure 19 illustrates the cleavage of the RAG2.8.2 target by heterodimeric combinatorial mutants. A. Secondary screening of combinations of I-*Cre*I mutants with the RAG2.8.2. target. B. Secondary screening of the same combinations of I-*Cre*I mutants with the RAG2.8. target. No cleavage is oberved with this sequence.

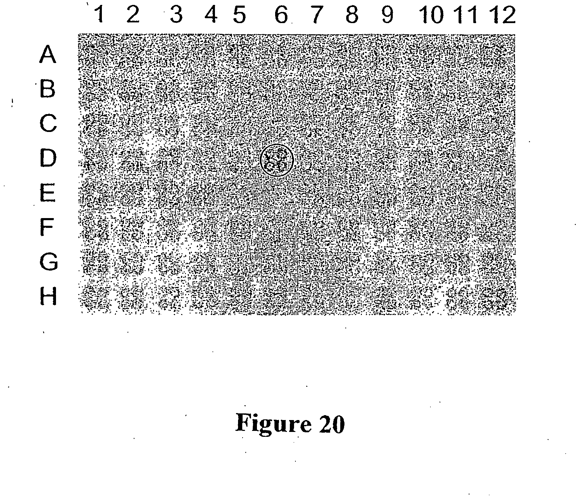

- figure 20 illustrates the cleavage of the RAG2.8 target. A series of I-*Cre*I N75 optimized mutants cutting RAG2.8.3 are coexpressed with mutants cutting RAG2.8.4 Cleavage is tested with the RAG2.8 target. A mutants cleaving RAG2.8 is circled (D6). D6 is an heterodimer resulting from the combination of two variants monomers: 33R40Q44A670N75N89A105A115T159R and 28N33S38R40K44R68Y70S75N77N. H12 is a positive control.

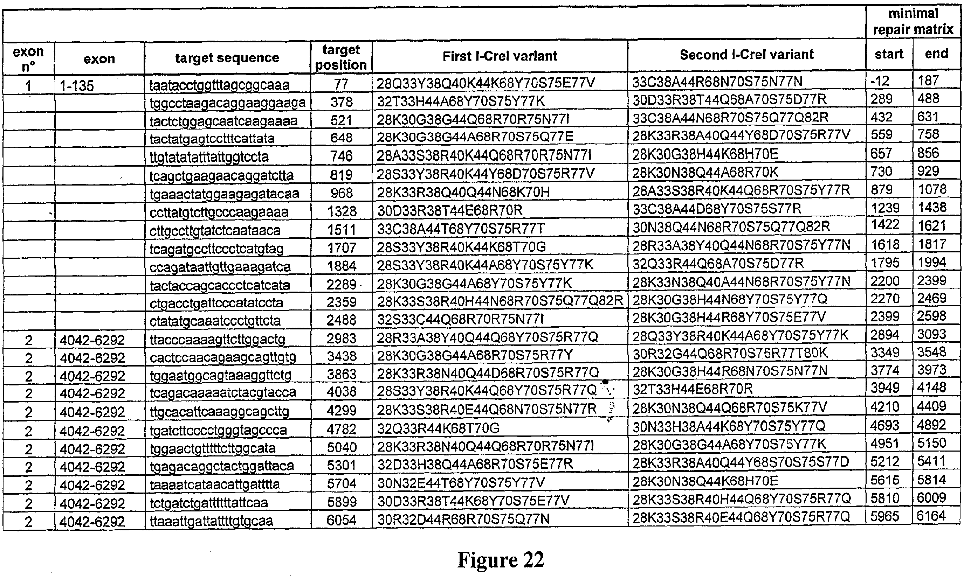

- figures 21 and 22 illustrate the DNA target sequences found in the human RAG1 and RAG2 genes and the corresponding I-*Cre*I variant which is able to cleave said DNA target. The exons closest to the target sequences, and the exons junctions are indicated (columns 1 and 2), the sequence of the DNA target is presented (column 3), with its position (column 4). The minimum repair matrix for repairing the cleavage at the target site is indicated by its first nucleotide (start, column 7) and last nucleotide (end, column 8). The sequence of each variant is defined by its amino acid residues at the indicated positions. For example, the first heterodimeric variant of figure 21 consists of a first monomer having Q, R, K, Y, E, S, R, V in positions 28, 38, 40, 44, 68, 70, 75 and 77, respectively and a second monomer having R, Q, K, T, S, N and V in positions 30, 32, 44, 68, 70, 75 and 77, respectively. The positions are indicated by reference to I-*Cre*I sequence SWISSPROT P05725 or pdb accession code lg9y; I-*Cre*I has K, N, S, Y, Q, S, Q, R, R, D, I, E and K, in positions 28, 30, 32, 33, 38, 40, 44, 68, 70, 75, 77, 80 and 82, respectively. The positions which are not indicated are not mutated and thus correspond to the wild-type I-*Cre*I sequence.

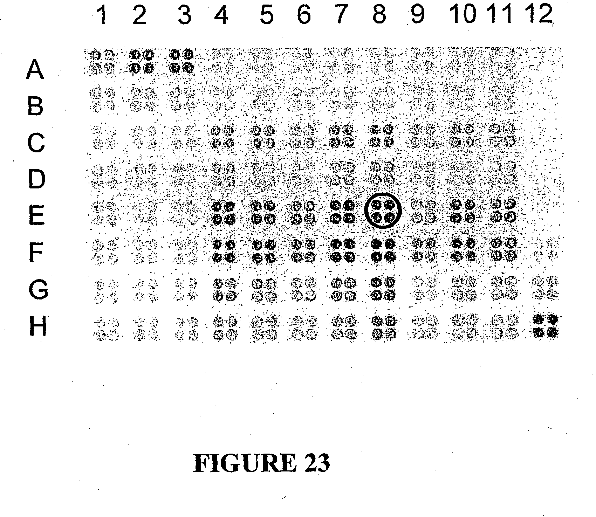

- figure 23 illustrates cleavage of the RAG2.8 target with optimized mutants in yeast. A series of I-*Cre*I derivatives cleaving the RAG2.8.3 sequence (identified in example 9) were co-expressed with a new series of I-*Cre*I mutants, obtained by random mutagenesis of mutants cleaving the RAG2.8.4 target. Cleavage of the RAG2.8 target is monitored in yeast using a functional assay described previously (Arnould et al., 20,06, J. Mol. Biol. 355, 443-458), and is revealed here by blue staining of the yeasts. This Figure features a series of mutants identified during a former primary screen. These mutants were rearrayed, and each mutant is tested in four different dots in a same cluster. The circled mutant (E8) corresponds to the 33R, 40Q, 44A, 70N, 75N/132V vs 28N, 33S, 38R, 40K, 44R, 68Y, 70S, 75Y, 77N/49A, 87L heterodimer described in Table XII. G12 and H12 are positive controls (I-*Sce*I meganuclease with I-*Sce*I target), F12 is a negative control (no meganuclease).

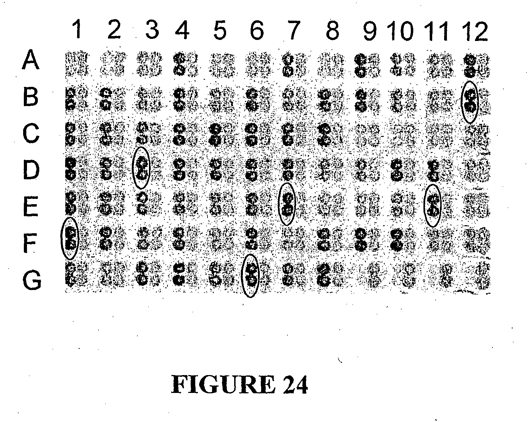

- figure 24 illustrates cleavage of the RAG1.10 target by co-expression of the KHSMAS/ARSYT mutant cleaving RAG1.10.3, and randomly mutagenized mutants cleaving RAG1.10.2. The figure displays the secondary screening of the 80 rearranged mutants (wells A1 to G8). In each four dots cluster, the two left dots corresponds to randomly mutagenized mutants, whereas the two right dots correspond to the non mutagenized KRSNQS/RYSDT protein identified in example 3 as a RAG1.10.2 cleaver (see Table V). The six mutants described in the Table XIII are circled.

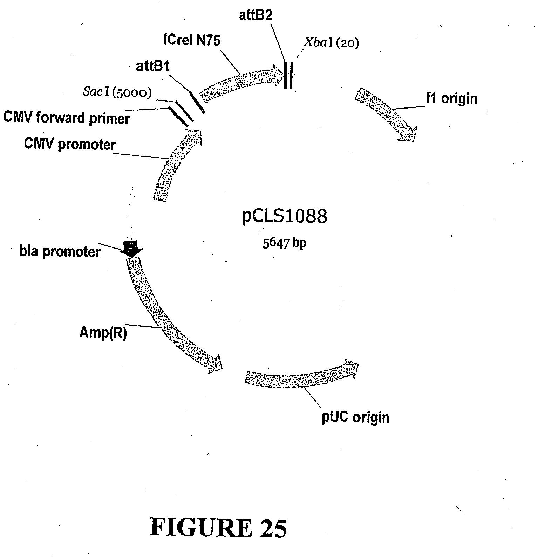

- figure 25 represents the map of pCLS1088, a plasmid for expression of I-*Cre*I N75 in mammalian cells.

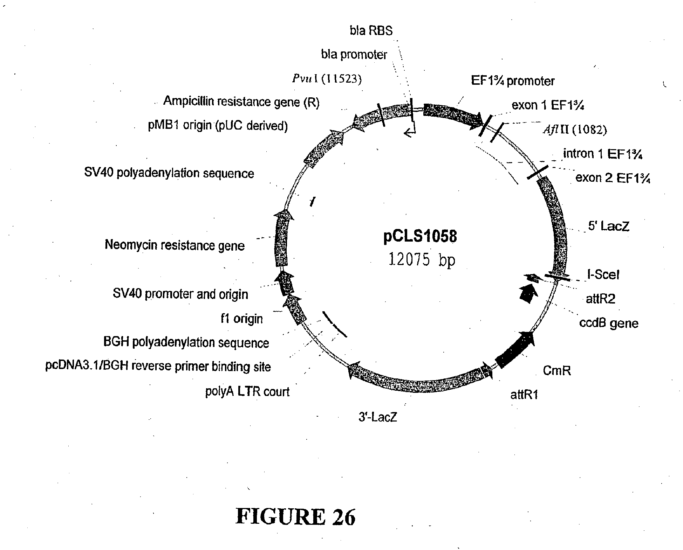

- figure 26 represents the map of pCLS1058, a plasmid for gateway cloning of DNA targets in a reporter vector for mammalian cells.

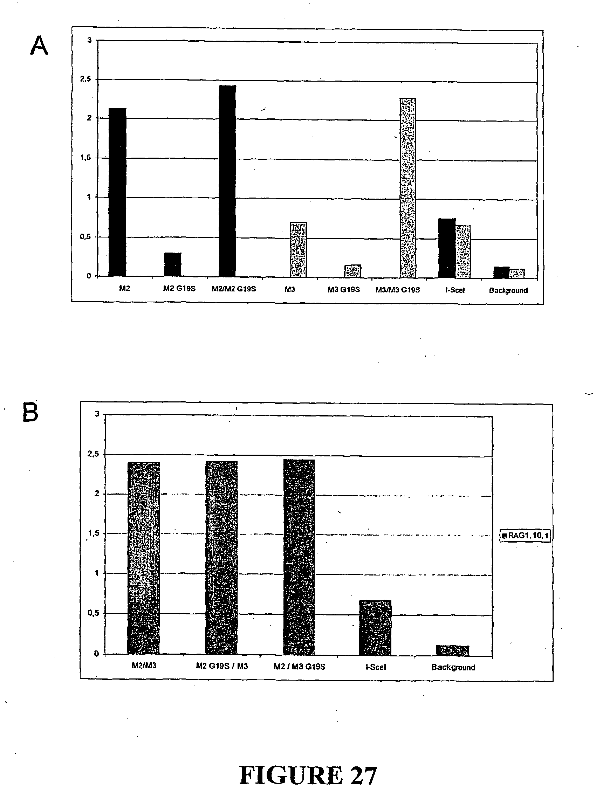

- figure 27 illustrates cleavage of the RAG1.10, RAG1.10.2 and RAG1.10.3 targets by M2 and M3 I-*Cre*I mutants with or without the G19S mutation in an extrachromosomal assay in CHO cells. The cleavage of the palindromic targets RAG1.10.2 and RAG1.10.3 is shown in panel A, while RAG1.10 cleavage is by heterodimeric meganucleases is shown in panel B. Cleavage of I-*Sce*I target by I-*Sce*I in the same experiments is shown as positive control.

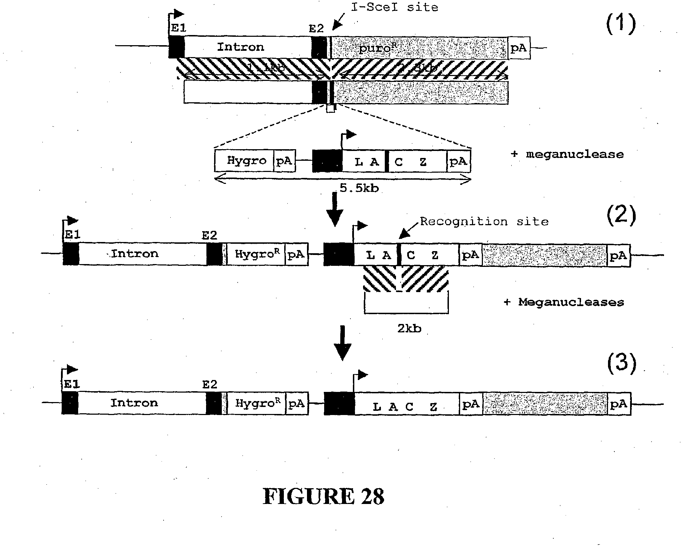

- figure 28 illustrates the design of reporter system in mammalian cells. The puromycin resistance gene, interrupted by an I-*Sce*I cleavage site 132bp downstream of the start codon, is under the control of the EFIa promoter (1). The transgene has been stably expressed in CHO-K1 cells in single copy. In order to introduce meganuclease target sites in the same chromosomal context, the repair matrix is composed of i) a promoterless hygromycin resistance gene, ii) a complete lacZ expression cassette and iii) two arms of homologous sequences (1.1 kb and 2.3 kb). Several repair matrixes have been constructed differing only by the recognition site that interrupts the lacZ gene (2). Thus, very similar cell lines have been produced as A1 cell line, I-*Sce*I cell line and I-*Cre*I cell line. A functional lacZ gene is restored when a lacZ repair matrix (2kb in length) is co-transfected with vectors expressing a meganuclease cleaving the recognition site (3). The level of meganuclease-induced recombination can be inferred from the number of blue colonies or foci after transfection.

### Example 1: Engineering of I-CreI variants with modified specificity in positions ±8 to ±10

The method for producing meganuclease variants and the assays based on cleavage-induced recombination in mammal or yeast cells, which are used for screening variants with altered specificity, are described in the International PCT Application WO 2004/067736 and in Arnould et al., J. Mol. Biol., 2006, 355, 443-458. These assays result in a functional LacZ reporter gene which can be monitored by standard methods.

### A) Material and methods

### a) Construction of the Ulib4, Ulib5 and Lib4 libraries

I-*Cre*I wt and I-*Cre*I D75N open reading frames were synthesized, as described previously (Epinat et al., N.A.R., 2003, 31, 2952-2962). Mutation D75N was introduced by replacing codon 75 with aac. Three combinatorial libraries (Ulib4, Ulib5 and Lib4) were derived from the I-*Cre*I D75N protein by replacing three different combinations of residues, potentially involved in the interactions with the bases in positions ± 8 to 10 of one DNA target half-site. The diversity of the meganuclease libraries was generated by PCR using degenerated primers harboring a unique degenerated codon (coding for 10 or 12 different amino acids), at each of the selected positions.

The three codons at positions N30, Y33 and Q38 (Ulib4 library) or K28, N30 and Q38 (Ulib5 library) were replaced by a degenerated codon VVK (18 codons) coding for 12 different amino acids: A,D,E,G,H,K,N,P,Q,R,S,T). In consequence, the maximal (theoretical) diversity of these protein libraries was 12³ or 1728. However, in terms of nucleic acids, the diversity was 18³ or 5832. Fragments carrying combinations of the desired mutations were obtained by PCR, using a pair of degenerated primers (Ulib456for and Ulib4rev; Ulib456for and Ulib5rev, figure 6B) and as DNA template, the D75N open reading frame (ORF), (figure 6A). The corresponding PCR products were cloned back into the I*-Cre*I N75 ORF in the yeast replicative expression vector pCLS0542 (Epinat et al., precited and figure 12), carrying a *LEU2* auxotrophic marker gene. In this 2 micron-based replicative vector, I*-Cre*I variants are under the control of a galactose inducible promoter.

In Lib4, ordered from BIOMETHODES, an arginine in position 70 was first replaced with a serine (R70S). Then positions 28, 33, 38 and 40 were randomized. The regular amino acids (K28, Y33, Q38 and S40) were replaced with one out of 10 amino acids (A,D,E,K,N,Q,R,S,T,Y). The resulting library has a theoretical complexity of 10000 in terms of proteins.

### b) Construction of target clones

The C1221 twenty-four bp palindrome (tcaaaacgtcgtacgacgttttga, (SEQ ID NO: 1) is a repeat of the half-site of the nearly palindromic natural I*-Cre*I target (tcaaaacgtcgtgagacagtttgg, SEQ ID NO: 221). C1221 is cleaved as efficiently as the I*-Cre*I natural target *in vitro* and *ex vivo* in both yeast and mammalian cells.

The 64 palindromic targets were derived from C1221 as follows: 64 pairs of oligonucleotides ((ggcatacaagtttcnnnacgtcgtacgacgtnnngacaatcgtctgtca (SEQ ID NO: 222) and reverse complementary sequences) were ordered form Sigma, annealed and cloned into pGEM-T Easy (PROMEGA) in the same orientation. Next, a 400 bp *Pvu*II fragment was excised and cloned into the yeast vector pFL39-ADH-LACURAZ, also called pCLS0042, and the mammalian vector pcDNA3 derivative, both described previously (Epinat et al., 2003, precited), resulting in 64 yeast reporter vectors (target plasmids).

Alternatively, double-stranded target DNA, generated by PCR amplification of the single stranded oligonucleotides, was cloned using the Gateway protocol (INVITROGEN) into yeast and mammalian reporter vectors.

### c) Yeast strains

The library of meganuclease expression variants was transformed into the *leu2* mutant haploid yeast strain FYC2-6A: alpha, trp1Δ63, leu2Δ1, his3Δ200. A classical chemical/heat choc protocol that routinely gives us 10⁶ independent transformants per µg of DNA derived from (Gietz and Woods, Methods Enzymol., 2002, 350, 87-96), was used for transformation. Individual transformant (Leu⁺) clones were individually picked in 96 wells microplates.13824 colonies were picked using a colony picker (QpixII, GENETIX), and grown in 144 microtiter plates.

The 64 target plasmids were transformed using the same protocol, into the haploid yeast strain FYBL2-7B: a, ura3Δ851, trp1Δ63, leu2Δ1, lys2Δ202, resulting in 64 tester strains.

### d) Mating of meganuclease expressing clones and screening in yeast

Meganuclease expressing clones were mated with each of the 64 target strains, and diploids were tested for beta-galactosidase activity, by using the screening assay illustrated on figure 2 of Arnould et al., 2006, precited. I*-Cre*I variant clones as well as yeast reporter strains were stocked in glycerol (20%) and replicated in novel microplates. Mating was performed using a colony gridder (QpixII, GENETIX). Mutants were gridded on nylon filters covering YPD plates, using a high gridding density (about 20 spots/cm²). A second gridding process was performed on the same filters to spot a second layer consisting of 64 different reporter-harboring yeast strains for each variant. Membranes were placed on solid agar YPD rich medium, and incubated at 30°C for one night, to allow mating. Next, filters were transferred to synthetic medium, lacking leucine and tryptophan, with galactose (1%) as a carbon source (and with G418 for coexpression experiments), and incubated for five days at 37 °C, to select for diploids carrying the expression and target vectors. After 5 days, filters were placed on solid agarose medium with 0.02% X-Gal in 0.5 M sodium phosphate buffer, pH 7.0, 0.1% SDS, 6% dimethyl formamide (DMF), 7 mM β-mercaptoethanol, 1% agarose, and incubated at 37 °C, to monitor β-galactosidase activity. After two days of incubation, positive clones were identified by scanning. The β-galactosidase activity of the clones was quantified using appropriate software. The clones showing an activity against at least one target were isolated (first screening). The spotting density was then reduced to 4 spots/cm² and each positive clone was tested against the 64 reporter strains in quadruplicate, thereby creating complete profiles (secondary screening).

### e) Sequence

The open reading frame (ORF) of positive clones identified during the first and/or secondary screening in yeast was amplified by PCR on yeast colonies using primers: PCR-Gal10-F (gcaactttagtgctgacacatacagg, SEQ ID NO: 223) and PCR-Gal10-R (acaaccttgattgcagacttgacc, SEQ ID NO: 224) from PROLIGO. Briefly, yeast colony is picked and resuspended in 100 µl of LGlu liquid medium and cultures overnight. After centrifugation, yeast pellet is resuspended in 10 µl of sterile water and used to perform PCR reaction in a final volume of 50 µl containing 1.5 µl of each specific primers (100 pmol/µl). The PCR conditions were one cycle of denaturation for 10 minutes at 94 °C, 35 cycles of denaturation for 30 s at 94 °C, annealing for 1 min at 55 °C, extension for 1.5 min at 72 °C, and a final extension for 5 min. The resulting PCR products were then sequenced.

### f) Re-Cloning of primary hits

The open reading frames (ORFs) of positive clones identified during the primary screening were recloned using the Gateway protocol (Invitrogen). ORFs were amplified by PCR on yeast colonies, as described in e). PCR products were then cloned in : (i) yeast gateway expression vector harboring a galactose inducible promoter, *LEU2* or KanR as selectable marker and a 2 micron origin of replication, and (ii) a pET 24d(+) vector from NOVAGEN. Resulting clones were verified by sequencing (MILLEGEN).

### B) Results

I*-Cre*I is a dimeric homing endonuclease that cleaves a 22 bp pseudo-palindromic target. Analysis of I*-Cre*I structure bound to its natural target has shown that in each monomer, eight residues establish direct interactions with seven bases (Jurica et al., Mol. Cell. Biol., 1998, 2, 469-476). According to these structural data, the bases of the nucleotides in positions ± 8 to 10 establish specific contacts with I*-Cre*I amino-acids N30, Y33 and Q38 (Figure 3). Thus, novel proteins with mutations in positions 30, 33 and 38 could display novel cleavage profiles with the 64 targets resulting from substitutions in positions ± 8, ± 9 and ± 10 of a palindromic target cleaved by I*-Cre*I. In addition, mutations might alter the number and positions of the residues involved in direct contact with the DNA bases. More specifically, positions other than 30, 33, 38, but located in the close vicinity on the folded protein, could be involved in the interaction with the same base pairs.

An exhaustive protein library vs. target library approach was undertaken to engineer locally this part of the DNA binding interface. First, the I*-Cre*I scaffold was mutated from D75 to N. The D75N mutation did not affect the protein structure, but decreased the toxicity of I*-Cre*I in overexpression experiments.

Next the Ulib4 library was constructed: residues 30, 33 and 38, were randomized, and the regular amino acids (N30, Y33, and Q38) replaced with one out of 12 amino acids (A,D,E,G,H,K,N,P,Q,R,S,T). The resulting library has a complexity of 1728 in terms of protein (5832 in terms of nucleic acids).

Then, two other libraries were constructed: Ulib5 and Lib4. In Ulib5, residues 28, 30 and 38 wee randomized, and the regular amino acids (K28, N30, and Q38) replaced with one out of 12 amino acids (ADEGHKNPQRST). The resulting library has a complexity of 1728 in terms of protein (5832 in terms of nucleic acids). In Lib4, an Arginine in position 70 was first replaced with a Serine. Then, positions 28, 33, 38 and 40 were randomized, and the regular amino acids (K28, Y33, Q38 and S40) replaced with one out of 10 amino acids (A,D,E,K,N,Q,R,S,T,Y). The resulting library has a complexity of 10000 in terms of proteins.

In a primary screening experiment, 20000 clones from Ulib4, 10000 clones from Ulib5 and 20000 clones from Lib4 were mated with each one of the 64 tester strains, and diploids were tested for beta-galactosidase activity. All clones displaying cleavage activity with at least one out of the 64 targets were tested in a second round of screening against the 64 targets, in quadriplate, and each cleavage profile was established. Then, meganuclease ORF were amplified from each strain by PCR, and sequenced, and 141 different meganuclease variants were identified.

The 141 validated clones showed very diverse patterns. Some of these new profiles shared some similarity with the wild type scaffold whereas many others were totally different. Results are summarized in Figure 7. Homing endonucleases can usually accommodate some degeneracy in their target sequences, and the I*-Cre*I N75 scaffold protein itself cleaves a series of 4 targets, corresponding to the aaa, aac, aag, an aat triplets in positions ±10 to ±8. A strong cleavage activity is observed with aaa, aag and aat, whereas aac is only faintly cut (and sometimes not observed). Similar pattern is found with other proteins, such as I*-Cre*I K28, N30, D33, Q38, S40, R70 and N75, I*-Cre*I K28, N30, Y33, Q38, S40, R70 and N75. With several proteins, such as I-CreI R28, N30, N33, Q38, D40, S70 and N75 and I*-Cre*I K28, N30 N33, Q38, S40, R70 and N75, aac is not cut anymore.

However, a lot of proteins display very different patterns. With a few variants, cleavage of a unique sequence is observed. For example, protein I*-Cre*I K28, R30, G33, T38, S40, R70 and N75 is active on the "ggg" target, which was not cleaved by wild type protein, while I*-Cre*I Q28, N30, Y33, Q38, R40, S70 and N75 cleaves aat, one of the targets cleaved by I*-Cre*I N75. Other proteins cleave efficiently a series of different targets: for example, I*-Cre*I N28, N30, S33, R38, K40, S70 and N75 cleaves ggg, tgg and tgt, *Cre*I K28, N30, H33, Q38, S40, R70 and N75 cleaves aag, aat, gac, gag, gat, gga, ggc, ggg, and ggt. The number of cleaved sequences ranges from 1 to 10. Altogether, 37 novel targets were cleaved by the mutants, including 34 targets which are not cleaved by I*-Cre*I and 3 targets which are cleaved by I*-Cre*I (aag, aat and aac, figure 7).

### Example 2: Strategy for engineering novel meganucleases cleaving a target from the RAG1 or RAG2 genes

A first series of I*-Cre*I variants having at least one substitution in positions 44, 68, 70, 75 and/or 77 of I*-Cre*I and being able to cleave mutant I*-Cre*I sites having variation in positions ± 3 to 5 was identified as described previously (Arnould et al., J. Mol. Biol., 2006, 355, 443-458).

A second series of I*-Cre*I variants having at least one substitution in positions 28, 30, 33 or 28, 33, 38 and 40 of I*-CreI* and being able to cleave mutant I-*CreI* sites having variation in positions ± 8 to 10 was identified as described in example 1. The cleavage pattern of the variants is presented in Figure 7.

Positions 28, 30, 33, 38 and 40 on one hand, and 44, 68 and 70, on another hand are on a same DNA-binding fold, and there is no structural evidence that they should behave independently. However, the two sets of mutations are clearly on two spatially distinct regions of this fold (Figure 8) located around different regions of the DNA target. These data suggest that I-*Cre*I comprises two independent functional subunits which could be combined to cleave novel chimeric targets. The chimeric target comprises the nucleotides in positions ± 3 to 5 and ± 8 to 10 which are bound by each subdomain.

This hypothesis was verified by using targets situated in a gene of interest, the RAG gene. The targets cleaved by the I*-Cre*I variants are 24 bp derivatives of C1221, a palindromic sequence cleaved by I-*Cre*I. However, the structure of I*-Cre*I bound to its DNA target suggests that the two external base pairs of these targets (positions -12 and 12) have no impact on binding and cleavage (Chevalier et al., Nat. Struct. Biol., 2001, 8, 312-316; Chevalier B.S. and B.L. Stoddard, Nucleic Acids Res., 2001, 29, 3757-3574; Chevalier et al., J. Mol. Biol., 2003, 329, 253-269) and in this study, only positions -11 to 11 were considered. Consequently, the series of targets identified in the RAG1 and RAG2 genes were defined as 22 bp sequences instead of 24 bp.

### 1) RAG1.10

RAG1.10 is a 22 bp (non-palindromic) target (Figure 9) located at position 5270 of the human RAG1 gene (accession number NC_000011.8, positions 836546139 to 36557877), 7 bp upstream from the coding exon of RAG1 (Figure 4).

The meganucleases cleaving RAG1.10 could be used to correct mutations in the vicinity of the cleavage site (Figure 1A). Since the efficiency of gene correction decreases when the distance to the DSB increases (Elliott et al., Mol. Cell. Biol., 1998, 18, 93-101), this strategy would be most efficient with mutations located within 500 bp of the cleavage site. Alternatively, the same meganucleases could be used to knock-in exonic sequences that would restore a functional RAG1 gene at the RAG1 locus (Figure 1B). This strategy could be used for any mutation downstream of the cleavage site.

RAG1.10 is partly a patchwork of the 10GTT_P, 10TGG_P and 5CAG_P and 5GAG_P targets (Figure 9) which are cleaved by previously identified meganucleases (Figure 7). Thus, RAG1.10 could be cleaved by combinatorial mutants resulting from these previously identified meganucleases.

Therefore, to verify this hypothesis, two palindromic targets, RAG1.10.2 and RAG1.10.3 were derived from RAG1.10 (Figure 9). Since RAG1.10.2 and RAG1.10.3 are palindromic, they should be cleaved by homodimeric proteins. In a first step, proteins able to cleave RAG1.10.2 and RAG1.10.3 sequences as homodimers were designed (examples 3 and 4). In a second step, the proteins obtained in examples 3 and 4 were co-expressed to obtain heterodimers cleaving RAG1.10 (example 5).

### 2) RAG2.8

RAG2.8 is a 22 bp (non-palindromic) target (Figure 10) located at position 968 of the human RAG2 gene (accession number NC_000011.8, complement of 36576362 to 36570071), in the beginning of the intron of RAG2 (Figure 5).

The meganucleases cleaving RAG2.8 could be used knock-in exonic sequences that would restore a functional RAG2 gene at the RAG2 locus (Figure 1B). This strategy could be used for any mutation downstream of the cleavage site (Figure 5).

RAG2.8 is partly a patchwork of the 10GAA_P, 10TGT_P and 5TAT_P and 5CTC_P targets (Figure 10) which are cleaved by previously identified meganucleases (Figure 7). Thus, RAG1.10 could be cleaved by combinatorial mutants resulting from these previously identified meganucleases.

In contrast with RAG1.10, RAG2.8 differs from C1221 in the 4 bp central region. According to the structure of the I*-Cre*I protein bound to its target, there is no contact between the 4 central base pairs (positions -2 to 2) and the I-*Cre*I protein (Chevalier et al., Nat. Struct. Biol., 2001, 8, 312-316; Chevalier B.S. and B.L. Stoddard, Nucleic Acids Res., 2001, 29, 3757-3574; Chevalier et al., J. Mol. Biol., 2003, 329, 253-269). Thus, the bases at these positions are not supposed to impact the binding efficiency. However, they could affect cleavage, which results from two nicks at the edge of this region. Thus, the ggaa sequence in -2 to 2 was first substituted with the gtac sequence from C1221, resulting in target RAG2.8.2. Then, two palindromic targets, RAG2.8.3 and RAG2.8.4, were derived from RAG2.8.2. Since RAG2.8.3 and RAG2.8.4 are palindromic, they should be cleaved by homodimeric proteins. In a first step, proteins able to cleave the RAG2.8.3 and RAG2.8.4 sequences as homodimers were designed, (examples 6 and 7) and then coexpressed them to obtain heterodimers cleaving RAG2.8 (example 8). In this case, no heterodimer was found to cleave the RAG2.8 target. A series of mutants cleaving RAG2.8.3 or RAG2.8.4 was chosen, and then refined. The chosen mutants were randomly mutagenized, and used to form novel heterodimers that were screened against the RAG2.8 target (example 9 and 10). Heterodimers cleaving the RAG2.8 target could be identified, displaying significant cleavage activity.

### Example 3: Making of meganucleases cleaving RAG1.10.2

This example shows that I*-Cre*I mutants can cut the RAG1.10.2 DNA target sequence derived from the left part of the RAG1.10 target in a palindromic form (Figure 9). Target sequences described in this example are 22 bp palindromic sequences. Therefore, they will be described only by the first 11 nucleotides, followed by the suffix _P, solely to indicate that (For example, target RAG1.10.2 will be noted also tgttctcaggt_P; SEQ ID NO: 212).

RAG1.10.2 is similar to 5CAG_P in positions ±1, ±2, ±3, ±4, ±5 and ±11 and to 10GTG_P in positions ±1, ±2, ±8, ±9 and ±10. It was hypothesized that positions ±6, ±7 and ±11 would have little effect on the binding and cleavage activity. Mutants able to cleave 5CAG_P (caaaaccaggt_P; SEQ ID NO: 210) were previously obtained by mutagenesis on I-*Cre*I at positions 44, 68, 70, 75, and 77, as decribed in Arnould et al., J. Mol. Biol., 2006, 355, 443-458. Mutants able to cleave the 10GTT_P target (cgttacgtcgt_P) were obtained by mutagenesis on I*-Cre*I N75 and D75 at positions 28, 30, 32, 33, 38, 40 (example 1 and figure 7). Thus, combining such pairs of mutants would allow for the cleavage of the RAG1.10.2 target.

Both sets of proteins are mutated at position 70. However, it was hypothesized that two separable functional subdomains exist in I*-Cre*I. That implies that this position has little impact on the specificity in bases 10 to 8 of the target.

Therefore, to check whether combined mutants could cleave the RAG1.10.2 target, mutations at positions 44, 68, 70, 75 and 77 from proteins cleaving 5CAG_P were combined with the 30, 32, 33, 38 and 40 mutations from proteins cleaving 10GTG_P.

### A) Material and Methods

### a) Construction of target vector

The target was cloned as follows: oligonucleotide corresponding to the target sequence flanked by gateway cloning sequence was ordered from Proligo (as example: 5' tggcatacaagttttgttctcaggtacctgagaacaacaatcgtctgtca 3' (SEQ ID NO: 225), for the RAG1.10.2 target). Double-stranded target DNA, generated by PCR amplification of the single stranded oligonucleotide, was cloned using the *Gateway^{R}* protocol (INVITROGEN) into yeast reporter vector (pCLS1055, Figure 11). Yeast reporter vector was transformed into S. *cerevisiae* strain FYBL2-7B (MAT alpha, ura3Δ851, trp1Δ63, leu2Δ1, lys2Δ202).

### b) Construction of combinatorial mutants

I*-Cre*I mutants cleaving 10GTG_P or 5CAG_P were identified as described in example 1 and figure 7, and Arnould et al., J. Mol. Biol., 2006, 355, 443-458, respectively for the 10TGC_P and the 5TTT_P targets. In order to generate I-*Cre*I derived coding sequence containing mutations from both series, separate overlapping PCR reactions were carried out that amplify the 5' end (amino acid positions 1-43) or the 3' end (positions 39-167) of the I-*Cre*I coding sequence. For both the 5' and 3' end, PCR amplification is carried out using Gal10F or Gal10R primers, specific to the vector (pCLS0542, figure 12), and primers specific to the I-*Cre*I coding sequence for amino acids 39-43 (assF 5'-ctannnttgaccttt-3' (SEQ ID NO: 226) or assR 5'-aaaggtcaannntag-3' (SEQ ID NO: 227)) where nnn codes for residue 40. The PCR fragments resulting from the amplification reaction realized with the same primers and with the same coding sequence for residue 40 were pooled. Then, each pool of PCR fragments resulting from the reaction with primers Gal10F and assR or assF and Gal10R was mixed in an equimolar ratio. Finally, approximately 25 ng of each of the two overlapping PCR fragments and 25 ng of vector DNA (pCLS0542) linearized by digestion with *Nco*I and *Eag*I were used to transform the yeast *Saccharomyces cerevisiae* strain FYC2-6A (MATα, trp1Δ63, leu2Δ1, his3Δ200) using a high efficiency LiAc transformation protocol (Gietz and Woods, Methods Enzymol, 2002, 350, 87-96). An intact coding sequence containing both groups of mutations is generated by *in vivo* homologous recombination in yeast.

### c) Mating of meganuclease expressing clones and screening in yeast:

The experimental procedure is as described in example 1, except that a low gridding density (about 4 spots/cm²) was used.

### d) Sequencing of mutants

To recover the mutant expressing plasmids, yeast DNA was extracted using standard protocols and used to transform *E*. *coli.* Sequencing of mutant ORF was then performed on the plasmids by MILLEGEN SA. Alternatively, ORFs were amplified from yeast DNA by PCR (Akada et al., Biotechniques, 2000, 28, 668-670) and sequencing was performed directly on PCR product by MILLEGEN SA

### B) Results

I*-Cre*I combinatorial mutants were constructed by associating mutations at positions 44, 68, 70, 75 and 77 with the 30, 33, 38 and 40 mutations on the I*-Cre*I N75 or D75 scaffold, resulting in a library of complexity 1300. Examples of combinations are displayed on Table V. This library was transformed into yeast and 2300 clones (1.8 times the diversity) were screened for cleavage against RAG1.10.2 DNA target (tgttctcaggt_P; SEQ ID NO: 212). 64 positives clones were found, which after sequencing and validation by secondary screening turned out to correspond to 32 different novel endonucleases (Table V). Examples of positives are shown in Figure 13.

**Table V: Cleavage of the RAG1.10.2 target by the combined variants***

| Amino acids at positions 44, 68, 70, 75 and 77 (AYSYK stands for A44, Y68, S70, Y75 and K77) | Amino acids at positions 28, 30, 32, 33, 38 and 40 (KRSNQS stands for K28, R30, S32, N33, Q38 and S40) | | | | | | | | | |
|---|---|---|---|---|---|---|---|---|---|---|
| | KRSNQS | KKSAQS | KRSCQS | KNSRTS | KKSGQS | KRDYQS | KNSHGS | KSSCQS | KKSSQS | KTSGQS |
| AYSYK | + | + | + | + | + | + | + | + | + | |
| ASSDR | | + | | + | | + | | | + | |
| RYSDT | + | + | + | + | | | | | + | |
| TYSYR | + | + | + | + | | | | | + | |
| KYSYN | + | | | | | + | | | | |
| ARNNI | | | | | | | | | | |
| ARDNI | | | | | | | | | | |
| ARENI | | | | | | | | | | |
| ARHNI | | | | | | | | | | |
| NRSYN | | | | | | | | | | |
| AESYK | | | | | | | | | | |
| ATSDR | | | | | | | | | | |
| NYSYK | + | | + | | | | | | | |
| NYSYR | + | + | + | | | + | | | | |
| QASDR | | | | | | | | | | |
| TRSYY | | | | | | | | | | |
| AASYK | | | | | | | | | | |
| SYSYV | | | | | | | | | | |
| NRGNI | | | | | | | | | | |
| NESRR | | | | | | | | | | |
| NRNNI | | | | | | | | | | |
| RRENI | | | | | | | | | | |
| AHQNI | | | | | | | | | | |
| AASDR | + | | | | | | | | | |
| RYSDQ | | | | | | | | | | |

| | | | | | | | | | | |
|---|---|---|---|---|---|---|---|---|---|---|
| *Only 250 out of the 1300 combinations are displayed. + indicates a functional combination. | | | | | | | | | | |

### Example 4: Making of meganucleases cleaving RAG1.10.3

This example shows that I*-Cre*I variants can cleave the RAG1.10.3 DNA target sequence derived from the right part of the RAG1.10.1 target in a palindromic form (Figure 9). All target sequences described in this example are 22 bp palindromic sequences. Therefore, they will be described only by the first 11 nucleotides, followed by the suffix _P; for example, RAG1.10.3 will be called ttggctgaggt_P; SEQ ID NO: 213.

RAG1.10.3 is similar to 5GAG_P in positions ±1, ±2, ±3, ±4, ±5 and ±7 and to 10TGG_P in positions ±1, ±2, ±7, ±8, ±9 and ±10. It was hypothesized that positions ±6 and ±11 would have little effect on the binding and cleavage activity. Mutants able to cleave 5GAG_P were previously obtained by mutagenesis on I*-Cre*I at positions 44, 68, 70, 75 and 77, as described in Arnould et al., J. Mol. Biol., 2006, 355, 443-458. Mutants able to cleave the 10GTG_P target were obtained by mutagenesis on I-*Cre*I N75 and D75 at positions 28, 30, 32, 33, 38, 40 and 70, as described in example 1 (Figure 7). Therefore, combining such pairs of mutants would allow for the cleavage of the RAG1.10.3 target.

Both sets of proteins are mutated at position 70. However, it was hypothesized that I*-Cre*I comprises two separable functional subdomains. That implies that this position has little impact on the specificity in base 10 to 8 of the target. Therefore, to check whether combined mutants could cleave the RAG1.10.3 target, mutations at positions 44, 68, 70, 75 and 77 from proteins cleaving 5GAG_P (caaaacgaggt_P; SEQ ID NO: 210) were combined with the 28, 30, 32, 33, 38, 40 mutations from proteins cleaving 10TGG_P (ctggacgtcgt_P; SEQ ID NO: 209).

### A) Material and Methods

See example 3.

### B) Results

I-*Cre*I combinatorial mutants were constructed by associating mutations at positions 44, 68, 70, 75 and 77 with the 28, 30, 33, 38 and 40 mutations on the I*-Cre*I N75 or D75 scaffold, resulting in a library of complexity 1215. Examples of combinatorial mutants are displayed on Table VI. This library was transformed into yeast and 2300 clones (1.9 times the diversity) were screened for cleavage against RAG1.10.3 DNA target (ttggctgaggt_P; SEQ ID NO: 213). 88 positives clones were found, which after sequencing and validation by secondary screening turned out to be correspond to 27 different novel endonucleases (see Table VI). Examples of positives are shown in Figure 14.

**Table VI: Cleavage of the RAG1.10.3 target by the combined variants***

| Amino acids at positions 44, 68, 70, 75 and 77 (KGA stands for K44, G68 and A70) | Amino acids at positions 28, 30, 32, 33, 38 and 40 (ANSSRK stands for A28, N30, S32, S33, R38 and K40) | | | | | | | | | |
|---|---|---|---|---|---|---|---|---|---|---|
| | ANSSRK | NNSSRR | QNSSRK | KNGTQS | KNDCQS | KRSQQS | KHSMAS | KNRWQS | KNSTAA | KNATQS |
| ARTNI | | + | | | | | | | | |
| AHQNI | | | + | | | | + | | | |
| ARSNI | | | + | | | | | | | |
| NRANI | | | | | | | | | | |
| NRNNI | | + | | | | | | | | |
| ARSYT | | | | | | | + | | | |
| YRSYQ | | + | + | | | | | | | |
| YRSQV | | + | | | | | + | | | |
| TRSYI | + | + | + | | | | | | | |
| AHHNI | | | + | | | | | | | |
| YNSNV | | | + | | | | | | | |
| SRSYT | | | | | | | + | | | |
| NHSYN | | | | | | | | | | |
| NRSYI | | | | | | | | | | |
| QTNNI | | | | | | | | | | |
| TRSNI | | | | | | | | | | |
| DRANI | | | | | | | | | | |
| DNSNI | | | | | | + | | | | |
| YRSDV | | | | | | | | | | |
| ARSYI | | | | | | | | | + | |
| AQANI | | | | | | | | + | | |
| ARNNI | + | + | | | | | + | | | |
| AANNI | + | + | + | | | | | | | |
| NRSYV | | + | | | | | | | | |
| ARSYQ | | | | | + | | | | | |

| | | | | | | | | | | |
|---|---|---|---|---|---|---|---|---|---|---|
| *Only 250 out of the 1215 combinations are displayed + indicates a functional combination | | | | | | | | | | |

### Example 5: Making of meganucleases cleaving RAG1.10

I*-Cre*I mutants able to cleave each of the palindromic RAG1.10 derived targets (RAG1.10.2 and RAG1.10.3) were identified in examples 3 and 4. Pairs of such mutants (one cutting RAG1.10.2 and one cutting RAG1.10.3), were co-expressed in yeast. Upon co-expression, there should be three active molecular species, two homodimers, and one heterodimer. It was assayed whether the heterodimers that should be formed cut the RAG1.10 target.

### A) Material and Methods

### a) Cloning of mutants in kanamycin resistant vector

In order to co-express two I*-Cre*I mutants in yeast, mutants cutting the RAG1.10.2 sequence were subcloned in a kanamycin resistant yeast expression vector (pCLS1107, Figure 15).

Mutants were amplified by PCR reaction using primers common for leucine vector (pCLS0542) and kanamycin vector (pCLS1107) (Gal10F and Gal10R). Approximately 25ng of PCR fragment and 25ng of vector DNA (pCLS1107) linearized by digestion with *DraIII* and *NgoMIV* are used to transform the yeast *Saccharomyces cerevisiae* strain FYC2-6A (MATα, trp1Δ63, leu2Δ1, his3Δ200) using a high efficiency LiAc transformation protocol. An intact coding sequence for the I*-Cre*I mutant is generated *by in vivo* homologous recombination in yeast.

### b) Mutants coexpression:

Yeast strain expressing a mutant cutting the RAG1.10.3 target was transformed with DNA coding for a mutant cutting the RAG1.10.2 target in pCLS1107 expression vector. Transformants were selected on -L Glu + G418 medium.

### c) Mating of meganucleases coexpressing clones and screening in yeast:

The experimental procedure is as described in example 1, except that a low gridding density (about 4 spots/cm²) was used.

### B) Results

Coexpression of mutants cleaving the RAG1.10.2 and RAG1.10.3 resulted in efficient cleavage of the RAG1.10 target in most cases (Figure 16). Functional combinations are summarized in Table VII.

**Table VII: Combinations that resulted in cleavage of the RAG1.10 target**

| Mutants cutting RAG1.10.3 amino acids at positions 28,30, 32, 33, 38, 40 / 44 68 70 75 77 (NNSSRR/ARTNI stands for N28, N30, S32, S33, R38, R401 A44, R68, T70, N75 and 177) | Mutants cutting RAG1.10.2 amino acids at positions 28, 30, 32, 33, 38, 40 / 44, 68, 70, 75 and 77 (KKSAQS/AYSYK stands for K28, K30, S32, A33, Q38, S40/ A44, Y68, S70, Y75 and K77) | | | | | | | |
|---|---|---|---|---|---|---|---|---|
| | KKSAQS/ AYSYK | KKSAQS/ ASSDR | KKSAQS/ RYSDT | KRSNQS/ TYSYR | KRSCQS/ AYSYK | KRSNQS/ KYSYN | KNSRTSI AYSYK | KKSGQS/ AYSYK |
| NNSSRR/ ARTNI | | | | | + | | | |
| NNSSRR/ YRSQV | + | + | + | + | + | + | + | + |
| NNSSRR/ ARNNI | + | + | | + | + | + | + | + |
| QNSSRK/ AHQNI | + | + | | + | + | + | + | + |
| KHSMAS/ ARSYT | + | | | | | | | |
| NNSSRR/ YRSYQ | + | + | + | + | + | + | + | + |
| NNSSRRI NRSYV | + | + | | + | + | + | + | |
| QNSSRK/ AANNI | + | + | | + | + | + | + | |

| | | | | | | | | |
|---|---|---|---|---|---|---|---|---|
| + indicates a functional combination | | | | | | | | |

### Example 6: Making of meganucleases cleaving RAG2.8.3

This example shows that I-*Cre*I mutants can cut the RAG2.8.3 DNA target sequence derived from the left part of the RAG2.8.2 target in a palindromic form (Figure 10). Target sequences described in this example are 22 bp palindromic sequences. Therefore, they will be described only by the first 11 nucleotides, followed by the suffix _P, for example, target RAG2.8.3 will be noted also tgaaactatgt_P; SEQ ID NO: 219.

RAG2.8.3 is similar to 5TAT_P in positions ±1, ±2, ±3, ±4, ±5, ±6, ±7, ±8 and ±9 and to 10GAA_P in positions ±1, ±2, ±6, ±7, ±8, ±9, and ±10. Mutants able to cleave 5TAT_P were previously obtained by mutagenesis on I-*Cre*I at positions 44, 68, 70, 75 and 77, as described in Arnould et al., J. Mol. Biol., 2006, 355, 443-458. Mutants able to cleave the 10GAA_P target were obtained by mutagenesis on I*-Cre*I N75 at positions 28, 30, 33, 38, 40 and 70, (example 1 and figure 7). Thus, combining such pairs of mutants would allow for the cleavage of the RAG2.8.3 target.

Both sets of proteins are mutated at position 70. However, it was hypothesized that two separable functional subdomains exist in I*-Cre*I. That implies that this position has little impact on the specificity in base 10 to 8 of the target. Therefore, to check whether combined mutants could cleave the RAG2.8.3 target, mutations at positions 44, 68, 70, 75 and 77 from proteins cleaving 5TAT_P (caaaaccctgt_P)were combined with the 28, 30, 33, 38 and 40 mutations from proteins cleaving 10GAA_P (cgaaacgtcgt_P).

### A) Material and Methods

See example 3.

### B) Results

I-*Cre*I combinatorial mutants were constructed by associating mutations at positions 44, 68, 70, 75 and 77 with the 28, 30, 33, 38 and 40 mutations on the I-*Cre*I scaffold, resulting in a library of complexity 648 (see Table VIII). This library was transformed into yeast and 1728 clones (2.7 times the diversity) were screened for cleavage against the RAG2.8 DNA target (tgaaactatgt_P; SEQ ID NO: 184). 24 positives clones were found, and after sequencing and validation by secondary screening, 11 combinatorial mutants listed in Table VIII were identified. Mutants with additional mutations were also identified, such as KNWGQS/QRRDI, KNESQS/QRRDI and KNRPQS/QRRDI (Table X). Such mutants likely result from PCR artefacts during the combinatorial process (see materials and methods). Examples of positives are shown in Figure 17.

**Table VIII: Cleavage of the RAG2.8.3 target by the combined variants***

| Amino acids at positions 44, 68, 70, 75 and 77 (ANENI stands for A44, N68, E70, N75 and I77) | Amino acids at positions 28, 30, 32, 33, 38 and 40 (KNSRQA stands for K28, N30, S32, R33, Q38 and S40) | | | | | | | | |
|---|---|---|---|---|---|---|---|---|---|
| | KNSRQS | KNSRQA | KNSRAQ | KNSRQQ | ANSRQR | SNSRQR | TNSRQR | KNSHQS | KNSRQY |
| AAKNI | | | | | | | | | |
| AANNI | | | | | | | | | |
| AASYK | | | | | | | | | |
| AESYK | | | | | | | | | |
| AGNNI | | | | | | | | | |
| AGRNI | | | | | | | | | |
| AHANI | | | | | | | | | |
| AHHNI | | | | | | | | | |
| AHQNI | | | | | | | | | |
| AHRNI | | | | | | | | | |
| AKANI | | | | | | | | | |
| AKENI | | | | | | | | | |
| AKGNI | | | | + | | | | | |
| AKKNI | | | | | | | | | |
| AKSYV | | | | | | | | | |
| ANENI | | | | | | | | | |
| ANHNI | | | | | | | | | |
| ANNNI | | | | | | | | | |
| ANSNI | | | | | | | | | |
| AQANI | | | | | | | | | |
| AQGNI | | | | | | | | | |
| AQHNI | | | | | | | | | |
| AQNNI | | | | | | | | | |
| ARANI | | | | + | | | | | |
| ARGNI | | | | | | | | | |
| ARHNI | | | | + | | | | | |
| ARLNI | | | | + | | | | | |
| ARNNI | | | | + | | | | | |
| ARRNI | | | | | | | | | |
| ARTNI | | | | | | | | | |
| ASGNI | | | | | | | | | |
| ASHNI | | | | | | | | | |
| ASRNI | | | | | | | | | |
| ASSYK | | | | | | | | | |
| ATANI | | | | | | | | | |
| ATENI | | | | | | | | | |
| ATNNI | | | | + | | | | | |
| ATQNI | | | | + | | | | | |
| AYSRT | | | | | | | | | |
| DRANI | | | | | | | | | |
| DRNNI | | | | | | | | | |
| ERHNI | | | | | | | | | |
| HATNI | | | | | | | | | |
| HRDNI | | | | | | | | | |
| KDANI | | | | | | | | | |
| KEGNI | | | | | | | | | |
| KRDNI | | | | | | | | | |
| KRQNI | | | | | | | | | |
| KYSYN | | | | | | | | | |
| NRANI | | | | + | | | | | |
| NRENI | | | | | | | | | |
| NRGNI | | | | | | | | | |
| NRNNI | | | | + | | | | | |
| NSGNI | | | | | | | | | |
| NTKNI | | | | | | | | | |
| QRSNI | | | | | | | | | + |
| QSANI | | | | | | | | | |
| QSHNI | | | | | | | | | |
| QTRNI | | | | | | | | | |
| RAGNI | | | | | | | | | |
| RHTNI | | + | | | | | | | |
| SRRNI | | | | | | | | | |
| THHNI | | | | | | | | | |
| THRNI | | | | | | | | | |
| TRENI | | | | | | | | | |
| TRQNI | | | | | | | | | |
| TRSNI | | | | | | | | | |
| TYSYR | | | | | | | | | |
| VRANI | | | | | | | | | |
| YASRI | | | | | | | | | |
| YRSNV | | | | | | | | | |
| YYSNQ | | | | | | | | | |

### Example 7: Making of meganucleases cleaving RAG2.8.4

This example shows that I-*Cre*I variants can cleave the RAG2.8.4 DNA target sequence derived from the right part of the RAG2.8.2 target in a palindromic form (Figure 10). All target sequences described in this example are 22 bp palindromic sequences. Therefore, they will be described only by the first 11 nucleotides, followed by the suffix_P, solely to indicate that (for example, RAG2.8.4 will be called t**tgtatctc**gt_P; SEQ ID NO: 220).

RAG2.8.4 is similar to 5CTC_P in positions ±1, ±2, ±3, ±4, ±5 and ±7 and to 10TGT_P in positions ±1, ±2, ±3, ±4, ±7, ±8, ±9 and ±10. It was hypothesized that positions ±6 and ±11 would have little effect on the binding and cleavage activity. Mutants able to cleave 5CTC_P (caaaac**ctc**gt_P; SEQ ID NO: 217) were previously obtained by mutagenesis on I-*Cre*I N75 at positions 44, 68, 70, 75 and 77, as described in Arnould et al., J. Mol. Biol., 2006, 355, 443-458. Mutants able to cleave the 10TGT_P target (c**tgt**acgtcgt_P; SEQ ID NO: 215) were obtained by mutagenesis on I-*Cre*I N75 at positions 28, 33, 3 8, 40 and 70, as described in example 1 (Figure 7). Therefore, combining such pairs of mutants would allow for the cleavage of the RAG2.8.4 target.

Both sets of proteins are mutated at position 70. However, it was hypothesized that I-*Cre*I comprises two separable functional subdomains. That implies that this position has little impact on the specificity in base 10 to 8 of the target. Therefore, to check whether combined mutants could cleave the RAG2.8.4 target, mutations at positions 44, 68, 70, 75 and 77 from proteins cleaving 5CTC_P were combined with the 28, 33, 38 and 40 mutations from proteins cleaving 10TGT_P (Table IX).

### A) Material and Methods

See example 3.

### B) Results

I-*Cre*I mutants used in this example, and cutting the 10TGT_P target or the 5CTC_P target are listed in Table IX. I-*Cre*I combined mutants were constructed by associating mutations at positions 44, 68, 70, 75 and 77 with the 28, 33, 38 and 40 mutations on the I-*Cre*I scaffold (Table IX), resulting in a library of complexity 290. This library was transformed into yeast and 1056 clones (3.6 times the diversity) were screened for cleavage against the RAG2.8.4 DNA target (**ttgt**at**ctc**gt_P; SEQ ID NO: 220). 105 positives clones were found, and after sequencing and validation by secondary screening 29 combinatorial mutants were identified (Table IX). Mutants with additional mutations were also identified, such as:
- NNSSRR/KYSNN (Table X)
- KNPPQS/QRRDI (Table X)-
- KNRWQS/QRRDI (Table X)
- KNSYQS/RYSNN (Figure 18)
- KNSSRS/QYSYN (Figure 18)
- NNSSRK/TRSRY 83S (Figure 18)
- NNSSRR/TYSRV 140A (Figure 18)
- NNSSRR/KYSYN 54L (Figure 18)

Such mutants likely result from PCR artefacts during the combinatorial process (see materials and methods). Example of positives are shown on Figure 18.

**Table IX: Cleavage of the RAG2.8.4 target by the combined variants***

| Amino acids at positions 44, 68, 70, 75 and 77 (AYSRV stands for A44, Y68, S70, R75 and V77) | Amino acids at positions 28, 30, 32, 33, 38 and 40 (ANRK stands for A28, N30, S32, N33, R38 and K40) | | | | |
|---|---|---|---|---|---|
| | ANSNRK | NNSSRR | NNSSRK | QNSSRK | KNSSRS |
| ARDNI | | | | | |
| ARSRY | | | | | |
| ASSDR | | | | | |
| AYSNT | | | | | |
| AYSRV | | | | | |
| HASRY | | | | | |
| HRDNI | | | | | |
| HRENI | | | | | |
| KANNI | | | | | |
| KASNI | | | | | |
| KATNI | | | | | |
| KGSNI | | | | | |
| KNANI | | | | | |
| KNDNI | | | | | |
| KNQNI | | | | | |
| KNTNI | | | | | |
| KQSNI | | | | | |
| KRANI | | | | | |
| KRDNI | | | + | + | |
| KRGNI | | | | | |
| KRNNI | | | | | |
| KRQNI | | | | | |
| KRTNI | | | | | |
| KSNNI | | | | | |
| KSSNI | | | | | |
| KSTNI | | | | | |
| KTANI | | | | | |
| KTQNI | | | | | |
| KTSNI | | | | + | |
| KYSNI | | + | + | + | |
| KYSYN | + | + | + | + | + |
| NESRK | | | | | |
| NESRR | | | | | |
| NHNNI | | | | | |
| NYSRV | | | | | |
| QHHNI | | | | | |
| QRQNI | | | | | |
| QRSYR | | | | | |
| RASNI | | | | + | |
| RATNI | | | | | |
| RNSNI | | | | | |
| RNSNN | | | | | |
| RRNNI | | | | | |
| RRSNI | | | | | |
| RRTNI | | | | | |
| RSGNI | | | | | |
| RSSNN | | | | | |
| RSTNI | | | | | |
| RYSNI | | + | | | + |
| RYSNN | | + | + | | + |
| RYSNT | + | + | + | + | |
| SYSRI | | | | | |
| TRRNI | | | | | |
| TRSRS | | | | | |
| TRSRY | | + | + | + | |
| TYSRA | | + | + | + | + |
| TYSRQ | | + | | | + |
| TYSRV | | + | | | |

| | | | | | |
|---|---|---|---|---|---|
| + indicates a functional combination | | | | | |

### Example 8: Making of meganucleases cleaving RAG2.8. 2

I-*Cre*I mutants able to cleave each of the palindromic RAG2.8 derived targets (RAG2.8.3 and RAG2.8.4) were identified in examples 6 and 7). Pairs of such mutants in yeast (one cutting RAG2.8.3 and one cutting RAG2.8.4) were co-expressed in yeast. Upon coexpression, there should be three active molecular species, two homodimers, and one heterodimer. It was assayed whether the heterodimers that should be formed cut the RAG2.8 and RAG2.8.2 targets.

### A) Material and Methods

See example 5.

### B) Results

Coexpression of mutants cleaving the RAG2.8.3 and RAG2.8.4 resulted in efficient cleavage of the RAG2.8.2 target in most cases (Figure 19). As a general rule, functional heterodimers cutting RAG2.8.2 sequence were always obtained when the two expressed proteins gave a strong signal as homodimer (Figure 19). However, none of these combinations was able to cut the RAG2.8 natural target that differs from the RAG2.8.2 sequence just by by 3 bp in positions -1, 1 and 2. (Figure 10). Functional combinations are summarized in Table X.

**Table X: Combinations that resulted in cleavage of the RAG2.8.2 target**

| **Mutants cutting RAG2.8.4, amino acids at positions 28, 30, 32, 33, 38, 40 / 44 68 70 75 77 NNSSRR/TYSRQ stands for N28, N30, S32 , S33, R38, R401 T44, Y68, S70, R75 and Q77** | **Mutants cutting RAG2.8.3 amino acids at positions 28, 30, 32, 33, 38, 40 / 44, 68, 70, 75 and 77 (KNSRQY/QRSNI stands for K28, N30, S32, R33, Q38, Y40/ Q44, R68, S70, N75 and 177)** | | | | | | | | |
|---|---|---|---|---|---|---|---|---|---|
| | KNSRQY/ QRSNI | KNSRQQ/ NRNNI | KNSRQQ/ ARANI | KNSRQQ/ ARNNI | KNSRQQ/ ATQNI | KNSRQQ/ ARHNI | KNWGQS/ QRRDI | KNESQS/ QRRDI | KNRPQS/ QRRDI |
| NNSSRR/ TYSRQ | + | + | + | + | + | + | | + | + |
| QNSSRK/ KYSYN | + | + | + | + | + | + | + | + | + |
| **NNSSRR/ KYSNN** | + | + | + | + | + | + | + | + | + |
| QNSSRK/ TRSRY | + | | + | | + | + | + | + | + |
| **KNPPQS/ QRRDI** | + | | + | + | + | + | + | + | + |
| **KNRWQS/ QRRDI** | + | + | + | + | + | + | + | + | + |
| QNSSRK/ RYSNT | + | + | + | + | + | + | + | + | + |
| NNSSRK/ RYSNN | + | | + | + | + | + | + | + | + |
| NNSSRK/ RYSNT | + | | | + | + | + | + | + | + |

| | | | | | | | | | |
|---|---|---|---|---|---|---|---|---|---|
| *Mutants in bold are mutants with unexpected mutations in examples 6 and 7. ** + indicates a functional combination | | | | | | | | | |

### Example 9: Making of meganucleases cleaving RAG2.8 by random mutagenesis of proteins cleaving RAG2.8.3 and assembly with proteins cleaving RAG2.8.4

I-*Cre*I mutants able to cleave the non palindromic RAG2.8.2 target have been identified by assembly of mutants cleaving the palindromic RAG2.8.3 and RAG2.8.4 target (example 8). However, none of these combinations was able to cleave RAG2.8, which differs from RAG2.8.2 only by 3 bp in positions -1, 1 and 2.

Therefore, the protein combinations cleaving RAG2.8.2 were mutagenized, and variants cleaving RAG2.8 were screened. According to the structure of the I-*Cre*I protein bound to its target, there is no contact between the 4 central base pairs (positions -2 to 2) and the I-*Cre*I protein (Chevalier et al., Nat. Struct. Biol., 2001, 8, 312-316; Chevalier B.S. and B.L. Stoddard, Nucleic Acids Res., 2001, 29, 3757-3574; Chevalier et al., J. Mol. Biol., 2003, 329, 253-269). Thus, it is difficult to rationally choose a set of positions to mutagenize, and mutagenesis was done on the C-terminal part of the protein (83 last amino acids) or on the whole protein. Random mutagenesis results in high complexity libraries. Therefore, to limit the complexity of the variants libraries to be tested, only one of the two components of the heterodimers cleaving RAG2.8.2 was mutagenized.

Thus, in a first step, proteins cleaving RAG2.8.3 were mutagenized, and in a second step it was assessed whether they could cleave RAG2.8 when coexpressed with proteins cleaving RAG2.8.4.

### A) Material and Methods

New I-*Cre*I variant libraries were created by random mutagenesis of a pool of chosen engineered meganucleases cleaving the RAG2.8.3 target. Mutagenesis was peformed by PCR using Mn²⁺ or derivatives of dNTPs as 8-oxo-dGTP and dPTP in two-step PCR process, as described in the protocol from Jena Bioscience GmbH in JBS dNTP-Mutageneis kit. Primers used are preATGCreFor (5'-gcataaattactatacttctatagacacgcaaacacaaatacacagcggccttgccacc-3', SEQ ID NO: 228) and ICreIpostRev (5'-ggctcgaggagctcgtctagaggatcgctcgagttatcagtcggccgc-3', SEQ ID NO: 229). The new libraries were cloned *in vivo* in the yeast in the linearized pCLS1107 vector (Figure 15) harbouring a galactose inducible promoter, a Kan^{R} as selectable marker and a 2 micron origin of replication. Positives resulting clones were verified by sequencing (MILLEGEN).

Pools of mutants were amplified by PCR reaction using preATGCreFor and ICreIpostRev primers common for leucine vector (pCLS0542) and kanamycin vector (pCLS1107). Approximately 75 ng of PCR fragment and 75 ng of vector DNA (pCLS1107) linearized by digestion with *DraIII* and *NgoMIV* were used to transform the yeast *Saccharomyces cerevisiae* strain FYC2-6A (MATα, trp1Δ63, leu2Δ1, his3Δ200) using a high efficiency LiAc transformation protocol, and kanamycin resistant colonies were selected. A library of intact coding sequence for the I-*Cre*I mutant was generated by *in vivo* homologous recombination in yeast.

Yeast colonies were then picked, using a Q-Pix2 robot (Genetix), and individually mated with a yeast strain of opposite mating type (FYBL2-7B:MAT a, ura3Δ851, trp1Δ63, leu2Δ1, lys2Δ202) containing the RAG2.8 target into the pCLS1055 yeast reporter vector (Figure 11) and expressing a mutant cleaving the RAG2.8.4 target, cloned into the pCLS0542 (Figure 12). Mating was performed as described previously (Arnould et al., 2006, J. Mol. Biol. 355, 443-458) or as described in example 1.

### B) Results

Three mutants cleaving RAG2.8.3 (I-*Cre*I 33R, 40Q, 44A, 70A and 75N or, I-*Cre*I 33R, 40Q, 44A, 70H and 75N and I-*Cre*I 33R, 40Q, 44A, 70N and 75N, also called KNSRQQ/ARANI, KNSRQQ/ARHNI and KNSRQQ/ARNNI according to nomenclature of Table IX) were pooled, randomly mutagenized and transformed into yeast (Figure 20). 2280 transformed clones were then individually picked and mated with a yeast strain that (i) contains the RAG2.8 target in a reporter plasmid (ii) expresses a variant cleaving the RAG2.8.4 target, chosen among those described in example 7. Two such strains were used, expressing either the I-*Cre*I 28N, 33S, 38R, 40K, 44R, 68Y, 70S, 75N and 77N (or NNSSRK/RYSNN) mutant, either the I-*Cre*I 28Q,33S, 38R, 40K, 44R, 68Y, 70S, 75N and 77T (or QNSSRK/RYSNT) mutant (see Table XI). Twenty-four clones were found to trigger cleavage of the RAG2.8 target when mated with such yeast strain. In a control experiment, none of these clones was found to trigger cleavage of RAG2.8 without coexpression of the NNSSRK/RYSNN or QNSSRK/RYSNT protein. Therefore, twenty four positives were containing proteins able to cleave RAG2.8 when forming heterodimers with NNSSRK/RYSNN or QNSSRK/RYSNT. Examples of such heterodimeric mutants are listed in Table XI. Examples of positives are shown on Figure 20.

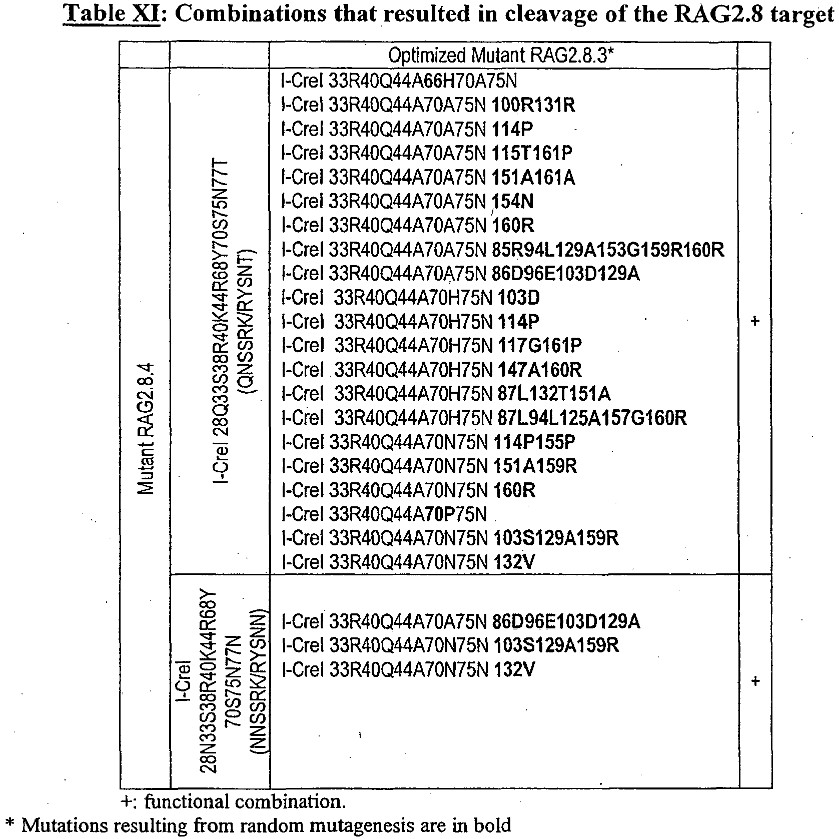

### Example 10: Making of meganucleases cleaving RAG2.8 with higher efficacy by random mutagenesis of proteins cleaving RAG2.8.4 and co-expression with proteins cleaving RAG2.8.3

I-*Cre*I mutants able to cleave the non palindromic RAG2.8 target were identified by co-expression of mutants cleaving the palindromic RAG2.8.3 and mutants cleaving the palindromic RAG2.8.4 target (Example 9). To increase the number and efficacy of I-*Cre*I mutants able to cleave the non palindromic RAG2.8 target, mutants cleaving the palindromic RAG2.8.4 target were mutagenized and new variants cleaving RAG2.8 with high efficacy, when co-expressed with mutants cleaving the RAG2.8.3 target, were screened.

### A) Material and Methods

The experimental procedures are similar to those described in example 9.

### B) Results

Three mutants cleaving RAG2.8.4 (I-*Cre*I 28Q33S38R40K44R68Y70S75N77T, I-*Cre*I 28N33S38R40K44R68Y70S75N77N, I-CreI 28N33S38R40K44R68Y70S75N77 also called QNSSRK/RYSNT, NNSSRK/RYSNN and NNSSRK/RYSNT KNSRQQ/ARHNI and KNSRQQ/ARNNI according to nomenclature of Table IX) were pooled, randomly mutagenized and transformed into yeast. 6696 transformed clones were then mated with a yeast strain that (i) contains the RAG2.8 target in a reporter plasmid (ii) expresses an optimized variant cleaving the RAG2.8.3 target, chosen among the variants identified in example 9. Two strains were used, expressing either the I-*Cre*I 33R40Q44A70N75N/103S129A159R or the I-*Cre*I 33R40Q44A70N75N/132V mutant (see table XI). More than one hundred ninety clones were found to trigger cleavage of the RAG2.8 target when mated with such yeast strain. In a control experiment, none of these clones was found to trigger cleavage of RAG2.8 without co-expression of each one of these 2 proteins. More than one hundred ninety positives were containing proteins able to cleave RAG2.8 when forming heterodimers with the I-*Cre*I 33R40Q44A70N75N/103S129A159R and the I-*Cre*I 33R40Q44A70N75N/132V. Examples of such heterodimeric mutants are listed in Table XII. Positives were rearrayed and tested again in quadriplicate in a secondary screen, as shown on Figure 23.

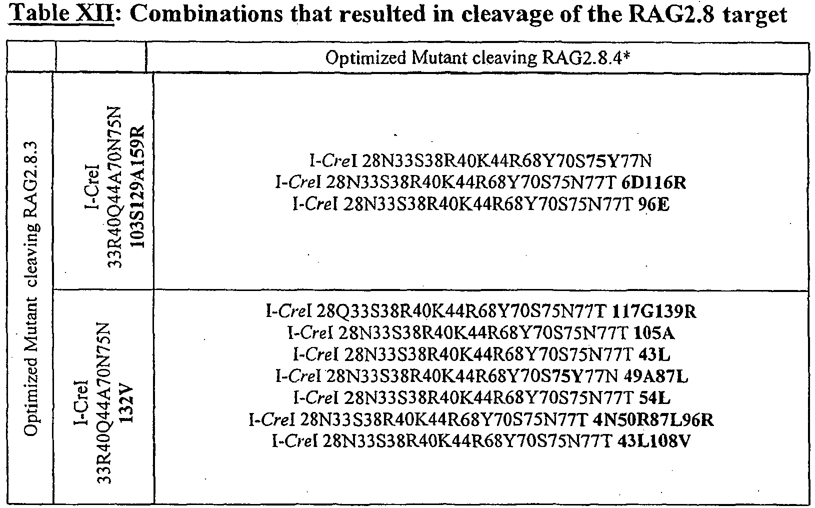

### Example 11: Improvement of meganucleases cleaving the RAG1.10 DNA sequence by random mutagenesis of proteins cleaving the RAG1.10.2 target and co-expression with proteins cleaving the RAG1.10.3 target

I-*Cre*I mutants able to cleave the RAG1.10 target were identified by assembly of mutants cleaving the palindromic RAG1.10.2 and RAG1.10.3 targets (example 5). Then, to improve the RAG1.10 cleavage efficiency, the combinatorial mutants cleaving the RAG1.10 DNA sequence were mutagenized and variants displaying stronger cleavage of this target were screened.

According to the structure of the I-*Cre*I protein bound to its target, there is no contact between the 4 central base pairs (positions -2 to 2) and the I-*Cre*I protein (Chevalier et al., Nat. Struct. Biol., 2001, 8, 312-316; Chevalier B.S. and B.L. Stoddard, Nucleic Acids Res., 2001, 29, 3757-3574; Chevalier et al., J. Mol. Biol., 2003, 329, 253-269). Thus, it is difficult to rationally choose a set of positions to mutagenize, and random mutagenesis was performed on the whole protein. Random mutagenesis results in high complexity libraries. Therefore, to limit the complexity of the variant libraries to be tested by mutagenizing only one of the two components of the heterodimers cleaving the RAG1.10 target was mutagenized.

Thus, in a first step proteins cleaving the RAG1.10.2 target were mutagenized, and in a second step, it was assessed whether they could improve the RAG1.10 cleavage efficiency when co-expressed with a protein cleaving the RAG1.10.3 DNA sequence.

### A) Material and Methods

The experimental procedures are similar to those described in example 9.

### B) Results

Five mutants cleaving the RAG1.10.2 sequence (KRSNQS/AYSYK, KKSAQS/AYSYK, KRSNQS/TYSYR, KNSRTS/AYSYK and KKSGQS/AYSYK) were pooled, randomly mutagenized and transformed into yeast. These five mutants are described according to the Table V nomenclature of Example 3 with the one letter code for amino acids at positions 28, 30, 32, 33, 38, 40 / 44, 68, 70 75 and 77. 2280 transformed clones were then mated with a yeast strain that contains (i) the RAG 1.10 target in a reporter plasmid, (ii) an expression plasmid containing a mutant that cleaves the RAG1.10.3 target (KHSMAS/ARSYT, see Table VI of Example 4). After mating with this yeast strain, 80 clones were found to cleave the RAG1.10 target more efficiently than the original RAG1.10.2 mutant. These 80 mutants were then rearranged (wells A1 to G8 of the rearranged plate, see Figure 24) and submitted to a validation screen conducted exactly in the same conditions as the first one. As can be seen on figure 24, several mutants were able to form heterodimers with KHSMAS/ARSYT, which show a stronger cleavage activity for the RAG1.10 target. Sequencing of the 80 positive clones allowed the identification of identical clones and finally 6 distinct novel mutants giving higher levels of cleavage of RAG1.10 were identified. They are all listed in Table XIII. Five mutants are close relatives to the initial KRSNQS/AYSYK protein, and differ from this mutant only by one or two additional substitution. In contrast, the KRSNQS/AYSDR protein, which differs from KRSNQS/AYSYK by positions 75 and 77, and from KRSNQS/TYSYR by positions 44 and 75, has no mutation in novel positions, different from those initially engineered to obtain RAG1.10.2 cleavers (see example 3).

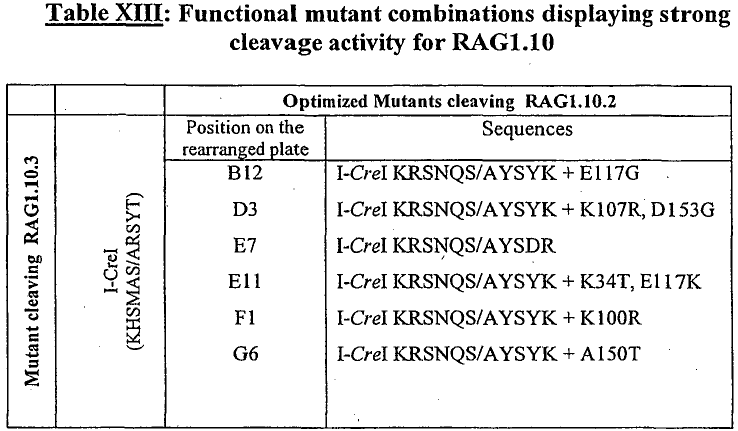

### Example 12: Improvement of meganucleases cleaving the RAG1.10 DNA target by introduction of a single G19S substitution

The G19S mutation was introduced into the KRSNQS/AYSDR mutant (noted M2 below) cleaving the RAG1.10.2 target (see example 11, Table XIII and Figure 24) and into the NNSSRR/YRSQV mutant (noted M3 below) cleaving the RAG1.10.3 target (see example 4, Table VI). These new proteins were then tested against the RAG1.10, RAG1.10.2 and RAG1.10.3 targets in extrachromosomal and chromosomal assays in mammalian cells.

### A) Material and Methods

### a) Introduction of the G19S mutation

Two overlapping PCR reactions were performed using two sets of primers: Gal10F (5'-gcaactttagtgctgacacatacagg-3'; SEQ ID NO: 223) and G19SRev (5'-gatgatgctaccgtcagagtccacaaagccggc-3'; SEQ ID NO: 230) for the first fragment and G19SFor (5'-gccggctttgtggactctgacggtagcatcatc-3'; SEQ ID NO: 231) and Gal10R (5'-acaaccttgattggagacttgacc-3'; SEQ ID NO: 224) for the second fragment. Approximately 25 ng of each PCR fragment and 75 ng of vector DNA (pCLS0542) linearized by digestion with *Nco*I and *Eag*I were used to transform the yeast *Saccharomyces cerevisiae* strain FYC2-6A (MATα, trp1Δ63, leu2Δ1, his3Δ200) using a high efficiency LiAc transformation protocol (Gietz, R.D. and R.A. Woods, Methods Enzymol., 2002, 350, 87-96). An intact coding sequence containing the G19S mutation is generated by *in vivo* homologous recombination in yeast.

### b) Sequencing of the mutants

To recover the mutant expressing plasmids, yeast DNA was extracted using standard protocols and used to transform *E*. *coli*. Sequence of mutant ORF were then performed on the plasmids by MILLEGEN SA.

### c) Cloning of the RAG1.10 G19S mutants into a mammalian expression vector

Each mutant ORF was amplified by PCR using the primers CCM2For:
(5'-aagcagagctctctggctaactagagaacccactgcttactggcttatcgaccatggccaataccaaatataacaaag agttcc-3'; SEQ ID NO: 232)
and CCMRevBis:
(5'-ctgctctagattagtcggccgccggggaggatttcttc-3'; SEQ ID NO: 233).

The PCR fragment was digested by the restriction enzymes *Sac*I and *Xba*I, and was then ligated into the vector pCLS1088 (Figure 25) digested also by *Sac*I and *Xba*I. Resulting clones were verified by sequencing (MILLEGEN).

### d) Cloning of the different RAG1.10 targets in a vector for extrachromosomal assay

The target of interest was cloned as follows: oligonucleotide corresponding to the target sequence flanked by gateway cloning sequence was ordered from Proligo. Double-stranded target DNA, generated by PCR amplification of the single stranded oligonucleotide, was cloned using the Gateway protocol (INVITROGEN) into CHO reporter vector (pCLS1058, Figure 26).

### e) Extrachromosomal assay in CHO cells

CHO cells were transfected with Polyfect transfection reagent according to the supplier's protocol (QIAGEN). Per assay, 150 ng of target vector was cotransfected with 12.5 ng of each one of both mutants (12.5 ng of mutant cleaving palindromic B2M11.2 target and 12.5 ng of mutant cleaving palindromic B2M11.3 target). 72 hours after transfection, culture medium was removed and 150 µl of lysis/revelation buffer added for β-galactosidase liquid assay (1 liter of buffer containing: 100 ml of lysis buffer (Tris-HCl 10 mM pH 7.5, NaCl 150 mM, Triton X100 0.1 %, BSA 0.1 mg/ml, protease inhibitors), 10 ml of Mg 100 X buffer (MgCl₂ 100 mM, β-mercaptoethanol 35 %), 110 ml ONPG 8 mg/ml and 780 ml of sodium phosphate 0.1M pH 7.5). After incubation at 37 °C, the optical density was measured at 420 nm. The entire process is performed on an automated Velocityl BioCel platform.

### f) Chromosomal assay in CHO cells

CHO cell lines harbouring the reporter system were seeded at a density of 2x10⁵ cells per 10cm dish in complete medium (Kaighn's modified F-12 medium (F12-K), supplemented with 2 mM L-glutamine, penicillin (100 UI/ml), streptomycin (100 µg/ml), amphotericin B (Fongizone) (0.25 µg/ml) (INVITROGEN-LIFE SCIENCE) and 10% FBS (SIGMA-ALDRICH CHIMIE). The next day, cells were transfected with Polyfect transfection reagent (QIAGEN). Briefly, 0.1 µg of lacz repair matrix vector pCLS1058 was co-transfected with various amounts of meganucleases expression vectors. After 72 hours of incubation at 37°C, cells were fixed in 0.5 % glutaraldehyde at 4°C for 10 min, washed twice in 100 mM phosphate buffer with 0.02 % NP40 and stained with the following staining buffer (10 mM Phosphate buffer, 1 mM MgCl₂, 33 mM K hexacyanoferrate (III), 33 mM K hexacyanoferrate (II), 0.1 % (v/v) X-Gal). After, an overnight incubation at 37°C, plates were examined under a light microscope and the number of LacZ positive cell clones counted. The frequency of LacZ repair is expressed as the number of LacZ+ foci divided by the number of transfected cells (5x10⁵) and corrected by the transfection efficiency.

### B) Results

The activity of the M2 and M3 I-*Cre*I mutants harboring the G19S mutation (M2 G19S and M3 G19S) against their respective targets RAG1.10.2 and RAG1.10.3 was monitored using the extrachromosomal assay in CHO cells. The mutants were tested either in a pure homodimeric way or in co-transfecting the mutants with and without the G19S mutation, which allowed the detection of the activity of both heterodimers M2/M2 G19S and M3/M3 G19S against their respective RAG1.10.2 and RAG1.10.3 targets (Figure 27A). Then the different heterodimers M2/M3, M2 G19S/M3 and M2/M3 G19S were tested against the RAG1.10 target (Figure 27B). As can be seen in Figures 27A and 27B, two aspects of the G19S mutation are observed.

First, this mutation abolishes the activity of the homodimers (M2 G19S and M3 G19S) against their palindromic targets. This effect is likely due to steric clashes within the dimerization interface. Most engineered endonucleases (ZFNs and HEs) so far are heterodimers, and include two separately engineered monomers, each binding one half of the target. Heterodimer formation is obtained by co-expression of the two monomers in the same cells (Porteus H.M., Mol. Ther., 2006, 13, 438-446; Smith et al., Nucleic acids Res. Epub 27 November 2006; International PCT Applications WO 2007/097854 and WO 2007/049156). However, it is actually associated with the formation of two homodimers (Arnould et al., J. Mol. Biol., 2006, 355, 443-458; Bibikova et al., Genetics, 2002, 161, 1169-1175), recognizing different targets, and individual homodimers can sometimes result in an extremely high level of toxicity (Bibikova et al., Genetics, 2002, 161, 1169-1175). This issue can be solved only by the suppression of functional homodimer formation, which could, in theory, be achieved by the fusion of the two monomers in a single chain molecule (Chevalier et al., Mol. Cell., 2002, 10, 895-905; Epinat et al., Nucleic Acids Res., 2005, 33, 5978-5990). However, this kind of design is relatively perilous, and can result in badly folded proteins (Epinat et al., Nucleic Acids Res., 2005, 33, 5978-5990). Impairing the functionnality of individual homodimers would be another solution, and the effect observed here should have tremendous implications in terms of specificity.

Second, introduction of the G19S mutation in the M3 mutant greatly increases the activity of the RAG1.10.3 target cleavage by the M3/M3 G19S heterodimer. This effect can not be really evidenced for the M2 mutant because it already cleaves the RAG1.10.2 target at saturating levels in this assay. The same remark can be made for the RAG1.10 target, which is cleaved at saturating levels by the M2/M3 heterodimer as well as the M2 G19S/M3 and M2/M3 G19S heterodimers.

These three last heterodimers were then tested in a chromosomal assay in CHO cells. This chromosomal assay has been extensively described in a recent publication (Arnould et al., J. Mol. Biol. Epub May 10, 2007). Briefly, a CHO cell line carrying a single copy transgene was first created. The transgene contains a human EF1α promoter upstream an I-*Sce*I cleavage site (Figure 28, step 1). Second, the I-*Sce*I meganuclease was used to trigger DSB-induced homologous recombination at this locus, and insert a 5.5 kb cassette with a novel meganuclease cleavage site (Figure 28, step2). This cassette contains a non functional LacZ open reading frame driven by a CMV promoter, and a promoter-less hygromycin marker gene. The LacZ gene itself is inactivated by a 50 bp insertion containing the meganuclease cleavage site to be tested (here, the RAG1.10 cleavage site). This cell line can in turn be used to evaluate DSB-induced gene targeting efficiencies (LacZ repair) with engineered I-*Cre*I derivatives cleaving the RAG1.10 target (Figure 28, step3).

This cell line was co-transfected with the repair matrix and various amounts of the vectors expressing the meganucleases. Results are summarized in Table XIV. The frequency of repair of the LacZ gene increased from a maximum of 2.4 x10⁻³ with the initial engineered heterodimers (M2/M3), to a maximum of 5.8 x 10⁻³ with the M2 G19S/M3 heterodimer. A more than two fold increase of the frequency of gene targeting was observed when the G19S was introduced in one of the two monomers (M2 or M3). Thus, these results confirm what was observed in the extrachromosomal substrate and show that the G19S substitution results in a significant improvement of activity.

**Table XIV: Frequency of meganuclease-induced LacZ repair in a reporter chromosomal system in CHO cells (described in Figure 28).**

| **Heterodimer** | **Frequency of LacZ repair** |
|---|---|
| M2/M3 | 2.4x10⁻³ |
| M2 G19S/M3 | 5.8x10⁻³ |
| M2/M3 G19S | 5.2x10⁻³ |
| M2 G19S/M3 G19S | 0 |

## Claims

1. An I-*Cre*I variant comprising two I-*Cre*I monomers, **characterized in that** at least one of the two I-*Cre*I monomers has at least two substitutions, one in each of the two functional subdomains of the LAGLIDADG core domain situated respectively from positions 26 to 40 and 44 to 77 of I-C*re*I amino acid sequence SEQ ID NO:234, said variant being able to cleave a DNA target sequence from a human RAG1 gene selected from the group consisting of the sequences SEQ ID NO: 148 to 177, and being obtainable by a method comprising at least the steps of:
(a) constructing a first series of I-*Cre*I variants having at least one substitution at positions 26, 28, 30, 32, 33, 38 and/or 40 of a first functional subdomain of the LAGLIDADG core domain situated from positions 26 to 40 of I-*Cre*I,
(b) constructing a second series of I-*Cre*I variants having at least one substitution at positions 44, 68, 70, 75, and/or 77 of a second functional subdomain of the LAGLIDADG core domain situated from positions 44 to 77 of I-*Cre*I,
(c) selecting the variants from the first series of step (a) which are able to cleave a mutant I-*Cre*I site wherein (i) the nucleotide triplet in positions -10 to -8 of the I-*Cre*I site has been replaced with the nucleotide triplet which is present in position -10 to -8 of said human RAG1 DNA target and (ii) the nucleotide triplet in positions +8 to +10 has been replaced with the reverse complementary sequence of the nucleotide triplet which is present in position -10 to -8 of said human RAG1 DNA target,
(d) selecting the variants from the second series of step (b) which are able to cleave a mutant I-*Cre*I site wherein (i) the nucleotide triplet in positions -5 to -3 of the I-*Cre*I site has been replaced with the nucleotide triplet which is present in position -5 to -3 of said human RAG1 DNA target and (ii) the nucleotide triplet in positions +3 to +5 has been replaced with the reverse complementary sequence of the nucleotide triplet which is present in position -5 to -3 of said human RAG1 DNA target,
(e) selecting the variants from the first series of step (a) which are able to cleave a mutant I-*Cre*I site wherein (i) the nucleotide triplet in positions +8 to +10 of the I-*Cre*I site has been replaced with the nucleotide triplet which is present in positions +8 to +10 of said human RAG1 DNA target and (ii) the nucleotide triplet in positions -10 to -8 has been replaced with the reverse complementary sequence of the nucleotide triplet which is present in position +8 to +10 of said human RAG1 DNA target,
(f) selecting the variants from the second series of step (b) which are able to cleave a mutant I-*Cre*I site wherein (i) the nucleotide triplet in positions +3 to +5 of the I-*Cre*I site has been replaced with the nucleotide triplet which is present in positions +3 to +5 of said human RAG1 DNA target and (ii) the nucleotide triplet in positions -5 to -3 has been replaced with the reverse complementary sequence of the nucleotide triplet which is present in position +3 to +5 of said human RAG1 DNA target,
(g) combining in a single variant, the mutation(s) in positions 26 to 40 and 44 to 77 of two variants from step (c) and step (d), to obtain a novel homo-dimeric I-*Cre*I variant which cleaves a sequence wherein (i) the nucleotide triplet in positions -10 to -8 is identical to the nucleotide triplet which is present in positions -10 to -8 of said human RAG1 DNA target, (ii) the nucleotide triplet in positions +8 to +10 is identical to the reverse complementary sequence of the nucleotide triplet which is present in positions -10 to -8 of said human RAG1 DNA target, (iii) the nucleotide triplet in positions -5 to -3 is identical to the nucleotide triplet which is present in positions -5 to -3 of said human RAG1 DNA target and (iv) the nucleotide triplet in positions +3 to +5 is identical to the reverse complementary sequence of the nucleotide triplet which is present in positions -5 to -3 of said human RAG1 DNA target, and/or
(h) combining in a single variant, the mutation(s) in positions 26 to 40 and 44 to 77 of two variants from step (e) and step (f), to obtain a novel homo-dimeric I-*Cre*I variant which cleaves a sequence wherein (i) the nucleotide triplet in positions +3 to +5 is identical to the nucleotide triplet which is present in positions +3 to +5 of said human RAG1 DNA target, (ii) the nucleotide triplet in positions -5 to -3 is identical to the reverse complementary sequence of the nucleotide triplet which is present in positions +3 to +5 of said human RAG1 DNA target, (iii) the nucleotide triplet in positions +8 to +10 of the I-*Cre*I site has been replaced with the nucleotide triplet which is present in positions +8 to +10 of said human RAG1 DNA target and (iv) the nucleotide triplet in positions -10 to -8 is identical to the reverse complementary sequence of the nucleotide triplet in positions +8 to +10 of said human RAG1 DNA target,
(i) combining the variants obtained in steps (g) and (h) to form heterodimers, and
(j) selecting the heterodimers from step (i) which are able to cleave said DNA target sequences from RAG1 gene.

2. The variant according to claim 1, which is an heterodimer able to cleave a DNA target sequence from a human RAG1 gene selected from the group consisting of the sequences SEQ ID NO: 148 to 177, said heterodimer consisting of a first and a second monomer having amino acids at positions 28, 30, 32, 33, 38, 40, 44, 68, 70, 75 and 77 of I-*Cre*I which are as indicated in Table XV:
| **First monomer** | **Second monomer** |
|---|---|
| 28Q30N32S33Y38R40K44Y68E70S75R77V | 28K30R32Q33Y38Q40S44K68T70S75N77V |
| 28K30N32S33S38R40H44A68Y70S75Y77K | 28A30N32S33S38R40K44D68N70S75N771 |
| 28K30D32S33R38T40S44Y68S70S75S77D | 28K30N32T33C38Q40S44K68Y70S75Q77N |
| 28N30N32S33S38R40R44A68R70T75N771 | 28K30R32S33C38Q40S44A68Y70S75Y77K |
| 28N30N32S33S38R40R44Y68R70S75Q77V | 28K30K32S33A38Q40S44A68Y70S75Y77K |
| 28N30N32S33S38R40R44Y68R70S75Q77V | 28K30K32S33A38Q40S44A68S70S75D77R |
| 28N30N32S33S38R40R44Y68R70S75Q77V | 28K30K32S33A38Q40S44R68Y70S75D77T |
| 28N30N32S33S38R40R44Y68R70S75Q77V | 28K30R32S33N38Q40S44T68Y70S75Y77R |
| 28N30N32S33S38R40R44Y68R70S75Q77V | 28K30R32S33C38Q40S44A68Y70S75Y77K |
| 28N30N32S33S38R40R44Y68R70S75Q77V | 28K30R32S33N38Q40S44K68Y70S75Y77N |
| 28N30N32S33S38R40R44Y68R70S75Q77V | 28K30N32S33R38T40S44A68Y70S75Y77K |
| 28N30N32S33S38R40R44Y68R70S75Q77V | 28K30K32S33G38Q40S44A68Y70S75Y77K |
| 28N30N32S33S38R40R44A68R70N75N77I | 28K30K32S33A38Q40S44A68Y70S75Y77K |
| 28N30N32S33S38R40R44A68R70N75N77I | 28K30K32S33A38Q40S44A68S70S75D77R |
| 28N30N32S33S38R40R44A68R70N75N77I | 28K30R32S33N38Q40S44T68Y70S75Y77R |
| 28N30N32S33S38R40R44A68R70N75N77I | 28K30R32S33C38Q40S44A68Y70S75Y77K |
| 28N30N32S33S38R40R44A68R70N75N77I | 28K30R32S33N38Q40S44K68Y70S75Y77N |
| 28N30N32S33S38R40R44A68R70N75N77I | 28K30N32S33R38T40S44A68Y70S75Y77K |
| 28N30N32S33S38R40R44A68R70N75N77I | 28K30K32S33G38Q40S44A68Y70S75Y77K |
| 28Q30N32S33S38R40K44A68H70Q75N77I | 28K30K32S33A38Q40S44A68Y70S75Y77K |
| 28Q30N32S33S38R40K44A68H70Q75N77I | 28K30K32S33A38Q40S44A68S70S75D77R |
| 28Q30N32S33S38R40K44A68H70Q75N77I | 28K30R32S33N38Q40S44T68Y70S75Y77R |
| 28Q30N32S33S38R40K44A68H70Q75N77I | 28K30R32S33C38Q40S44A68Y70S75Y77K |
| 28Q30N32S33S38R40K44A68H70Q75N77I | 28K30R32S33N38Q40S44K68Y70S75Y77N |
| 28Q30N32S33S38R40K44A68H70Q75N77I | 28K30N32S33R38T40S44A68Y70S75Y77K |
| 28Q30N32S33S38R40K44A68H70Q75N77I | 28K30K32S33G38Q40S44A68Y70S75Y77K |
| 28K30H32S33M38A40S44A68R70S75Y77T | 28K30K32S33A38Q40S44A68Y70S75Y77K |
| 28K30H32S33M38A40S44A68R70S75Y77T | 28K30R32S33N38Q40S44A68Y70S75D77R |
| 28N30N32S33S38R40R44Y68R70S75Y77Q | 28K30K32S33A38Q40S44A68Y70S75Y77K |
| 28N30N32S33S38R40R44Y68R70S75Y77Q | 28K30K32S33A38Q40S44A68S70S75D77R |
| 28N30N32S33S38R40R44Y68R70S75Y77Q | 28K30K32S33A38Q40S44R68Y70S75D77T |
| 28N30N32S33S38R40R44Y68R70S75Y77Q | 28K30R32S33N38Q40S44T68Y70S75Y77R |
| 28N30N32S33S38R40R44Y68R70S75Y77Q | 28K30R32S33C38Q40S44A68Y70S75Y77K |
| 28N30N32S33S38R40R44Y68R70S75Y77Q | 28K30R32S33N38Q40S44K68Y70S75Y77N |
| 28N30N32S33S38R40R44Y68R70S75Y77Q | 28K30N32S33R38T40S44A68Y70S75Y77K |
| 28N30N32S33S38R40R44Y68R70S75Y77Q | 28K30K32S33G38Q40S44A68Y70S75Y77K |
| 28N30N32S33S38R40R44N68R70S75Y77V | 28K30K32S33A38Q40S44A68Y70S75Y77K |
| 28N30N32S33S38R40R44N68R70S75Y77V | 28K30K32S33A38Q40S44A68S70S75D77R |
| 28N30N32S33S38R40R44N68R70S75Y77V | 28K30R32S33N38Q40S44T68Y70S75Y77R |
| 28N30N32S33S38R40R44N68R70S75Y77V | 28K30R32S33C38Q40S44A68Y70S75Y77K |
| 28N30N32S33S38R40R44N68R70S75Y77V | 28K30R32S33N38Q40S44K68Y70S75Y77N |
| 28N30N32S33S38R40R44N68R70S75Y77V | 28K30N32S33R38T40S44A68Y70S75Y77K |
| 28N30N32S33S38R40R44N68R70S75Y77V | 28K30K32S33G38Q40S44A68Y70S75Y77K |
| 28Q30N32S33S38R40K44A68A70N75N77I | 28K30K32S33A38Q40S44A68Y70S75Y77K |
| 28Q30N32S33S38R40K44A68A70N75N77I | 28K30K32S33A38Q40S44A68S70S75D77R |
| 28Q30N32S33S38R40K44A68A70N75N77I | 28K30R32S33N38Q40S44T68Y70S75Y77R |
| 28Q30N32S33S38R40K44A68A70N75N77I | 28K30R32S33C38Q40S44A68Y70S75Y77K |
| 28Q30N32S33S38R40K44A68A70N75N77I | 28K30R32S33N38Q40S44K68Y70S75Y77N |
| 28Q30N32S33S38R40K44A68A70N75N77I | 28K30N32S33R38T40S44A68Y70S75Y77K |
| 28K30N32S33R38E40R44Q68R70S75D77K | 28K30G32S38G40S44R68R70S75Q77N |
| 28Q30N32S33S38R40K44N68R70S75R77N | 28K30D32S33R38T40S44A68N70S75Y77R |
| 28Q30N32S33R38Q40R44K68Y70S75Y77Q | 28K30G32S33Y38G40S44Q68R70S75R77Y |
| 28K30N32S33Y38Q40S44Q68R70R75E77R | 28K30N32S33S38W40S44N68R70S75R77N |
| 28K30N32S33G38A40S44Q68R70R75N77I | 28K30N32T33T38Q40S44N68R70S75R77N |
| 28K30G32S33Y38T40S44D68R70S75R77Q | 28R30N32S33S38Y40Q44T68R70S75E77R |
| 28K30N32S33G38Y40S44N68R70S75R77N | 28K30N32S33T38A40S44Q68A70N75D77I |
| 28K30N32G33R38Q40S44Q68R70R75D77I | 28K30G32S33Y38G40S44E68R70H75D77I |
| 28K30N32S33T38A40S44R68Y70S75Y77N | 28K30G32S33Y38T40S44K68N70A75D77I |
| 28K30S32G33R38Q40S44Q68R70R75N77I | 28K30N32S33S38R40D44Y68Y70S75Q77I |
| 28K30N32S33N38R40A44Q68R70R75N77I | 28K30N32S33R38A40S44D68R70S75R77Q |
| 28K30N32S33S38R40D44Q68R70S75N77I | 28Q30N32S33Y38R40K44Q68R70S75N77I |
| 28A30N32S33T38Q40R44Q68R70S75N77L | 28K30N32E33Y38Q40S44Y68R70S75D77V |
| 28Q30N32S33R38R40K44K68T70S75N77V | 28R30N32S33R38Y40Q44N68T70S75R77V |
| 28K30N32S33R38A40Q44D68R70N75D77I | 28K30N32S33C38T40S44N68R70S75R77N |
| 28K30N32S33Y38Q40S44Q68R70S75N77I | 28Q30N32S33Y38R40K44N68R70S75R77N |
| 28K30R32D33Y38Q40S44D68N70S75N77I | 28K30G32S33Y38H40S44N68R70S75R77D |
| 28K30N32S33S38D40S44T68Y70S75Y77K | 28R30N32S33A38Y40Q44T68Y70S75R77V |
| 28K30G32S33Y38T40S44Y68Y70S75Q77I | 28K30N32S33R38N40Q44Q68R70S75K77E |
| 28K30N32S33C38S40S44T68Y70S75Y77K | 28K30N32S33T38Q40S44Q68R70R75N77I |
| 28K30N32S33T38A40S44T68Y70S75Y77K | 28K30D32S33R38T40S44Q68R70R75N77I |
| 28K30G32S33Y38H40S44N68E70S75K77R | 28A30N32S33S38R40K44D68Y70S75S77R |
| 28Q30N32S33Y38R40K44Q68Y70S75R77Q | 28Q30N32S33Y38Q40K44A68N70S75Y77R |
| 28K30N32S33H38Q40Q44D68N70S75N77I | 28K30D32S33R38Q40S44N68Y70S75R77V |
| 28K30N32K33T38Q40S44N68Y70S75Y77Q | 28K30N32S33S38R40E44Y68Y70S75Q77I |
| 28K30N32S33Y38Q40S44A68G70N75D77I | 28K30N32S33W38Q40H44Y68R70S75N77V |
| 28N30N32S33S38R40R44Y68R70S75Q77V | 28K30R32S33N38Q40S44A68Y70S75D77R |

3. The variant according to claim 1 or claim 2, which comprises at least one substitution that improves its binding and/or cleavage properties towards said DNA target sequence from a human RAG1 gene, said substitution being at a position of I-*Cre*I which is selected from the group consisting of positions 4, 6, 19, 34, 43, 49, 50, 54, 66, 79, 80, 82, 85, 86, 87, 94, 96, 100, 103, 105, 107, 108, 114, 115, 116, 117, 125, 129, 131, 132, 139, 147, 150, 151, 153, 154, 155, 157, 159 and/or 160.

4. The variant according to claim 3, wherein said substitutions are selected from the group consisting of: G19S, G19A, F54L, S79G, F87L, V105A and I132V.

5. The variant according to claim 3 or claim 4, which is an heterodimer consisting of a first and a second monomer having amino acids at positions 28, 30, 32, 33, 38, 40, 44, 68, 70, 75, 77 and at least one of the positions of I-*Cre*I defined in claim 3, which are as indicated in Table XVII:
| **First monomer** | **Second monomer** |
|---|---|
| 28K30H32S33M38A40S44A68R70S75Y77T | 28K30R32S33N38Q40S44A68Y70S75Y77K117G |
| 28K30H32S33M38A40S44A68R70S75Y77T | 28K30R32S33N38Q40S44A68Y70S75Y77K107R 153G |
| 28K30H32S33M38A40S44A68R70S75Y77T | 28K30R32S33N34T38Q40S44A68Y70S75Y77K117K |
| 28K30H32S33M38A40S44A68R70S75Y77T | 28K30R32S33N38Q40S44A68Y70S75Y77K100R |
| 28K30H32S33M38A40S44A68R70S75Y77T | 28K30R32S33N38Q40S44A68Y70S75Y77K150T |
| 19S28N30N32S33S38R40R44Y68R70S75Q77V | 28K30R32S33N38Q40S44A68Y70S75D77R |
| 28N30N32S33S38R40R44Y68R70S75Q77V | 19S28K30R32S33N38Q40S44A68Y70S75D77R |

6. The variant according to anyone of claims 1 to 5, which is an heterodimer consisting of a first and a second monomer selected from the following pairs of sequences : SEQ ID NO: 2 and 39, SEQ ID NO: 3 and 40, SEQ ID NO: 4 and 41, SEQ ID NO: 5 and 42, SEQ ID NO: 6 or 10 and any of the SEQ ID NO: 42 to 49, SEQ ID NO: 7, 8, 11 and any of the SEQ ID NO: 42 to 44 and 46 to 49, SEQ ID NO: 9 and any of SEQ ID NO: 43, 248 to 253, SEQ ID NO: 12 and any of the SEQ ID NO: 42 to 44 and 46 to 48, SEQ ID NO: 13 and 50, SEQ ID NO: 14 and 51, SEQ ID NO: 15 and 52, SEQ ID NO: 16 and 53, SEQ ID NO: 17 and 54, SEQ ID NO: 18 and 55, SEQ ID NO: 19 and 56, SEQ ID NO: 20 and 57, SEQ ID NO: 21 and 58, SEQ ID NO: 22 and 59, SEQ ID NO: 23 and 60, SEQ ID NO: 24 and 61, SEQ ID NO: 25 and 62, SEQ ID NO: 26 and 63, SEQ ID NO: 27 and 64, SEQ ID NO: 28 and 65, SEQ ID NO: 29 and 66, SEQ ID NO: 30 and 67, SEQ ID NO: 31 and 68, SEQ ID NO: 32 and 69, SEQ ID NO: 33 and 70, SEQ ID NO: 34 and 71, SEQ ID NO: 35 and 72, SEQ ID NO: 36 and 73, SEQ ID NO: 37 and 74, SEQ ID NO: 38 and 75, SEQ ID NO: 6 and the variant of SEQ ID NO:250 further including the G19S mutation, the variant of SEQ ID NO:6 further including the G19S mutation and SEQ ID NO: 250, and wherein said heterodimer is able to cleave a DNA target sequence from a human RAG1 gene selected from the group consisting of the sequences SEQ ID NO: 148 to 177.

7. A single-chain chimeric endonuclease derived from an I-*Cre*I variant according to anyone of claims 1 to 6.

8. A polynucleotide fragment encoding at least one of the monomers of a variant according to anyone of claims 1 to 6 or a single-chain chimeric endonuclease according to claim 7.

9. An expression vector comprising at least one polynucleotide fragment according to claim 8.

10. The vector according to claim 9, which includes a targeting construct comprising a sequence to be introduced in a RAG1 gene flanked by sequences homologous to the sequences of the RAG1 gene flanking one of the RAG1 gene target sequence selected from the group consisting of the sequences SEQ ID NO: 148 to 177.

11. The vector according to claim 10, wherein said sequence to be introduced is a sequence which repairs a mutation in the human RAG1 gene.

12. The vector according to claim 10, wherein the sequence to be introduced comprises the human RAG1 open reading frame and a polyadenylation site to stop transcription in 3'.

13. The vector according to anyone of claims 10 to 12, wherein said sequences homologous to the sequences of the RAG gene flanking the RAG1 gene target sequence consist of a fragment of the human RAG1 gene comprising positions: 6 to 205, 1603 to 1802, 2219 to 2418, 5181 to 5380, 5222 to 5421, 5499 to 5698, 5709 to 5908, 5936 to 6135, 6049 to 6248, 6097 to 6296, 6212 to 6411, 6270 to 6469, 6521 to 6720, 6559 to 6758, 6667 to 6866, 6710 to 6909, 6853 to 7052, 6976 to 7175, 7012 to 7211, 7168 to 7367, 7207 to 7406, 7231 to 7430, 7478 to 7677, 7622 to 7821, 7709 to 7908, 7920 to 8119, 8144 to 8343, 8149 to 8348, 8252 to 8451, and/or 8271 to 8470 of said human RAG1 gene.

14. A composition comprising at least one variant according to anyone of claims 1 to 6, one single-chain chimeric endonuclease according to claim 7, and/or at least one vector according to anyone of claims 9 to 13.

15. The use of at least one variant according to anyone of claims 1 to 6, one single-chain chimeric endonuclease according to claim 7, and/or one vector according to anyone of claims 9 to 13, for the preparation of a medicament for preventing, improving or curing a SCID syndrome associated with a mutation in the human RAG1 gene, in an individual in need thereof.

16. A host cell which comprises at least one polynucleotide fragment according to claim 8 or a vector according to anyone of claims 9 to 13.

17. A non-human transgenic animal comprising at least one poly-nucleotide fragment according to claim 8.

18. A transgenic plant comprising at least one polynucleotide fragment according to claim 8.

19. Use of at least one variant according to anyone of claims 1 to 6, one single-chain chimeric endonuclease according to claim 7, one vector according to anyone of claims 9 to 13, for genome engineering, for non-therapeutic purposes.

## Patentansprüche

1. I-*Cre*I Variante, die zwei I-*Cre*I Monomere umfasst, **dadurch gekennzeichnet, dass** mindestens eines der zwei I-*Cre*I Monomere mindestens zwei Substitutionen aufweist, eine in jeder der zwei funktionalen Unterdomänen der LAGLIDADG Kerndomäne, die jeweils von den Positionen 26 bis 40 und 44 bis 77 der I-*Cre*I Aminosäuresequenz SEQ ID NO: 234 gelegen sind, wobei die Variante in der Lage ist, eine DNS-Zielsequenz von einem humanen RAG1 Gen zu spalten, die aus der Gruppe ausgewählt ist, die aus den Sequenzen SEQ ID NO: 148 bis 177 besteht, und durch ein Verfahren erhältlich ist, das mindestens die Schritte umfasst:
(a) Erstellen einer ersten Serie von I-*Cre*I Varianten, die mindestens eine Substitution in den Positionen 26, 28, 30, 32, 33, 38 und/oder 40 einer ersten funktionalen Unterdomäne der LAGLIDADG Kemdomäne aufweisen, die von den Positionen 26 bis 40 der I-*Cre*I gelegen ist,
(b) Erzeugen einer zweiten Serie von I-*Cre*I Varianten, die mindestens eine Substitution in den Positionen 44, 68, 70, 75 und/oder 77 einer zweiten funktionalen Unterdomäne der LAGLIDADG Kerndomäne aufweisen, die von den Positionen 44 bis 77 der I-*Cre*I gelegen ist,
(c) Auswählen der Varianten aus der ersten Serie von Schritt (a), die in der Lage sind, eine mutierte I-*Cre*I Stelle zu spalten, wobei (i) das Nukleotid-Triplett in den Positionen -10 bis -8 der I-*Cre*I Stelle durch ein Nukleotid-Triplett ersetzt wurde, das in den Positionen -10 bis -8 des humanen RAG1 DNS-Ziels vorgibt, und (ii) das Nukleotid-Triplett in den Positionen +8 bis +10 durch die reverskomplementäre Sequenz des Nukleotid-Tripletts ersetzt wurde, das in den Positionen -10 bis -8 des humanen RAG1 DNS-Ziels vorliegt,
(d) Auswählen der Varianten aus der zweiten Serie von Schritt (b), die in der Lage sind, eine mutierte I*-Cre*I Stelle zu spalten, wobei (i) das Nukleotid-Triplett in den Positionen -5 bis -3 der I*-Cre*I Stelle durch das Nukleotid-Triplett ersetzt wurde, das in den Positionen -5 bis -3 des humanen RAG1 DNS-Ziels vorliegt und (ii) das Nukleotid-Triplett in den Positionen +3 bis +5 durch die reverskomplementäre Sequenz des Nukleotid-Tripletts ersetzt wurde, das in den Positionen -5 bis -3 des humanen RAG1 DNS-Ziels vorliegt,
(e) Auswählen der Varianten aus der ersten Serie von Schritt (a), die in der Lage sind, eine mutierte I*-Cre*I Stelle zu spalten, wobei (i) das Nukleotid-Triplett in den Positionen +8 bis +10 der I*-Cre*I Stelle durch das Nukleotid-Triplett ersetzt wurde, das in den Positionen +8 bis +10 des humanen RAG1 DNS-Ziels vorliegt, und (ii) das Nukleotid-Triplett in den Positionen -10 bis -8 durch die reverskomplementäre Sequenz des Nukleotid-Tripletts ersetzt wurde, das in den Positionen +8 bis +10 des humanen RAG1 DNS-Ziels vorliegt,
(f) Auswählen der Varianten aus der zweiten Serie von Schritt (b), die in der Lage sind, eine mutierte I*-Cre*I Stelle zu spalten, wobei (i) das Nukleotid-Triplett in den Positionen +3 bis +5 der I*-Cre*I Stelle durch das Nukleotid-Triplett ersetzt wurde, das in den Positionen +3 bis +5 des humanen RAG1 DNS-Ziels vorliegt, und (ii) das Nukleotid-Triplett in den Positionen -5 bis -3 durch die reverskomplementäre Sequenz des Nukleotid-Tripletts ersetzt wurde, das in den Positionen +3 bis +5 des humanen RAG1 DNS-Ziels vorliegt,
(g) Kombinieren der Mutation(en) in den Positionen 26 bis 40 und 44 bis 77 zweier Varianten von Schritt (c) und Schritt (d) in einer einzigen Variante, um eine neue homodimere I*-Cre*I Variante zu erhalten, die eine Sequenz spaltet, wobei (i) das Nukleotid-Triplett in den Positionen -10 bis -8 mit dem Nukleotid-Triplett identisch ist, das in den Positionen -10 bis -8 des humanen RAG1 DNS-Ziels vorliegt, (ii) das Nukleotid-Triplett in den Positionen +8 bis +10 mit der reverskomplementären Sequenz des Nukleotid-Tripletts identisch ist, das in den Positionen -10 bis -8 des humanen RAG1 DNS-Ziels vorliegt, (iii) das Nukleotid-Triplett in den Positionen -5 bis -3 mit dem Nukleotid-Triplett identisch ist, das in den Positionen -5 bis -3 des humanen RAG1 DNS-Ziels vorliegt und (iv) das Nukleotid-Triplett in den Positionen +3 bis +5 mit der reverskomplementären Sequenz des Nukleotid-Tripletts identisch ist, das in den Positionen -5 bis -3 des humanen RAG1 DNS-Ziels vorliegt und/oder
(h) Kombinieren der Mutation(en) in den Positionen 26 bis 40 und 44 bis 77 zweier Varianten von Schritt (e) und Schritt (f) in einer einzigen Variante, um eine neue homodimere I*-Cre*I Variante zu erhalten, die eine Sequenz spaltet, wobei (i) das Nukleotid-Triplett in den Positionen +3 bis +5 mit dem Nukleotid-Triplett identisch ist, das in den Positionen +3 bis +5 des humanen RAG1 DNS-Ziels vorgibt, (ii) das Nukleotid-Triplett in den Positionen -5 bis -3 mit der reverskomplementären Sequenz des Nukleotid-Tripletts identisch ist, das in den Positionen +3 bis +5 des humanen RAG1 DNS-Ziels vorliegt, (iii) das Nukleotid-Triplett in den Positionen +3 bis +10 der I*-Cre*I Stelle durch das Nukleotid-Triplett ersetzt wurde, das in den Positionen +8 bis +10 des humanen RAG1 DNS-Ziels vorliegt und (iv) das Nukleotid-Triplett in den Positionen -10 bis -8 mit der reverskomplementären Sequenz des Nukleotid-Tripletts in den Positionen +8 bis +10 des humanen RAG1 DNS-Ziels identisch ist,
(i) Kombinieren der Varianten, die in den Schritten (g) und (h) enthalten wurden, um Heterodimere zu bilden, und
(j) Auswählen der Heretodimere von Schritt (i), die in der Lage sind, die DNS-Zielsequenzen von dem RAG1 Gen zu spalten.

2. Variante nach Anspruch 1, die ein Heterodimer ist, das in der Lage ist, eine DNS-Zielsequenz von einem humanen RAG1 Gen zu spalten, die aus der Gruppe ausgewählt ist, die aus den Sequenzen SEQ ID NO: 148 bis 177 besteht, wobei das Heterodimer aus einem ersten und einem zweiten Monomer besteht, die in den Positionen 28, 30, 32, 33, 38, 40, 44, 68, 70, 75 und 77 der I*-Cre*I Animosäuren aufweisen, die sind, wie in Tabelle XV angegeben:
| **Erstes Monomer** | **Zweites Monomer** |
|---|---|
| 28Q30N32S33Y38R40K44Y68E70S75R77V | 28K30R32Q33Y38Q40S44K68T70S75N77V |
| 28K30N32S33S38R40H44A68Y70S75Y77K | 28A30N32S33S38R40K44D68N70S75N77I |
| 28K30D32S33R38T40S44Y68S70S75S77D | 28K30N32T33C38Q40S44K68Y70S75Q77N |
| 28N30N32S33S38R40R44A68R70T75N77I | 28K30R32S33C38Q40S44A68Y70S75Y77K |
| 28N30N32S33S38R40R44Y68R70S75Q77V | 28K30K32S33A38Q40S44A68Y70S75Y77K |
| 28N30N32S33S38R40R44Y68R70S75Q77V | 28K30K32S33A38Q40S44A68S70S75D77R |
| 28N30N32S33S38R40R44Y68R70S75Q77V | 28K30K32S33A38Q40S44R68Y70S75D77T |
| 28N30N32S33S38R40R44Y68R70S75Q77V | 28K30R32S33N38Q40S44T68Y70S75Y77R |
| 28N30N32S33S38R40R44Y68R70S75Q77V | 28K30R32S33C38Q40S44A68Y70S75Y77K |
| 28N30N32S33S38R40R44Y68R70S75Q77V | 28K30R32S33N38Q40S44K68Y70S75Y77N |
| 28N30N32S33S38R40R44Y68R70S75Q77V | 28K30N32S33R38T40S44A68Y70S75Y77K |
| 28N30N32S33S38R40R44Y68R70S75Q77V | 28K30K32S33G38Q40S44A68Y70S75Y77K |
| 28N30N32S33S38R40R44A68R70N75N77I | 28K30K32S33A38Q40S44A68Y70S75Y77K |
| 28N30N32S33S38R40R44A68R70N75N77I | 28K30K32S33A38Q40S44A68S70S75D77R |
| 28N30N32S33S38R40R44A68R70N75N77I | 28K30R32S33N38Q40S44T68Y70S75Y77R |
| 28N30N32S33S38R40R44A68R70N75N77I | 28K30R32S33C38Q40S44A68Y70S75Y77K |
| 28N30N32S33S38R40R44A68R70N75N77I | 28K30R32S33N38Q40S44K68Y70S75Y77N |
| 28N30N32S33S38R40R44A68R70N75N77I | 28K30N32S33R38T40S44A68Y70S75Y77K |
| 28N30N32S33S38R40R44A68R70N75N77I | 28K30K32S33G38Q40S44A68Y70S75Y77K |
| 28Q30N32S33S38R40K44A68H70Q75N77I | 28K30K32S33A38Q40S44A68Y70S75Y77K |
| 28Q30N32S33S38R40K44A68H70Q75N77I | 28K30K32S33A38Q40S44A68S70S75D77R |
| 28Q30N32S33S38R40K44A68H70Q75N77I | 28K30R32S33N38Q40S44T68Y70S75Y77R |
| 28Q30N32S33S38R40K44A68H70Q75N77I | 28K30R32S33C38Q40S44A68Y70S75Y77K |
| 28Q30N32S33S38R40K44A68H70Q75N77I | 28K30R32S33N38Q40S44K68Y70S75Y77N |
| 28Q30N32S33S38R40K44A68H70Q75N77I | 28K30N32S33R38T40S44A68Y70S75Y77K |
| 28Q30N32S33S38R40K44A68H70Q75N77I | 28K30K32S33G38Q40S44A68Y70S75Y77K |
| 28K30H32S33M38A40S44A68R70S75Y77I | 28K30K32S33A38Q40S44A68Y70S75Y77K |
| 28K30H32S33M38A40S44A68R70S75Y77I | 28K30R32S33N38Q40S44A68Y70S75D77R |
| **Erstes Monomer** | **Zweites Monomer** |
|---|---|
| 28N30N32S33S38R40R44Y68R70S75Y77Q | 28K30K32S33A38Q40S44A68Y70S75Y77K |
| 28N30N32S33S38R40R44Y68R70S75Y77Q | 28K30K32S33A38Q40S44A68S70S75D77R |
| 28N30N32S33S38R40R44Y68R70S75Y77Q | 28K30K32S33A38Q40S44R68Y70S75D77T |
| 28N30N32S33S38R40R44Y68R70S75Y77Q | 28K30R32S33N38Q40S44T68Y70S75Y77R |
| 28N30N32S33S38R40R44Y68R70S75Y77Q | 28K30R32S33C38Q40S44A68Y70S75Y77K |
| 28N30N32S33S38R40R44Y68R70S75Y77Q | 28K30R32S33N38Q40S44K68Y70S75Y77N |
| 28N30N32S33S38R40R44Y68R70S75Y77Q | 28K30N32S33R38T40S44A68Y70S75Y77K |
| 28N30N32S33S38R40R44Y68R70S75Y77Q | 28K30K32S33G38Q40S44A68Y70S75Y77K |
| 28N30N32S33S38R40R44N68R70S75Y77V | 28K30K32S33A38Q40S44A68Y70S75Y77K |
| 28N30N32S33S38R40R44N68R70S75Y77V | 28K30K32S33A38Q40S44A68S70S75D77R |
| 28N30N32S33S38R40R44N68R70S75Y77V | 28K30R32S33N38Q40S44T68Y70S75Y77R |
| 28N30N32S33S38R40R44N68R70S75Y77V | 28K30R32S33C38Q40S44A68Y70S75Y77K |
| 28N30N32S33S38R40R44N68R70S75Y77V | 28K30R32S33N38Q40S44K68Y70S75Y77N |
| 28N30N32S33S38R40R44N68R70S75Y77V | 28K30N32S33R38T40S44A68Y70S75Y777K |
| 28N30N32S33S38R40R44N68R70S75Y77V | 28K30K32S33G38Q40S44A68Y70S75Y77K |
| 28Q30N32S33S38R40K44A68A70N75N77I | 28K30K32S33A38Q40S44A68Y70S75Y77K |
| 28Q30N32S33S38R40K44A68A70N75N77I | 28K30K32S33A38Q40S44A68S70S75D77R |
| 28Q30N32S33S38R40K44A68A70N75N77I | 28K30R32S33N38Q40S44T68Y70S75Y77R |
| 28Q30N32S33S38R40K44A68A70N75N77I | 28K30R32S33C38Q40S44A68Y70S75Y77K |
| 28Q30N32S33S38R40K44A68A70N75N77I | 28K30R32S33N38Q40S44K68Y70S75Y77N |
| 28Q30N32S33S38R40K44A68A70N75N77I | 28K30N32S33R38T40S44A68Y70S75Y77K |
| 28K30N32S33R38E40R44Q68R70S75D77K | 28K30G32S38G40S44R68R70S75Q77N |
| 28Q30N32S33S38R40K44N68R70S75R77N | 28K30D32S33R38T40S44A68N70S75Y77R |
| 28Q30N32S33R38Q40R44K68Y70S75Y77Q | 28K30G32S33Y38G40S44Q68R70S75R77Y |
| 28K30N32S33Y38Q40S44Q68R70R75E77R | 28K30N32S33S38W40S44N68R70S75R77N |
| 28K30N32S33G38A40S44Q68R70R75N77I | 28K30N32T33T38Q40S44N68R70S75R77N |
| 28K30G32S33Y38T40S44D68R70S75R77Q | 28R30N32S33S38Y40Q44T68R70S75E77R |
| 28K30N32S33G38Y40S44N68R70S75R77N | 28K30N32S33T38A40S44Q68A70N75D77I |
| 28K30N32G33R38Q40S44Q68R70R75D77I | 28K30G32S33Y38G40S44E68R70H75D77I |
| 28K30N32S33T38A40S44R68Y70S75Y77N | 28K30G32S33Y38T40S44K68N70A75D77I |
| 28K30S32G33R38Q40S44Q68R70R75N77I | 28K30N32S33S38R40D44Y68Y70S75Q77I |
| 28K30N32S33N38R40A44Q68R70R75N77I | 28K30N32S33R38A40S44D68R70S75R77Q |
| 28K30N32S33S38R40D44Q68R70S75N77I | 28Q30N32S33Y38R40K44Q68R70S75N77I |
| 28A30N32S33T38Q40R44Q68R70S75N77L | 28K30N32E33Y38Q40S44Y68R70S75D77V |
| 28Q30N32S33R38R40K44K68T70S75N77V | 28R30N32S33R38Y40Q44N68T70S75R77V |
| 28K30N32S33R38A40Q44D68R70N75D77I | 28K30N32S33C38T40S44N68R70S75R77N |
| 28K30N32S33Y38Q40S44Q68R70S75N77I | 28Q30N32S33Y38R40K44N68R70S75R77N |
| 28K30R32D33Y38Q40S44D68N70S75N77I | 28K30G32S33Y38H40S44N68R70S75R77D |
| 28K30N32S33S38D40S44T68Y70S75Y77K | 28R30N32S33A38Y40Q44T68Y70S75R77V |
| 28K30G32S33Y38T40S44Y68Y70S75Q77I | 28K30N32S33R38N40Q44Q68R70S75K77E |
| 28K30N32S33C38S40S44T68Y70S75Y77K | 28K30N32S33T38Q40S44Q68R70R75N77I |
| 28K30N32S33T38A40S44T68Y70S75Y77K | 28K30D32S33R38T40S44Q68R70R75N77I |
| 28K30G32S33Y38H40S44N68E70S75K77R | 28A30N32S33S38R40K44D68Y70S75S77R |
| 28Q30N32S33Y38R40K44Q68Y70S75R77Q | 28Q30N32S33Y38Q40K44A68N70S75Y77R |
| 28K30N32S33H38Q40Q44D68N70S75N77I | 28K30D32S33R38Q40S44N68Y70S75R77V |
| 28K30N32K33T38Q40S44N68Y70S75Y77Q | 28K30N32S33S38R40E44Y68Y70S75Q77I |
| 28K30N32S33Y38Q40S44A68G70N75D77I | 28K30N32S33W38Q40H44Y68R70S75N77V |
| 28N30N32S33S38R40R44Y68R70S75Q77V | 28K30R32S33N38Q40S44A68Y70S75D77R |

3. Variante nach Anspruch 1 oder 2, die mindestens eine Substitution umfasst, die ihre Bindungs- und/oder Spaltungseigenschaften gegenüber der DNS-Zielsequenz von einem humanen RAG1 Gen verbessert, wobei die Substitution in einer Position der I-*Cre*I vorliegt, die aus der Gruppe ausgewählt ist, die aus den Positionen 4, 6 19, 34, 43, 49, 50, 54, 66, 79, 80, 82, 85, 86, 87, 94, 96, 100, 103, 105, 107, 108, 114, 115, 116, 117, 125, 129, 131, 132, 139, 147, 150, 151, 153, 154, 155, 157, 159 und/oder 160 besteht.

4. Variante nach Anspruch 3, wobei die Substitutionen aus der Gruppe ausgewählt sind, die aus: G19S, G19A, F54L, S79G, F87L, V105A und I132V besteht.

5. Variante nach Anspruch 3 oder 4, die ein Heterodimer ist, das aus einem ersten und einem zweiten Monomer besteht, die in den Positionen 28, 30, 32, 33, 38, 40, 44, 68, 70, 75, 77 und in mindestens einer der Positionen der I*-Cre*I*,* die in Anspruch 3 definiert sind, Aminosäuren aufweisen, die sind, wie in Tabelle XVII angegeben:
| **Erstes Monomer** | **Zweites Monomer** |
|---|---|
| 28K30H32S33M38A40S44A68R70S75Y77T | 28K30R32S33N38Q40S44A68Y70S75Y77K117G |
| 28K30H32S33M38A40S44A68R70S75Y77T | 28K30R32S33N38Q40S44A68Y70S75Y77K107R 153G |
| 28K30H32S33M38A40S44A68R70S75Y77T | 28K30R32S33N34T38Q40S44A68Y70S75Y77K117K |
| 28K30H32S33M38A40S44A68R70S75Y77T | 28K30R32S33N38Q40S44A68Y70S75Y77K100R |
| 28K30H32S33M38A40S44A68R70S75Y77T | 28K30R32S33N38Q40S44A68Y70S75Y77K150T |
| 19S28N30N32S33S38R40R44Y68R70S75Q77V | 28K30R32S33N38Q40S44A68Y70S75D77R |
| 28N30N32S33S38R40R44Y68R70S75Q77V | 19S28K30R32S33N38Q40S44A68Y70S75D77R |

6. Variante nach einem der Ansprüche 1 bis 5, die ein Hetereodimer ist, das aus einem ersten und einem zweiten Monomer besteht, die aus den folgenden Paaren von Sequenzen ausgewählt sind: SEQ ID NO: 2 und 39, SEQ ID NO: 3 und 40, SEQ ID NO: 4 und 41, SEQ ID NO: 5 und 42, SEQ ID NO: 6 oder 10 und jede von den SEQ ID NO: 42 bis 49, SEQ ID NO: 7, 8, 11 und jede von den SEQ ID NO: 42 bis 44 und 46 bis 49, SEQ ID NO: 9 und jede von SEQ ID NO: 43,248 bis 253, SEQ ID NO: 12 und jede von den SEQ ID NO:15 und 42 bis 44 und 46 bis 48, SEQ ID NO: 13 und 50, SEQ ID NO: 14 und 51, SEQ ID NO: 15 und 52, SEQ ID NO: 16 und 53, SEQ ID NO: 17 und 54, SEQ ID NO: 18 und 55, SEQ ID NO: 19 und 56, SEQ ID NO: 20 und 57, SEQ ID NO: 21 und 58, SEQ ID NO: 22 und 59, SEQ ID NO: 23 und 60, SEQ ID NO: 24 und 61, SEQ ID NO: 25 und 62, SEQ ID NO: 26 und 63, SEQ ID NO: 27 und 64, SEQ ID NO; 28 und 65, SEQ ID NO: 29 und 66, SEQ ID NO: 30 und 67, SEQ ID NO: 31 und 68, SEQ ID NO: 32 und 69, SEQ ID NO: 33 und 70, SEQ ID NO: 34 und 71, SEQ ID NO: 35 und 72, SEQ ID NO: 36 und 73, SEQ ID NO: 37 und 74, SEQ ID NO: 38 und 75, SEQ ID NO: 6 und die Variante nach SEQ ID NO: 250, die ferner die G19S Mutation umfasst, wobei die Variante nach SEQ ID NO: 6 ferner die G19S Mutation und SEQ ID NO: 250 umfasst und wobei das Heterodimer in der Lage ist, eine DNS-Zielsequenz von einem humanen RAG1 Gen zu spalten, die aus der Gruppe ausgewählt ist, die aus den Sequenzen SEQ ID NO: 148 bis 177 besteht.

7. Chimäre single-chain Endonuklease, die von einer I*-Cre*I Variante nach einem der Ansprüche 1 bis 6 abgeleitet ist.

8. Polynukleotidfragment, das für mindestens eines der Monomere einer Variante nach einem der Ansprüche 1 bis 6 oder eine chimäre single-chain Endonuklease nach Anspruch 7 kodiert.

9. Expressionsvektor, der mindestens ein Polynukleotidfragment nach Anspruch 8 umfasst.

10. Vektor nach Anspruch 9, der ein Targeting-Konstrukt enthält, das in ein RAG1 Gen einzufügende Sequenz umfasst, die von Sequenzen flankiert ist, die homolog zu den Sequenzen des RAG1 Gens sind, die eine von der RAG1 Gen Zielsequenzen flankieren, die aus der Gruppe ausgewählt ist, die aus den Sequenzen SEQ ID NO: 148 bis 177 besteht.

11. Vektor nach Anspruch 10, wobei die einzufügende Sequenz eine Sequenz ist, die eine Mutation in dem humanen RAG1 Gen präpariert.

12. Vektor nach Anspruch 10, wobei die einzufügende Sequenz den humanen RAG1 offenen Leserahmen und eine Polyadenylierungsstelle umfasst, um die Transkription in 3' zu stoppen.

13. Vektor nach einem der Ansprüche 10 bis 12, wobei die zu der Sequenz des RAG Gens homologen Sequenzen, welche die RAG1 Gen Zielsequenz flankieren aus einem Fragment des humanen RAG1 Gens bestehen, das die Positionen 6 bis 205, 1603 bis 1802, 2219 bis 2418, 5181 bis 5380, 5222 bis 5421, 5499 bis 5698, 5709 bis 5908, 5936 bis 6135, 6049 bis 6248, 6097 bis 6296, 6212 bis 6411, 6270 bis 6469, 6521 bis 6720, 6559 bis 6758, 6667 bis 6866, 6710 bis 6909, 6853 bis 7052, 6976 bis 7175, 7012 bis 7211, 7168 bis 7367, 7207 bis 7406, 7231 bis 7430, 7478 bis 7677, 7622 bis 7821, 7709 bis 7908, 7920 bis 8119, 8144 bis 8343, 8149 bis 8348, 8252 bis 8451, und/oder 8271 to 8470 des humanen RAG1 Gens umfasst.

14. Zusammensetzung, die mindestens eine Variante nach einem der Ansprüche 1 bis 6, eine chimäre single-chain Endouklease nach Anspruch 7, und/oder mindestens einen Vektor nach einem der Ansprüche 9 bis 13 umfasst.

15. Verwendung mindestens einer Variante nach einem der Ansprüche 1 bis 6, einer chimären single-chain Endonuklease nach Anspruch 7, und/oder eines Vektors nach einem der Ansprüche 9 bis 13, zur Herstellung eines Medikaments zum Verändern, Verbessern oder Heilen eines SCID Syndroms, das mit einer Mutation in dem humanen RAG1 Gen in Zusammenhang steht, in einem Individuum, das dieses benötigt.

16. Wirtszelle, die mindestens ein Polynukleotidfragment nach Anspruch 8 oder einen Vektor nach einem der Ansprüche 9 bis 13 umfasst.

17. Nichthumanes transgenes Tier, das mindestens ein Polynukleotidfragment nach Anspruch 8 umfasst.

18. Transgene Pflanze, die mindestens ein Polynukleotidfragment nach Anspruch 8 umfasst.

19. Verwendung mindestens einer Variante nach einem der Ansprüche 1 bis 6, einer chimären single-chain Endonuklease nach Anspruch 7, eines Vektors nach einem der Ansprüche 9 bis 13, für Gentechnologie für nichttherapeutische Zwecke.

## Revendications

1. Variant d'I-*Cre*I comprenant deux monomères d'I-*Cre*I, **caractérisé en ce qu'**au moins l'un des deux monomères d'I-*Cre*I comporte au moins deux substitutions, une dans chacun des deux sous-domaines fonctionnels du domaine central LAGLIDADG situés respectivement aux positions 26 à 40 et 44 à 77 de la séquence d'acides aminés d'I-*Cre*I SEQ ID NO : 234, ledit variant étant capable de cliver une séquence d'ADN cible d'un gène RAG1 humain choisie dans le groupe constitué des séquences SEQ ID NO : 148 à 177, et pouvant être obtenu par un procédé comprenant au moins les étapes suivantes :
(a) la construction d'une première série de variants d'I-*Cre*I comportant au moins une substitution au niveau aux positions 26, 28, 30, 32, 33, 38 et/ou 40 d'un premier sous-domaine fonctionnel du domaine central LAGLIDADG situé aux positions 26 à 40 d'I-*Cre*I,
(b) la construction d'une seconde série de variants d'I-*Cre*I comportant au moins une substitution au niveau des positions 44, 68, 70, 75 et/ou 77 d'un second sous-domaine fonctionnel du domaine central LAGLIDADG situé aux positions 44 à 77 d'I-*Cre*I,
(c) la sélection des variants de la première série de l'étape (a) qui sont capables de cliver un site I-*Cre*I mutant dans lequel (i) le triplet nucléotidique aux positions -10 à -8 du site I*-Cre*I a été remplacé par le triplet nucléotidique qui est présent aux positions -10 à -8 dudit ADN cible RAG1 humain et (ii) le triplet nucléotidique aux positions +8 à +10 a été remplacé par la séquence complémentaire inverse du triplet nucléotidique qui est présent aux positions -10 à -8 dudit ADN cible RAG1 humain,
(d) la sélection des variants de la seconde série de l'étape (b) qui sont capables de cliver un site I*-Cre*I mutant dans lequel (i) le triplet nucléotidique aux positions -5 à -3 du site I-CreI a été remplacé par le triplet nucléotidique qui est présent aux positions -5 à -3 dudit ADN cible RAG1 humain et (ii) le triplet nucléotidique aux positions +3 à +5 a été remplacé par la séquence complémentaire inverse du triplet nucléotidique qui est présent aux positions -5 à -3 dudit ADN cible RAG1 humain,
(e) la sélection des variants de la première série de l'étape (a) qui sont capables de cliver un site I*-Cre*I mutant dans lequel (i) le triplet nucléotidique aux positions +8 à +10 du site I*-Cre*I a été remplacé par le triplet nucléotidique qui est présent aux positions +8 à +10 dudit ADN cible RAG1 humain et (ii) le triplet nucléotidique aux positions -10 à -8 a été remplacé par la séquence complémentaire inverse du triplet nucléotidique qui est présent aux positions +8 à +10 dudit ADN cible RAG1 humain,
(f) la sélection des variants de la seconde série de l'étape (b) qui sont capables de cliver un site I*-Cre*I mutant dans lequel (i) le triplet nucléotidique aux positions +3 à +5 du site I*-Cre*I a été remplacé par le triplet nucléotidique qui est présent aux positions +3 à +5 dudit ADN cible RAG1 humain et (ii) le triplet nucléotidique aux positions -5 à -3 a été remplacé par la séquence complémentaire inverse du triplet nucléotidique qui est présent aux positions +3 à +5 dudit ADN cible RAG1 humain,
(g) la combinaison dans un seul variant, des mutations aux positions 26 à 40 et 44 à 77 de deux variants issus de l'étape (c) et de l'étape (d), pour obtenir un nouveau variant homodimère d'I-*Cre*I qui clive une séquence dans laquelle (i) le triplet nucléotidique aux positions -10 à -8 est identique au triplet nucléotidique qui est présent aux positions -10 à -8 dudit ADN cible RAG1 humain, (ii) le triplet nucléotidique aux positions +8 à +10 est identique à la séquence complémentaire inverse du triplet nucléotidique qui est présent aux positions -10 à -8 dudit ADN cible RAG1 humain, (iii) le triplet nucléotidique aux positions -5 à -3 est identique au triplet nucléotidique qui est présent aux positions -5 à -3 dudit ADN cible RAG1 humain et (iv) le triplet nucléotidique aux positions +3 à +5 est identique à la séquence complémentaire inverse du triplet nucléotidique qui est présent aux positions -5 à -3 dudit ADN cible RAG1 humain, et/ou
(h) la combinaison dans un seul variant, des mutations aux positions 26 à 40 et 44 à 77 de deux variants issus de l'étape (e) et de l'étape (f), pour obtenir un nouveau variant homodimère d'I-*Cre*I qui clive une séquence dans laquelle (i) le triplet nucléotidique aux positions +3 à +5 est identique au triplet nucléotidique qui est présent aux positions +3 à +5 dudit ADN cible RAG1 humain, (ii) le triplet nucléotidique aux positions -5 à -3 est identique à la séquence complémentaire inverse du triplet nucléotidique qui est présent aux positions +3 à +5 dudit ADN cible RAG1 humain, (iii) le triplet nucléotidique aux positions +8 à +10 du site I*-Cre*I a été remplacé par le triplet nucléotidique qui est présent aux positions +8 à +10 dudit ADN cible RAG1 humain et (iv) le triplet nucléotidique aux positions -10 à -8 est identique à la séquence complémentaire inverse du triplet nucléotidique qui est présent aux positions +8 à +10 dudit ADN cible RAG1 humain,
(i) la combinaison des variants obtenus dans les étapes (g) et (h) pour former des hétérodimères, et
(j) la sélection des hétérodimères issus de l'étape (i) qui sont capables de cliver lesdites séquences d'ADN cible du gène RAG1.

2. Variant selon la revendication 1, qui est un hétérodimère capable de cliver une séquence d'ADN cible d'un gène RAG1 humain choisie dans le groupe constitué des séquences SEQ ID NO : 148 à 177, ledit hétérodimère étant constitué d'un premier et d'un second monomères ayant les acides aminés au niveau des positions 28, 30, 32, 33, 38, 40, 44, 68, 70, 75 et 77 d' I-*Cre*I qui sont tels qu'indiqués dans le tableau XV :
| **Premier monomère** | **Second monomère** |
|---|---|
| 28Q30N32S33Y38R40K44Y68E70S75R77V | 28K30R32Q33Y38Q40S44K68T70S75N77V |
| 28K30N32S33S38R40H44A68Y70S75Y77K | 28A30N32S33S38R40K44D68N70S75N77I |
| 28K30D32S33R38T40S44Y68S70S75S77D | 28K30N32T33C38Q40S44K68Y70S75Q77N |
| 28N30N32S33S38R40R44A68R70T75N77I | 28K30R32S33C38Q40S44A68Y70S75Y77K |
| 28N30N32S33S38R40R44Y68R70S75Q77V | 28K30K32S33A38Q40S44A68Y70S75Y77K |
| 28N30N32S33S38R40R44Y68R70S75Q77V | 28K30K32S33A38Q40S44A68S70S75D77R |
| 28N30N32S33S38R40R44Y68R70S75Q77V | 28K30K32S33A38Q40S44R68Y70S75D77T |
| 28N30N32S33S38R40R44Y68R70S75Q77V | 28K30R35S33N38Q40S44T68Y70S75Y77R |
| 28N30N32S33S38R40R44Y68R70S75Q77V | 28K30R32S33C38Q40S44A68Y70S75Y77K |
| 28N30N32S33S38R40R44Y68R70S75Q77V | 28K30R32S33N38Q40S44K68Y70S75Y77N |
| 28N30N32S33S38R40R44Y68R70S75Q77V | 28K30N32S33R38T40S44A68Y70S75Y77K |
| 28N30N32S33S38R40R44Y68R70S75Q77V | 28K30K32S33G38Q40S44A68Y70S75Y77K |
| 28N30N32S33S38R40R44A68R70N75N77I | 28K30K32S33A38Q40S44A68Y70S75Y77K |
| 28N30N32S33S38R40R44A68R70N75N77I | 28K30K32S33A38Q40S44A68S70S75D77R |
| 28N30N32S33S38R40R44A68R70N75N77I | 28k30R32S33N38Q40S44T68Y70S75Y77R |
| 28N30N32S33S38R40R44A68R70N75N77I | 28K30R32S33C38Q40S44A68Y70S75Y77K |
| 28N30N32S33S38R40R44A68R70N75N77I | 28K30R32S33N38Q40S44K68Y70S75Y77N |
| 28N30N32S33S38R40R44A68R70N75N77I | 28K30N32S33R38T40S44A66Y70S75Y77K |
| 28N30N32S33S38R40R44A68R70N75N77I | 28K30K32S33G38Q40S44A68Y70S75Y77K |
| 28Q30N32S33S38R40K44A68H70Q75N77I | 28K30K32S33A38Q40S44A68Y70S75Y77K |
| 28Q30N32S33S38R40K44A68H70Q75N77I | 28K30K32S33A38Q40S44A68S70S75D77R |
| 28Q30N32S33S38R40K44A68H70Q75N77I | 28K30R32S33N38Q40S44T68Y70S75Y77R |
| 28Q30N32S33S38R40K44A68H70Q75N77I | 28K30R32S33C38Q40S44A68Y70S75Y77K |
| 28Q30N32S33S38R40K44A68H70Q75N77I | 28K30R32S33N38Q40S44K68Y70S75Y77N |
| 28Q30N32S33S38R40K44A68H70Q75N77I | 28K30N32S33R38T40S44A68Y70S75Y77K |
| 28Q30N32S33S38R40K44A68H70Q75N77I | 28K30K32S33G38Q40S44A68Y70S75Y77K |
| 28K30H32S33M38A40S44A68R70S75Y77T | 28K30K32S33A38Q40S44A68Y70S75Y77K |
| 28K30H32S33M38A40S44A68R70S75Y77T | 28K30R32S33N38Q40S44A68Y70S75D77R |
| **Premier monomère** | **Second monomère** |
|---|---|
| 28N30N32S33S38R40R44Y68R70S75Y77Q | 28K30K32S33A38Q40S44A68Y70S75Y77K |
| 28N30N32S33S38R40R44Y68R70S75Y77Q | 28K30K32S33A38Q40S44A68S70S75D77R |
| 28N30N32S33S38R40R44Y68R70S75Y77Q | 28K30K32S33A38Q40S44R68Y70S75D77T |
| 28N30N32S33S38R40R44Y68R70S75Y77Q | 28K30R32S33N38Q40S44T68Y70S75Y77R |
| 28N30N32S33S38R40R44Y68R70S75Y77Q | 28K30R32S33C380Q40S44A68Y70S75Y77K |
| 28N30N32S33S38R40R44Y68R70S75Y77Q | 28K30R32S33N38Q40S44K68Y0S7577N |
| 28N30N32S33S38R40R44Y68R70S75Y77Q | 28K30N32S33R38T40S44A68Y70S75Y77K |
| 28N30N32S33S38R40R44Y68R70S75Y77Q | 28K30K32S33G38Q40S44A68Y70S75Y77K |
| 28N30N32S33S38R40R44N68R70S75Y77V | 28k30K32S33A38Q40S44A68Y70S75Y77K |
| 28N30N32S33S38R40R44N68R70S75Y77V | 28K30K32S33A38Q40S44A68S70S75D77R |
| 28N30N32S33S38R40R44N68R70S75Y77V | 28K30R32S33N38Q40S44T68Y70S75Y77R |
| 28N30N32S33S38R40R44N68R70S75Y77V | 28K30R32S33C38Q40S44A68Y70S75Y77K |
| 28N30N32S33S38R40R44N68R40S75Y77V | 28K30R32S33N38Q40S44K68Y70S75Y77N |
| 26N30N32S33S38R40R44N68R70S75Y77V | 28K30N32S33R38T40S44A68Y70S75Y77K |
| 28N30N32S33S38R40R44N68R70S75Y77V | 28k30K32S33G38Q40S44A68Y70S75Y77K |
| 28Q30N32S33S38R40K44A68A70N75N77I | 28K3QK32S33A38Q40S44A68Y70S75Y77K |
| 28Q30N32S33S38R40K44A68A70N75N77I | 28k30K32S33A38Q40S44A68S7Q75D77R |
| 28Q30N32S33S38R40K44A68A70N75N77I | 28K30R32S33N38Q40S44T68Y70S75Y77R |
| 28Q30N32S33S38R40K44A68A70N75N77I | 28K30R32S33C38Q40S4AA68Y70S75Y77K |
| 28Q30N32S33S38R40K44A68A70N75N77I | 28K30R32S33N38Q40S44K68Y70S75Y77N |
| 28Q30N32S33S38R40K44A68A70N75N77I | 28K30N32S33R38T40S44A68Y70S75Y77K |
| 28K30N32S33R38E40R44Q68R70S75D77K | 28K30G32S38G40S44R68R70S75Q77N |
| 28Q30N32S33S38R40K44N68R70S75R77N | 28K30D32S33R38T40S44A68N70S75Y77R |
| 28Q30N32S33R38Q40R44K68Y70S75Y77Q | 28K30G32S33Y38G40S44Q68R70S75R77Y |
| 28K30N32S33Y38Q40S44Q68R70R75E77R | 28K30N32S33S38W40S44N68R70S75R77N |
| 28K30N32S33G38A40S44Q68R70R75N77I | 28K30N32T33T38Q40S44N68R70S75R77N |
| 28K30G32S33Y38T40S44D68R70S75R77Q | 28R30N32S33S38Y40Q44T68R70S75E77R |
| 28K30N32S33G38Y40S44N68R70S75R77N | 28K30N32S33T38A40S44Q68A70N75D77I |
| 28K30N32G33R38Q40S44Q68R70R75D77I | 28K30G32S33Y38G40S44E68R70H75D77I |
| 28K30N32S33T38A40S44R68Y70S75Y77N | 28K30G32S33Y38T40S44K68N70A75D77I |
| 28K30S32G33R38Q40S44Q68R70R75N77I | 28K30N32S33S38R40D44Y68Y70S75Q77I |
| 28K30N32S33N38R40A44Q68R70R75N77I | 28K30N32S33R38A40S44O68R70S75R77Q |
| 28K30N32S33S38R40D44Q68R70S75N77I | 28Q30N32S33Y38R40K44Q68R70S75N77I |
| 28A30N32S33T38Q40R44Q68R70S75N77I | 28K30N32E33Y38Q40S44Y68R70S75D77V |
| 28Q30N32S33R38R40K44K68T70S75N77V | 28R30N32S33R38Y40Q44N68T70S75R77V |
| 28K30N32S33R38A40Q44D68R70N75D77I | 28K30N32S33C38T40S44N68R70S75R77N |
| 28K30N32S33Y38Q40S44Q68R70S75N77I | 28Q30N32S33Y38R40K44N68R70S75R77N |
| 28K30R32D33Y38Q40S44D68N70S75N77I | 28K30G32S33Y38H40S44N68R70S75R77D |
| 28K30N32S33S38D40S44T68Y70S75Y77K | 28R30N32S33A38Y40Q44T68Y70S75R77V |
| 28K30G32S33Y38T40S44Y68Y70S75Q77I | 28K30N32S33R38N40Q44Q68R70S75K77E |
| 28K30N32S33C38S40S44T68Y70S75Y77K | 28K30N32S33T38Q40S44Q68R70R75N77I |
| 28K30N32S33T38A40S44T68Y70S75Y77K | 28K30D32S33R38T40S44Q68R70R75N77I |
| 28K30G32S33Y38H40S44N68E70S75K77R | 28A30N32S33S38R40K44D68Y70S75S77R |
| 28Q30N32S33Y38R40K44Q68Y70S75R77Q | 28Q30N32S33Y38Q40K44A68N70S75Y77R |
| 28K30N32S33H38Q40Q44D68N70S75N77I | 28K30D32S33R38Q40S44N68Y70S75R77V |
| 28K30N32K33T38Q40S44N68Y70S75Y77Q | 28K30N32S33S38R40E44Y68Y70S75Q77I |
| 28K30N32S33Y38Q40S44A68G70N75D77I | 28K30N32S33W38Q40H44Y68R70S75N77V |
| 28N30N32S33S38R40R44Y68R70S75Q77V | 28K30R32S33N380Q40S44A68Y70S75D77R |

3. Variant selon la revendication 1 ou la revendication 2, qui comprend au moins une substitution qui améliore ses propriétés de liaison et/ou de clivage envers ladite séquence d'ADN cible d'un gène RAG1 humain, ladite substitution étant au niveau d'une position d'I-*Cre*I qui est choisie dans le groupe constitué des positions 4, 6, 19, 34, 43, 49, 50, 54, 66, 79, 80, 82, 85, 86, 87, 94, 96, 100, 103, 105, 107, 108, 114, 115, 116, 117, 125, 129, 131, 132, 139, 147, 150, 151, 153, 154, 155, 157, 159 et/ou 160.

4. Variant selon la revendication 3, dans lequel lesdites substitutions sont choisies dans le groupe constitué de : G19S, G19A, F54L, S79G, F87L, V105A et I132V.

5. Variant selon la revendication 3 ou la revendication 4, qui est un hétérodimère constitué d'un premier et d'un second monomères ayant les acides aminés au niveau des positions 28, 30, 32, 33, 38, 40, 44, 68, 70, 75, 77 et d'au moins l'une des positions d'I-*Cre*I définies dans la revendication 3, qui sont tels qu'indiqués dans le tableau XVII :
| **Premier monomère** | **Second monomère** |
|---|---|
| 28K30H32S33M38A40S44A68R70S75Y77T | 28K30R32S33N38Q40S44A68Y70S75Y77K117G |
| 28K30H32S33M38A40S44A68R70S75Y77T | 28K30R32S33N38Q40S44A68Y70S75Y77K107R 153G |
| 28K30H32S33M38A40S44A68R70S75Y77T | 28K30R32S33N34T38Q40S44A68Y70S75Y77K117K |
| 28K30H32S33M38A40S44A68R70S75Y77T | 28K30R32S33N38Q40S44A68Y70S75Y77K100R |
| 28K30H32S33M38A40S44A68R70S75Y77T | 28K30R32S33N38Q40S44A68Y70S75Y77K150T |
| 19S28N30N32S33S38R40R44Y68R70S75Q77V | 28K30R32S33N36Q40S44A68Y70S75D77R |
| 28N30N32S33S38R40R44Y68R70S75Q77V | 19S28K30R32S33N38Q40S44A68Y70S75D77R |

6. Variant selon l'une quelconque des revendications 1 à 5, qui est un hétérodimère constitué ) d'un premier et d'un second monomères choisis parmi les paires de séquences suivantes : SEQ ID NO : 2 et 39, SEQ ID NO : 3 et 40, SEQ ID NO : 4 et 41, SEQ ID NO : 5 et 42, SEQ ID NO : 6 ou 10 et l'une quelconque des SEQ ID NO : 42 à 49, SEQ ID NO : 7, 8, 11 et l'une 5 quelconque des SEQ ID NO : 42 à 44 et 46 à 49, SEQ ID NO : 9 et l'une quelconque des SEQ ID NO : 43, 248 à 253, SEQ ID NO : 12 et l'une quelconque des SEQ ID NO : 42 à 44 et 46 à 48, SEQ ID NO : 13 et 50, SEQ ID NO : 14 et 51, SEQ ID NO : 15 et 52, SEQ ID NO : 16 et 53, SEQ ID NO : 17 et 54, SEQ ID NO : 18 et 55, SEQ ID NO : 19 et 56, SEQ ID NO : 20 et 57, SEQ ID NO : 21 et 58, SEQ ID NO : 22 et 59, SEQ ID NO : 23 et 60, SEQ ID NO : 24 et 61, SEQ ID NO : 25 et 62, SEQ ID NO : 26 et 63, SEQ ID NO : 27 et 64, SEQ ID NO : 28 et 65, SEQ ID NO : 29 et 66, SEQ ID NO : 30 et 67, SEQ ID NO : 31 et 68, SEQ ID NO : 32 et 69, SEQ ID NO : 33 et 70, SEQ ID NO : 34 et 71, SEQ ID NO : 35 et 72, SEQ ID NO : 36 et 73, SEQ ID NO : 37 et 74, SEQ ID NO : 38 et 75, SEQ ID NO : 6 et le variant de SEQ ID NO : 250 comprenant en outre la mutation G19S, le variant de SEQ ID NO : 6 comprenant en outre la mutation G19S et SEQ ID NO : 250, et où ledit hétérodimère est capable de cliver une séquence d'ADN cible d'un gène RAG1 humain choisie dans le groupe constitué des séquences SEQ ID NO : 148 à 177.

7. Endonucléase chimérique à chaîne unique dérivée d'un variant d'I-*Cre*I selon l'une quelconque des revendications 1 à 6.

8. Fragment polynucléotidique codant pour au moins l'un des monomères d'un variant selon l'une quelconque des revendications 1 à 6 ou l'endonucléase chimérique à chaîne unique selon la revendication 7.

9. Vecteur d'expression comprenant au moins un fragment polynucléotidique selon la revendication 8.

10. Vecteur selon la revendication 9, qui comprend une construction de ciblage comprenant une séquence à introduire dans un gène RAG1 humain flanquée de séquences homologues aux séquences du gène RAG1 flanquant l'une des séquences cibles du gène RAG1 choisies dans le groupe constitué des séquences SEQ ID NO : 148 à 177.

11. Vecteur selon la revendication 10, dans lequel ladite séquence à introduire est une séquence qui répare une mutation dans le gène RAG1 humain.

12. Vecteur selon la revendication 10, dans lequel la séquence à introduire comprend le cadre de lecture ouvert du RAG1 humain et un site de polyadénylation pour stopper la transcription en 3'.

13. Vecteur selon l'une quelconque des revendications 10 à 12, dans lequel lesdites séquences homologues aux séquences du gène RAG flanquant la séquence cible du gène RAG1 sont constituées d'un fragment du gène RAG1 humain comprenant les positions : 6 à 205, 1603 à 1802, 2219 à 2418, 5181 à 5380, 5222 à 5421, 5499 à 5698, 5709 à 5908, 5936 à 6135, 6049 à 6248, 6097 à 6296, 6212 à 6411, 6270 à 6469, 6521 à 6720, 6559 à 6758, 6667 à 6866, 6710 à 6909, 6853 à 7052, 6976 à 7175, 7012 à 7211, 7168 à 7367, 7207 à 7406, 7231 à 7430, 7478 à 7677, 7622 à 7821, 7709 à 7908, 7920 à 8119, 8144 à 8343, 8149 à 8348, 8252 à 8451 et/ou 8271 à 8470 dudit gène RAG1 humain.

14. Composition comprenant au moins un variant selon l'une quelconque des revendications 1 à 6, une endonucléase chimérique à chaîne unique selon la revendication 7 et/ou au moins un vecteur selon l'une quelconque des revendications 9 à 13.

15. Utilisation d'au moins un variant selon l'une quelconque des revendications 1 à 6, d'une endonucléase chimérique à chaîne unique selon la revendication 7 et/ou d'un vecteur selon l'une quelconque des revendications 9 à 13, pour la préparation d'un médicament destiné à la prévention, l'amélioration ou la guérison d'un syndrome d'IDCS associé à une mutation dans le gène RAG1 humain, chez un individu en ayant besoin.

16. Cellule hôte qui comprend au moins un fragment polynucléotidique selon la revendication 8 ou un vecteur selon l'une quelconque des revendications 9 à 13.

17. Animal transgénique non humain comprenant au moins un fragment polynucléotidique selon la revendication 8.

18. Plante transgénique comprenant au moins un fragment polynucléotidique selon la revendication 8.

19. Utilisation d'au moins un variant selon l'une quelconque des revendications 1 à 6, d'une endonucléase chimérique à chaîne unique selon la revendication 7, d'un vecteur selon l'une quelconque des revendications 9 à 13, pour une modification de génome, pour des objectifs non thérapeutiques.


### SEQUENCE LISTING

<110> CELLECTIS ARNOULD, Sylvain GRIZOT, sylvestre
<120> MEGANUCLEASE VARIANTS CLEAVING A DNA TARGET SEQUENCE FROM A RAG
   GENE AND USES THEREOF
<130> BLOcpHLP1546-12
<160> 253
<170> PatentIn version 3.3
<210> 1
   <211> 24
   <212> DNA
   <213> Artificial
<220>
   <223> C1221
<400> 1
   tcaaaacgtc gtacgacgtt ttga 24
<210> 2
   <211> 163
   <212> PRT
   <213> Artificial
<220>
   <223> Variant cleaving RAG1
<400> 2
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Gln Pro Asn Gln Ser
   20 25 30
   Tyr Lys Phe Lys His Arg Leu Lys Leu Ala Phe Tyr Val Thr Gln Lys
   35 40 45
   Thr Gln Arg Arg Trp Phe Leu Asp Lys Leu Val Asp Glu Ile Gly Val
   50 55 60
   Gly Tyr Val Glu Asp Ser Gly Ser Val Ser Arg Tyr Val Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 3
   <211> 163
   <212> PRT
   <213> Artificial
<220>
   <223> Variant cleaving RAG1
<400> 3
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Ser Lys Phe Lys His Arg Leu His Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Tyr Tyr Lys Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 4
   <211> 163
   <212> PRT
   <213> Artificial
<220>
   <223> variant cleaving RAG1
<400> 4
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asp Gln Ser
               20                  25                  30
   Arg Lys Phe Lys His Thr Leu Ser Leu Ala Phe Tyr Val Thr Gln Lys
           35                  40                  45
   Thr Gln Arg Arg Trp Phe Leu Asp Lys Leu Val Asp Glu Ile Gly Val
       50                  55                  60
   Gly Tyr Val Ser Asp Ser Gly Ser Val Ser Ser Tyr Asp Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 5
   <211> 163
   <212> PRT
   <213> Artificial
<220>
   <223> Variant cleaving RAG1
<400> 5
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Asn Pro Asn Gln Ser
   20 25 30
   Ser Lys Phe Lys His Arg Leu Arg Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Thr Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 6
   <211> 163
   <212> PRT
   <213> Artificial
<220>
   <223> Variant cleaving RAG1
<400> 6
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Asn Pro Asn Gln Ser
   20 25 30
   Ser Lys Phe Lys His Arg Leu Arg Leu Ala Phe Tyr Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Gln Tyr Val Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 7
   <211> 163
   <212> PRT
   <213> Artificial
<220>
   <223> Variant cleaving RAG1
<400> 7
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Asn Pro Asn Gln Ser
   20 25 30
   Ser Lys Phe Lys His Arg Leu Arg Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Asn Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 8
   <211> 163
   <212> PRT
   <213> Artificial
<220>
   <223> Variant cleaving RAG1
<400> 8
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Gln Pro Asn Gln Ser
   20 25 30
   Ser Lys Phe Lys His Arg Leu Lys Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val His Asp Gln Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 9
   <211> 163
   <212> PRT
   <213> Artificial
<220>
   <223> Variant cleaving RAG1
<400> 9
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro His Gln Ser
               20                  25                  30
   Met Lys Phe Lys His Ala Leu Ser Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Ser Gly Ser Val Ser Tyr Tyr Thr Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 10
   <211> 163
   <212> PRT
   <213> Artificial
<220>
   <223> Variant cleaving RAG1
<400> 10
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Asn Pro Asn Gln Ser
               20                  25                  30
   Ser Lys Phe Lys His Arg Leu Arg Leu Ala Phe Tyr Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Tyr Tyr Gln Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 11
   <211> 163
   <212> PRT
   <213> Artificial
<220>
   <223> Variant cleaving RAG1
<400> 11
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Asn Pro Asn Gln Ser
               20                  25                  30
   Ser Lys Phe Lys His Arg Leu Arg Leu Ala Phe Asn Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Ser Gly Ser Val Ser Tyr Tyr Val Leu Ser Glu
   65                  70                  75                  80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 12
   <211> 163
   <212> PRT
   <213> Artificial
<220>
   <223> Variant cleaving RAG1
<400> 12
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Gln Pro Asn Gln Ser
               20                  25                  30
   Ser Lys Phe Lys His Arg Leu Lys Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Ala Asp Asn Gly Ser Val Ser Asn Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 13
   <211> 163
   <212> PRT
   <213> Artificial
<220>
   <223> Variant cleaving RAG1
<400> 13
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Arg Lys Phe Lys His Glu Leu Arg Leu Ala Phe Gln Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Ser Gly Ser Val Ser Asp Tyr Lys Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 14
   <211> 163
   <212> PRT
   <213> Artificial
<220>
   <223> Variant cleaving RAG1
<400> 14
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Gln Pro Asn Gln Ser
   20 25 30
   Ser Lys Phe Lys His Arg Leu Lys Leu Ala Phe Asn Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Arg Tyr Asn Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 15
   <211> 163
   <212> PRT
   <213> Artificial
<220>
   <223> variant cleaving RAG1
<400> 15
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Gln Pro Asn Gln Ser
   20 25 30
   Arg Lys Phe Lys His Gln Leu Arg Leu Ala Phe Lys Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Gln Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 16
   <211> 163
   <212> PRT
   <213> Artificial
<220>
   <223> Variant cleaving RAG1
<400> 16
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Tyr Lys Phe Lys His Gln Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Glu Tyr Arg Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 17
   <211> 163
   <212> PRT
   <213> Artificial
<220>
   <223> variant cleaving RAG1
<400> 17
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Gly Lys Phe Lys His Ala Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 18
   <211> 163
   <212> PRT
   <213> Artificial
<220>
   <223> Variant cleaving RAG1
<400> 18
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Gly Gln Ser
   20 25 30
   Tyr Lys Phe Lys His Thr Leu Ser Leu Ala Phe Asp Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Arg Tyr Gln Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 19
   <211> 163
   <212> PRT
   <213> Artificial
<220>
   <223> Variant cleaving RAG1
<400> 19
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Gly Lys Phe Lys His Tyr Leu Ser Leu Ala Phe Asn Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Ser Gly Ser Val Ser Arg Tyr Asn Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 20
   <211> 163
   <212> PRT
   <213> Artificial
<220>
   <223> Variant cleaving RAG1
<400> 20
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Gly
               20                  25                  30
   Arg Lys Phe Lys His Gln Leu Ser Leu Ala Phe Gln Val Thr Gln Lys
           35                  40                  45
   Thr Gln Arg Arg Trp Phe Leu Asp Lys Leu Val Asp Glu Ile Gly Val
   50 55 60
   Gly Tyr Val Arg Asp Arg Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 21
   <211> 163
   <212> PRT
   <213> Artificial
<220>
   <223> variant cleaving RAG1
<400> 21
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Thr Lys Phe Lys His Ala Leu Ser Leu Ala Phe Arg Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Asn Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 22
   <211> 163
   <212> PRT
   <213> Artificial
<220>
   <223> Variant cleaving RAG1
<400> 22
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Gly
   20 25 30
   Arg Lys Phe Lys His Gln Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 23
   <211> 163
   <212> PRT
   <213> Artificial
<220>
   <223> Variant cleaving RAG1
<400> 23
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Asn Lys Phe Lys His Arg Leu Ala Leu Ala Phe Gln Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Arg Gly Ser Val Ser Asn Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 24
   <211> 163
   <212> PRT
   <213> Artificial
<220>
   <223> Variant cleaving RAG1
<400> 24
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Ser Lys Phe Lys His Arg Leu Asp Leu Ala Phe Gln Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Ser Gly Ser Val Ser Asn Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 25
   <211> 163
   <212> PRT
   <213> Artificial
<220>
   <223> Variant cleaving RAG1
<400> 25
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Ala Pro Asn Gln Ser
               20                  25                  30
   Thr Lys Phe Lys His Gln Leu Arg Leu Ala Phe Gln Val Thr Gln Lys
           35                  40                  45
   Thr Gln Arg Arg Trp Phe Leu Asp Lys Leu Val Asp Glu Ile Gly Val
   50 55 60
   Gly Tyr Val Arg Asp Ser Gly Ser Val Ser Asn Tyr Leu Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 26
   <211> 163
   <212> PRT
   <213> Artificial
<220>
   <223> variant cleaving RAG1
<400> 26
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Gln Pro Asn Gln Ser
   20 25 30
   Arg Lys Phe Lys His Arg Leu Lys Leu Ala Phe Lys Val Thr Gln Lys
   35 40 45
   Thr Gln Arg Arg Trp Phe Leu Asp Lys Leu Val Asp Glu Ile Gly Val
   50 55 60
   Gly Tyr Val Thr Asp Ser Gly Ser Val Ser Asn Tyr Val Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro

<210> 27
   <211> 163
   <212> PRT
   <213> Artificial
<220>
   <223> Variant cleaving RAG1
<400> 27
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Ala Leu Gln Leu Ala Phe Asp Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Asn Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 28
   <211> 163
   <212> PRT
   <213> Artificial
<220>
   <223> Variant cleaving RAG1
<400> 28
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Tyr Lys Phe Lys His Gln Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 29
   <211> 163
   <212> PRT
   <213> Artificial
<220>
   <223> Variant cleaving RAG1
<400> 29
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Arg Gln Asp
               20                  25                  30
   Tyr Lys Phe Lys His Gln Leu Ser Leu Ala Phe Asp Val Thr Gln Lys
           35                  40                  45
   Thr Gln Arg Arg Trp Phe Leu Asp Lys Leu Val Asp Glu Ile Gly Val
       50                  55                  60
   Gly Tyr Val Asn Asp Ser Gly Ser Val Ser Asn Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 30
   <211> 163
   <212> PRT
   <213> Artificial
<220>
   <223> Variant cleaving RAG1
<400> 30
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Ser Lys Phe Lys His Asp Leu Ser Leu Ala Phe Thr Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Lys Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 31
   <211> 163
   <212> PRT
   <213> Artificial
<220>
   <223> Variant cleaving RAG1
<400> 31
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Gly Gln Ser
   20 25 30
   Tyr Lys Phe Lys His Thr Leu Ser Leu Ala Phe Tyr Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Gln Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 32
   <211> 163
   <212> PRT
   <213> Artificial
<220>
   <223> Variant cleaving RAG1
<400> 32
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Cys Lys Phe Lys His Ser Leu Ser Leu Ala Phe Thr Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Lys Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 33
   <211> 163
   <212> PRT
   <213> Artificial
<220>
   <223> Variant cleaving RAG1
<400> 33
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Thr Lys Phe Lys His Ala Leu Ser Leu Ala Phe Thr Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Lys Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 34
   <211> 163
   <212> PRT
   <213> Artificial
<220>
   <223> variant cleaving RAG1
<400> 34
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Gly Gln Ser
   20 25 30
   Tyr Lys Phe Lys His His Leu Ser Leu Ala Phe Asn Val Thr Gln Lys
   35 40 45
   Thr Gln Arg Arg Trp Phe Leu Asp Lys Leu Val Asp Glu Ile Gly Val
   50 55 60
   Gly Tyr Val Glu Asp Ser Gly Ser Val Ser Lys Tyr Arg Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 35
   <211> 163
   <212> PRT
   <213> Artificial
<220>
   <223> variant cleaving RAG1
<400> 35
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Gln Pro Asn Gln Ser
   20 25 30
   Tyr Lys Phe Lys His Arg Leu Lys Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Arg Tyr Gln Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 36
   <211> 163
   <212> PRT
   <213> Artificial
<220>
   <223> variant cleaving RAG1
<400> 36
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   His Lys Phe Lys His Gln Leu Gln Leu Ala Phe Asp Val Thr Gln Lys
   35 40 45
   Thr Gln Arg Arg Trp Phe Leu Asp Lys Leu Val Asp Glu Ile Gly Val
   50 55 60
   Gly Tyr Val Asn Asp Ser Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 37
   <211> 163
   <212> PRT
   <213> Artificial
<220>
   <223> Variant cleaving RAG1
<400> 37
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Lys
   20 25 30
   Thr Lys Phe Lys His Gln Leu Ser Leu Ala Phe Asn Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Gln Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 38
   <211> 163
   <212> PRT
   <213> Artificial
<220>
   <223> Variant cleaving RAG1
<400> 38
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Tyr Lys Phe Lys His Gln Leu Ser Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Gly Asp Asn Gly Ser Val Ser Asp Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 39
   <211> 163
   <212> PRT
   <213> Artificial
<220>
   <223> Variant cleaving RAG1
<400> 39
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Arg Gln Gln
   20 25 30
   Tyr Lys Phe Lys His Gln Leu Ser Leu Ala Phe Lys Val Thr Gln Lys
   35 40 45
   Thr Gln Arg Arg Trp Phe Leu Asp Lys Leu Val Asp Glu Ile Gly Val
   50 55 60
   Gly Tyr Val Thr Asp Ser Gly Ser Val Ser Asn Tyr Val Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 40
   <211> 163
   <212> PRT
   <213> Artificial
<220>
   <223> Variant cleaving RAG1
<400> 40
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Ala Pro Asn Gln Ser
   20 25 30
   Ser Lys Phe Lys His Arg Leu Lys Leu Ala Phe Asp Val Thr Gln Lys
   35 40 45
   Thr Gln Arg Arg Trp Phe Leu Asp Lys Leu Val Asp Glu Ile Gly Val
   50 55 60
   Gly Tyr Val Asn Asp Ser Gly Ser Val Ser Asn Tyr Ile Leu Ser Glu
   65 70 75 80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 41
   <211> 163
   <212> PRT
   <213> Artificial
<220>
   <223> Variant cleaving RAG1
<400> 41
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Thr
   20 25 30
   Cys Lys Phe Lys His Gln Leu Ser Leu Ala Phe Lys Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Gln Tyr Asn Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 42
   <211> 163
   <212> PRT
   <213> Artificial
<220>
   <223> Variant cleaving RAG1
<400> 42
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Arg Gln Ser
   20 25 30
   Cys Lys Phe Lys His Gln Leu Ser Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Lys Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 43
   <211> 163
   <212> PRT
   <213> Artificial
<220>
   <223> variant cleaving RAG1
<400> 43
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Lys Gln Ser
               20                  25                  30
   Ala Lys Phe Lys His Gln Leu Ser Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Tyr Tyr Lys Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 44
   <211> 163
   <212> PRT
   <213> Artificial
<220>
   <223> Variant cleaving RAG1
<400> 44
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Lys Gln Ser
               20                  25                  30
   Ala Lys Phe Lys His Gln Leu Ser Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Ser Asp Ser Gly Ser Val Ser Asp Tyr Arg Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 45
   <211> 163
   <212> PRT
   <213> Artificial
<220>
   <223> Variant cleaving RAG1
<400> 45
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Lys Gln Ser
   20 25 30
   Ala Lys Phe Lys His Gln Leu Ser Leu Ala Phe Arg Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Asp Tyr Thr Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 46
   <211> 163
   <212> PRT
   <213> Artificial
<220>
   <223> Variant cleaving RAG1
<400> 46
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Arg Gln Ser
   20 25 30
   Asn Lys Phe Lys His Gln Leu Ser Leu Ala Phe Thr Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Arg Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 47
   <211> 163
   <212> PRT
   <213> Artificial
<220>
   <223> Variant cleaving RAG1
<400> 47
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Arg Gln Ser
   20 25 30
   Asn Lys Phe Lys His Gln Leu Ser Leu Ala Phe Lys Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Asn Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 48
   <211> 163
   <212> PRT
   <213> Artificial
<220>
   <223> Variant cleaving RAG1
<400> 48
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Arg Lys Phe Lys His Thr Leu Ser Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Tyr Tyr Lys Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 49
   <211> 163
   <212> PRT
   <213> Artificial
<220>
   <223> Variant cleaving RAG1
<400> 49
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Lys Gln Ser
               20                  25                  30
   Gly Lys Phe Lys His Gln Leu Ser Leu Ala Phe Ala Val Thr Gln Lys
               35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Tyr Tyr Lys Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 50
   <211> 163
   <212> PRT
   <213> Artificial
<220>
   <223> Variant cleaving RAG1
<400> 50
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Gly Gln Ser
               20                  25                  30
   Tyr Lys Phe Lys His Gly Leu Ser Leu Ala Phe Arg Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Ser Gly Ser Val Ser Gln Tyr Asn Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 51
   <211> 163
   <212> PRT
   <213> Artificial
<220>
   <223> Variant cleaving RAG1
<400> 51
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asp Gln Ser
               20                  25                  30
   Arg Lys Phe Lys His Thr Leu Ser Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Asn Asp Ser Gly Ser Val Ser Tyr Tyr Arg Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro

<210> 52
   <211> 163
   <212> PRT
   <213> Artificial
<220>
   <223> Variant cleaving RAG1
<400> 52
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Gly Gln Ser
               20                  25                  30
   Tyr Lys Phe Lys His Gly Leu Ser Leu Ala Phe Gln Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Ser Gly Ser Val Ser Arg Tyr Tyr Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 53
   <211> 163
   <212> PRT
   <213> Artificial
<220>
   <223> Variant cleaving RAG1
<400> 53
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Ser Lys Phe Lys His Trp Leu Ser Leu Ala Phe Asn Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Ser Gly Ser Val Ser Arg Tyr Asn Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 54
   <211> 163
   <212> PRT
   <213> Artificial
<220>
   <223> Variant cleaving RAG1
<400> 54
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Thr
               20                  25                  30
   Thr Lys Phe Lys His Gln Leu Ser Leu Ala Phe Asn Val Thr Gln Lys
           35                  40                  45
   Thr Gln Arg Arg Trp Phe Leu Asp Lys Leu Val Asp Glu Ile Gly Val
   50 55 60
   Gly Tyr Val Arg Asp Ser Gly Ser Val Ser Arg Tyr Asn Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 55
   <211> 163
   <212> PRT
   <213> Artificial
<220>
   <223> Variant cleaving RAG1
<400> 55
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Arg Pro Asn Gln Ser
   20 25 30
   Ser Lys Phe Lys His Tyr Leu Gln Leu Ala Phe Thr Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Glu Tyr Arg Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 56
   <211> 163
   <212> PRT
   <213> Artificial
<220>
   <223> Variant cleaving RAG1
<400> 56
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Thr Lys Phe Lys His Ala Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Ala Asp Asn Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 57
   <211> 163
   <212> PRT
   <213> Artificial
<220>
   <223> Variant cleaving RAG1
<400> 57
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Gly Gln Ser
   20 25 30
   Tyr Lys Phe Lys His Gly Leu Ser Leu Ala Phe Glu Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp His Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 58
   <211> 163
   <212> PRT
   <213> Artificial
<220>
   <223> Variant cleaving RAG1
<400> 58
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Gly Gln Ser
   20 25 30
   Tyr Lys Phe Lys His Thr Leu Ser Leu Ala Phe Lys Val Thr Gln Lys
   35 40 45
   Thr Gln Arg Arg Trp Phe Leu Asp Lys Leu Val Asp Glu Ile Gly Val
   50 55 60
   Gly Tyr Val Asn Asp Ala Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 59
   <211> 163
   <212> PRT
   <213> Artificial
<220>
   <223> variant cleaving RAG1
<400> 59
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Ser Lys Phe Lys His Arg Leu Asp Leu Ala Phe Tyr Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Gln Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 60
   <211> 163
   <212> PRT
   <213> Artificial
<220>
   <223> Variant cleaving RAG1
<400> 60
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Ala Leu Ser Leu Ala Phe Asp Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Arg Tyr Gln Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 61
   <211> 163
   <212> PRT
   <213> Artificial
<220>
   <223> Variant cleaving RAG1
<400> 61
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Gln Pro Asn Gln Ser
   20 25 30
   Tyr Lys Phe Lys His Arg Leu Lys Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 62
   <211> 163
   <212> PRT
   <213> Artificial
<220>
   <223> Variant cleaving RAG1
<400> 62
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Glu
   20 25 30
   Tyr Lys Phe Lys His Gln Leu Ser Leu Ala Phe Tyr Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Asp Tyr Val Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 63
   <211> 163
   <212> PRT
   <213> Artificial
<220>
   <223> Variant cleaving RAG1
<400> 63
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Arg Pro Asn Gln Ser
   20 25 30
   Arg Lys Phe Lys His Tyr Leu Gln Leu Ala Phe Asn Val Thr Gln Lys
   35 40 45
   Thr Gln Arg Arg Trp Phe Leu Asp Lys Leu Val Asp Glu Ile Gly Val
   50 55 60
   Gly Tyr Val Thr Asp Ser Gly Ser Val Ser Arg Tyr Val Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 64
   <211> 163
   <212> PRT
   <213> Artificial
<220>
   <223> variant cleaving RAG1
<400> 64
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Cys Lys Phe Lys His Thr Leu Ser Leu Ala Phe Asn Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Arg Tyr Asn Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 65
   <211> 163
   <212> PRT
   <213> Artificial
<220>
   <223> Variant cleaving RAG1
<400> 65
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Gln Pro Asn Gln Ser
   20 25 30
   Tyr Lys Phe Lys His Arg Leu Lys Leu Ala Phe Asn Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Arg Tyr Asn Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 66
   <211> 163
   <212> PRT
   <213> Artificial
<220>
   <223> Variant cleaving RAG1
<400> 66
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Gly Gln Ser
   20 25 30
   Tyr Lys Phe Lys His His Leu Ser Leu Ala Phe Asn Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Arg Tyr Asp Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 67
   <211> 163
   <212> PRT
   <213> Artificial
<220>
   <223> Variant cleaving RAG1
<400> 67
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Arg Pro Asn Gln Ser
               20                  25                  30
   Ala Lys Phe Lys His Tyr Leu Gln Leu Ala Phe Thr Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Arg Tyr Val Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 68
   <211> 163
   <212> PRT
   <213> Artificial
<220>
   <223> Variant cleaving RAG1
<400> 68
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Asn Leu Gln Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Lys Tyr Glu Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 69
   <211> 163
   <212> PRT
   <213> Artificial
<220>
   <223> Variant cleaving RAG1
<400> 69
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Thr Lys Phe Lys His Gln Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 70
   <211> 163
   <212> PRT
   <213> Artificial
<220>
   <223> Variant cleaving RAG1
<400> 70
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asp Gln Ser
               20                  25                  30
   Arg Lys Phe Lys His Thr Leu Ser Leu Ala Phe Gln Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Arg Gly Ser Val Ser Asn Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 71
   <211> 163
   <212> PRT
   <213> Artificial
<220>
   <223> Variant cleaving RAG1
<400> 71
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Ala Pro Asn Gln Ser
               20                  25                  30
   Ser Lys Phe Lys His Arg Leu Lys Leu Ala Phe Asp Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Ser Tyr Arg Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 72
   <211> 163
   <212> PRT
   <213> Artificial
<220>
   <223> Variant cleaving RAG1
<400> 72
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Gln Pro Asn Gln Ser
               20                  25                  30
   Tyr Lys Phe Lys His Gln Leu Lys Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Asn Asp Ser Gly Ser Val Ser Tyr Tyr Arg Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 73
   <211> 163
   <212> PRT
   <213> Artificial
<220>
   <223> Variant cleaving RAG1
<400> 73
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asp Gln Ser
   20 25 30
   Arg Lys Phe Lys His Gln Leu Ser Leu Ala Phe Asn Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Arg Tyr Val Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 74
   <211> 163
   <212> PRT
   <213> Artificial
<220>
   <223> Variant cleaving RAG1
<400> 74
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Ser Lys Phe Lys His Arg Leu Glu Leu Ala Phe Tyr Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Gln Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 75
   <211> 163
   <212> PRT
   <213> Artificial
<220>
   <223> Variant cleaving RAG1
<400> 75
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Trp Lys Phe Lys His Gln Leu His Leu Ala Phe Tyr Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Ser Gly Ser Val Ser Asn Tyr Val Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 76
   <211> 163
   <212> PRT
   <213> Artificial
<220>
   <223> Variant cleaving RAG2
<400> 76
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Gln Pro Asn Gln Ser
               20                  25                  30
   Tyr Lys Phe Lys His Gln Leu Lys Leu Ala Phe Lys Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Glu Tyr Val Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 77
   <211> 163
   <212> PRT
   <213> Artificial

<220>
   <223> Variant cleaving RAG2
<400> 77
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Thr
               20                  25                  30
   His Lys Phe Lys His Gln Leu Ser Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Tyr Tyr Lys Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 78
   <211> 163
   <212> PRT
   <213> Artificial
<220>
   <223> Variant cleaving RAG2
<400> 78
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Gly Gln Ser
               20                  25                  30
   Tyr Lys Phe Lys His Gly Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 79
   <211> 163
   <212> PRT
   <213> Artificial
<220>
   <223> Variant cleaving RAG2
<400> 79
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Gly Gln Ser
               20                  25                  30
   Tyr Lys Phe Lys His Gly Leu Ser Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Ser Gly Ser Val Ser Gln Tyr Glu Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 80
   <211> 163
   <212> PRT
   <213> Artificial
<220>
   <223> Variant cleaving RAG2
<400> 80
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Ala Pro Asn Gln Ser
   20 25 30
   Ser Lys Phe Lys His Arg Leu Lys Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 81
   <211> 163
   <212> PRT
   <213> Artificial
<220>
   <223> Variant cleaving RAG2
<400> 81
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Ser Pro Asn Gln Ser
   20 25 30
   Tyr Lys Phe Lys His Arg Leu Lys Leu Ala Phe Tyr Val Thr Gln Lys
   35 40 45
   Thr Gln Arg Arg Trp Phe Leu Asp Lys Leu Val Asp Glu Ile Gly Val
   50 55 60
   Gly Tyr Val Asp Asp Ser Gly Ser Val Ser Arg Tyr Val Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 82
   <211> 163
   <212> PRT
   <213> Artificial
<220>
   <223> Variant cleaving RAG2
<400> 82
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Arg Lys Phe Lys His Gln Leu Gln Leu Ala Phe Asn Val Thr Gln Lys
           35                  40                  45
   Thr Gln Arg Arg Trp Phe Leu Asp Lys Leu Val Asp Glu Ile Gly Val
       50                  55                  60
   Gly Tyr Val Lys Asp His Gly Ser Val Ser Asp Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 83
   <211> 163
   <212> PRT
   <213> Artificial
<220>
   <223> Variant cleaving RAG2
<400> 83
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Gln Pro Asn Gln Ser
               20                  25                  30
   Ser Lys Phe Lys His Arg Leu Lys Leu Ala Phe Arg Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Asn Tyr Thr Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 84
   <211> 163
   <212> PRT
   <213> Artificial
<220>
   <223> Variant cleaving RAG2
<400> 84
<210> 85
   <211> 163
   <212> PRT
   <213> Artificial
<220>
   <223> Variant cleaving RAG2
<400> 85
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asp Gln Ser
               20                  25                  30
   Arg Lys Phe Lys His Thr Leu Ser Leu Ala Phe Glu Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Arg Gly Ser Val Ser Asp Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 86
   <211> 164
   <212> PRT
   <213> Artificial
<220>
   <223> Variant cleaving RAG2
<400> 86
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Cys Lys Phe Lys His Ala Leu Ser Leu Ala Phe Thr Val Thr Gln Lys
   35 40 45
   Thr Gln Arg Arg Trp Phe Leu Asp Lys Leu Val Asp Glu Ile Gly Val
   50 55 60
   Gly Tyr Val Arg Tyr Asp Ser Gly Ser Val Ser Arg Tyr Thr Leu Ser
   65 70 75 80
   Glu Ile Lys Pro Leu His Asn Phe Leu Thr Gln Leu Gln Pro Phe Leu
   85 90 95
   Lys Leu Lys Gln Lys Gln Ala Asn Leu Val Leu Lys Ile Ile Trp Arg
   100 105 110
   Leu Pro Ser Ala Lys Glu Ser Pro Asp Lys Phe Leu Glu Val Cys Thr
   115 120 125
   Trp Val Asp Gln Ile Ala Ala Leu Asn Asp Ser Lys Thr Arg Lys Thr
   130 135 140
   Thr Ser Glu Thr Val Arg Ala Val Leu Asp Ser Leu Ser Glu Lys Lys
   145 150 155 160
   Lys Ser Ser Pro
<210> 87
   <211> 163
   <212> PRT
   <213> Artificial
<220>
   <223> Variant cleaving RAG2
<400> 87
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Ser Pro Asn Gln Ser
               20                  25                  30
   Tyr Lys Phe Lys His Arg Leu Lys Leu Ala Phe Lys Val Thr Gln Lys
           35                  40                  45
   Thr Gln Arg Arg Trp Phe Leu Asp Lys Leu Val Asp Glu Ile Gly Val
       50                  55                  60
   Gly Tyr Val Thr Asp Gly Gly Ser Val Ser Asp Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 88
   <211> 163
   <212> PRT
   <213> Artificial
<220>
   <223> Variant cleaving RAG2
<400> 88
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Ser Pro Asn Gln Ser
               20                  25                  30
   Tyr Lys Phe Lys His Arg Leu Lys Leu Ala Phe Ala Val Thr Gln Lys
           35                  40                  45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Lys Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 89
   <211> 163
   <212> PRT
   <213> Artificial
<220>
   <223> Variant cleaving RAG2
<400> 89
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Gly Gln Ser
   20 25 30
   Tyr Lys Phe Lys His Gly Leu Ser Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Lys Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 90
   <211> 163
   <212> PRT
   <213> Artificial
<220>
   <223> Variant cleaving RAG2
<400> 90
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Ser Gln Ser
   20 25 30
   Tyr Lys Phe Lys His Arg Leu His Leu Ala Phe Asn Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Gln Tyr Gln Leu Ser Glu
   65 70 75 80
   Ile Arg Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 91
   <211> 163
   <212> PRT
   <213> Artificial
<220>
   <223> Variant cleaving RAG2
<400> 91
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Cys Lys Phe Lys His Gln Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 92
   <211> 163
   <212> PRT
   <213> Artificial
<220>
   <223> Variant cleaving RAG2
<400> 92
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Arg Pro Asn Gln Ser
               20                  25                  30
   Ala Lys Phe Lys His Tyr Leu Gln Leu Ala Phe Gln Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Arg Tyr Gln Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 93
   <211> 163
   <212> PRT
   <213> Artificial
<220>
   <223> Variant cleaving RAG2
<400> 93
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Gly Gln Ser
               20                  25                  30
   Tyr Lys Phe Lys His Gly Leu Ser Leu Ala Phe Ala Val Thr Gln Lys
           35                  40                  45
   Thr Gln Arg Arg Trp Phe Leu Asp Lys Leu Val Asp Glu Ile Gly Val
   50 55 60
   Gly Tyr Val Arg Asp Ser Gly Ser Val Ser Arg Tyr Tyr Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 94
   <211> 163
   <212> PRT
   <213> Artificial
<220>
   <223> Variant cleaving RAG2
<400> 94
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Asn Leu Gln Leu Ala Phe Asp Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Arg Tyr Gln Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 95
   <211> 163
   <212> PRT
   <213> Artificial
<220>
   <223> Variant cleaving RAG2
<400> 95
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Ser Pro Asn Gln Ser
   20 25 30
   Tyr Lys Phe Lys His Arg Leu Lys Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Arg Tyr Gln Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 96
   <211> 163
   <212> PRT
   <213> Artificial
<220>
   <223> Variant cleaving RAG2
<400> 96
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Ser Lys Phe Lys His Arg Leu Glu Leu Ala Phe Gln Val Thr Gln Lys
           35                  40                  45
   Thr Gln Arg Arg Trp Phe Leu Asp Lys Leu Val Asp Glu Ile Gly Val
       50                  55                  60
   Gly Tyr Val Asn Asp Ser Gly Ser Val Ser Asn Tyr Arg Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 97
   <211> 163
   <212> PRT
   <213> Artificial
<220>
   <223> Variant cleaving RAG2
<400> 97
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Gln
   20 25 30
   Arg Lys Phe Lys His Gln Leu Ser Leu Ala Phe Lys Val Thr Gln Lys
   35 40 45
   Thr Gln Arg Arg Trp Phe Leu Asp Lys Leu Val Asp Glu Ile Gly Val
   50 55 60
   Gly Tyr Val Thr Asp Gly Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 98
   <211> 163
   <212> PRT
   <213> Artificial
<220>
   <223> Variant cleaving RAG2
<400> 98
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Asn Leu Gln Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 99
   <211> 163
   <212> PRT
   <213> Artificial
<220>
   <223> Variant cleaving RAG2
<400> 99
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Asp
   20 25 30
   His Lys Phe Lys His Gln Leu Ser Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Glu Tyr Arg Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 100
   <211> 163
   <212> PRT
   <213> Artificial
<220>
   <223> Variant cleaving RAG2
<400> 100
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Glu
   20 25 30
   Tyr Lys Phe Lys His Gln Leu Ser Leu Ala Phe Thr Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Val Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 101
   <211> 163
   <212> PRT
   <213> Artificial
<220>
   <223> Variant cleaving RAG2
<400> 101
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asp Gln Ser
   20 25 30
   Arg Lys Phe Lys His Thr Leu Ser Leu Ala Phe Lys Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Glu Tyr Val Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 102
   <211> 163
   <212> PRT
   <213> Artificial
<220>
   <223> Variant cleaving RAG2
<400> 102
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Arg Gln Asp
               20                  25                  30
   Tyr Lys Phe Lys His Gln Leu Ser Leu Ala Phe Arg Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Ser Gly Ser Val Ser Gln Tyr Asn Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro

<210> 103
   <211> 163
   <212> PRT
   <213> Artificial
<220>
   <223> Variant cleaving RAG2
<400> 103
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Cys Lys Phe Lys His Ala Leu Ser Leu Ala Phe Arg Val Thr Gln Lys
           35                  40                  45
   Thr Gln Arg Arg Trp Phe Leu Asp Lys Leu Val Asp Glu Ile Gly Val
       50                  55                  60
   Gly Tyr Val Asn Asp Ser Gly Ser Val Ser Asn Tyr Asn Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 104
   <211> 163
   <212> PRT
   <213> Artificial
<220>
   <223> Variant cleaving RAG2
<400> 104
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asp Gln Ser
   20 25 30
   Arg Lys Phe Lys His Thr Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Ala Asp Ser Gly Ser Val Ser Asp Tyr Arg Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 105
   <211> 163
   <212> PRT
   <213> Artificial
<220>
   <223> Variant cleaving RAG2
<400> 105
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Cys Lys Phe Lys His Ala Leu Ser Leu Ala Phe Asn Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Gln Tyr Gln Leu Ser Glu
   65 70 75 80
   Ile Arg Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 106
   <211> 163
   <212> PRT
   <213> Artificial
<220>
   <223> Variant cleaving RAG2
<400> 106
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Ala Leu Gln Leu Ala Phe Tyr Val Thr Gln Lys
   35 40 45
   Thr Gln Arg Arg Trp Phe Leu Asp Lys Leu Val Asp Glu Ile Gly Val
   50 55 60
   Gly Tyr Val Asp Asp Ser Gly Ser Val Ser Arg Tyr Val Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 107
   <211> 163
   <212> PRT
   <213> Artificial
<220>
   <223> Variant cleaving RAG2
<400> 107
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Gly Gln Ser
   20 25 30
   Tyr Lys Phe Lys His His Leu Ser Leu Ala Phe Lys Val Thr Gln Lys
   35 40 45
   Thr Gln Arg Arg Trp Phe Leu Asp Lys Leu Val Asp Glu Ile Gly Val
   50 55 60
   Gly Tyr Val His Asp Glu Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 108
   <211> 163
   <212> PRT
   <213> Artificial
<220>
   <223> Variant cleaving RAG2
<400> 108
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Tyr Lys Phe Lys His Gln Leu Ser Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Lys Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 109
   <211> 163
   <212> PRT
   <213> Artificial
<220>
   <223> Variant cleaving RAG2
<400> 109
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Ala Pro Asn Gln Ser
   20 25 30
   Ser Lys Phe Lys His Arg Leu Lys Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Tyr Tyr Arg Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 110
   <211> 163
   <212> PRT
   <213> Artificial
<220>
   <223> Variant cleaving RAG2
<400> 110
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Arg Lys Phe Lys His Gln Leu Gln Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly His Val Arg Asp Ala Gly Ser Val Ser Asn Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 111
   <211> 163
   <212> PRT
   <213> Artificial
<220>
   <223> Variant cleaving RAG2
<400> 111
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Arg Lys Phe Lys His Gln Leu Gln Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Ala Gly Ser Val Ser Asn Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Arg Gln Ala Asn Leu Val Leu Lys Ile Ile Trp Arg Leu
               100                 105                 110
   Pro Ser Ala Lys Glu Ser Pro Asp Lys Phe Leu Glu Val Arg Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 112
   <211> 163
   <212> PRT
   <213> Artificial
<220>
   <223> Variant cleaving RAG2
<400> 112
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Arg Lys Phe Lys His Gln Leu Gln Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ala Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Pro Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 113
   <211> 163
   <212> PRT
   <213> Artificial
<220>
   <223> Variant cleaving RAG2
<400> 113
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Gln Leu Gln Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ala Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Thr Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Pro Ser Pro
<210> 114
   <211> 163
   <212> PRT
   <213> Artificial
<220>
   <223> Variant cleaving RAG2
<400> 114
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Arg Lys Phe Lys His Gln Leu Gln Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Ala Gly Ser Val Ser Asn Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Ala Leu Asp Ser Leu Ser Glu Lys Lys Lys
   145                 150                 155                 160
   Ala Ser Pro
<210> 115
   <211> 163
   <212> PRT,
   <213> Artificial
<220>
   <223> Variant cleaving RAG2
<400> 115
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Gln Leu Gln Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ala Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Asn Leu Ser Glu Lys Lys Lys
   145 150 155 160
   Ser Ser Pro
<210> 116
   <211> 163
   <212> PRT
   <213> Artificial
<220>
   <223> Variant cleaving RAG2
<400> 116
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Gln Leu Gln Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ala Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Arg
   145 150 155 160
   Ser Ser Pro
<210> 117
   <211> 163
   <212> PRT
   <213> Artificial
<220>
   <223> Variant cleaving RAG2
<400> 117
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Gln Leu Gln Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ala Gly Ser Val Ser Asn Tyr Ile Leu Ser Glu
   65 70 75 80
   Ile Lys Pro Leu Arg Asn Phe Leu Thr Gln Leu Gln Pro Leu Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Ala Asp Gln Ile Ala Ala Leu Asn Asp Ser Lys Thr Arg Lys Thr Thr
   130 135 140
   Ser Glu Thr Val Arg Ala Val Leu Gly Ser Leu Ser Glu Lys Arg Arg
   145 150 155 160
   Ser Ser Pro
<210> 118
   <211> 163
   <212> PRT
   <213> Artificial
<220>
   <223> Variant cleaving RAG2
<400> 118
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Gln Leu Gln Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ala Gly Ser Val Ser Asn Tyr Ile Leu Ser Glu
   65 70 75 80
   Ile Lys Pro Leu His Asp Phe Leu Thr Gln Leu Gln Pro Phe Leu Glu
   85 90 95
   Leu Lys Gln Lys Gln Ala Asp Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Ala Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 119
   <211> 163
   <212> PRT
   <213> Artificial
<220>
   <223> Variant cleaving RAG2
<400> 119
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Gln Leu Gln Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp His Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asp Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 120
   <211> 163
   <212> PRT
   <213> Artificial
<220>
   <223> Variant cleaving RAG2
<400> 120
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Arg Lys Phe Lys His Gln Leu Gln Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp His Gly Ser Val Ser Asn Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Pro Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 121
   <211> 163
   <212> PRT
   <213> Artificial
<220>
   <223> Variant cleaving RAG2
<400> 121
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Gln Leu Gln Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp His Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Gly Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Pro Ser Pro
<210> 122
   <211> 163
   <212> PRT
   <213> Artificial
<220>
   <223> Variant cleaving RAG2
<400> 122
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Gln Leu Gln Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp His Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Ala Val Arg Ala Val Leu Asp Ser Leu Ser Glu Lys Lys Arg
   145 150 155 160
   Ser Ser Pro
<210> 123
   <211> 163
   <212> PRT
   <213> Artificial
<220>
   <223> Variant cleaving RAG2
<400> 123
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Gln Leu Gln Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp His Gly Ser Val Ser Asn Tyr Ile Leu Ser Glu
   65 70 75 80
   Ile Lys Pro Leu His Asn Leu Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Thr Ala Ala Leu Asn Asp Ser Lys Thr Arg Lys Thr Thr
   130 135 140
   Ser Glu Thr Val Arg Ala Ala Leu Asp Ser Leu Ser Glu Lys Lys Lys
   145 150 155 160
   Ser Ser Pro
<210> 124
   <211> 163
   <212> PRT
   <213> Artificial
<220>
   <223> Variant cleaving RAG2
<400> 124 Ser Ser Pro
<210> 125
   <211> 163
   <212> PRT
   <213> Artificial
<220>
   <223> Variant cleaving RAG2
<400> 125
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Gln Leu Gln Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Asn Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Pro Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Pro Ser Glu Lys Lys Lys
   145 150 155 160
   Ser Ser Pro
<210> 126
   <211> 163
   <212> PRT
   <213> Artificial
<220>
   <223> variant cleaving RAG2
<400> 126
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Arg Lys Phe Lys His Gln Leu Gln Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Asn Gly Ser Val Ser Asn Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Ala Leu Asp Ser Leu Ser Glu Lys Arg Lys
   145                 150                 155                 160
   Ser Ser Pro
<210> 127
   <211> 163
   <212> PRT
   <213> Artificial
<220>
   <223> Variant cleaving RAG2
<400> 127
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Arg Lys Phe Lys His Gln Leu Gln Leu Ala Phe Ala Val Thr Gln Lys
           35                  40                  45
   Thr Gln Arg Arg Trp Phe Leu Asp Lys Leu Val Asp Glu Ile Gly Val
   50 55 60
   Gly Tyr Val Arg Asp Asn Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Arg
   145 150 155 160
   Ser Ser Pro

<210> 128
   <211> 163
   <212> PRT
   <213> Artificial
<220>
   <223> variant cleaving RAG2
<400> 128
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Gln Leu Gln Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Pro Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 129
   <211> 163
   <212> PRT
   <213> Artificial
<220>
   <223> Variant cleaving RAG2
<400> 129
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Gln Leu Gln Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ala Gly Ser Val Ser Asn Tyr Ile Leu Ser Glu
   65 70 75 80
   Ile Lys Pro Leu His Asp Phe Leu Thr Gln Leu Gln Pro Phe Leu Glu
   85 90 95
   Leu Lys Gln Lys Gln Ala Asp Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Ala Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 130
   <211> 163
   <212> PRT
   <213> Artificial
<220>
   <223> variant cleaving RAG2
<400> 130
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Cys Lys Phe Lys His Ala Leu Ser Leu Ala Phe Asp Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Ser Tyr Arg Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 131
   <211> 163
   <212> PRT
   <213> Artificial
<220>
   <223> Variant cleaving RAG2
<400> 131
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Tyr Lys Phe Lys His Gln Leu Ser Leu Ala Phe Asn Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Gln Tyr Gln Leu Ser Glu
   65 70 75 80
   Ile Arg Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 132
   <211> 163
   <212> PRT
   <213> Artificial
<220>
   <223> Variant cleaving RAG2
<400> 132
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Arg Pro Asn Gln Ser
   20 25 30
   Ala Lys Phe Lys His Tyr Leu Gln Leu Ala Phe Asn Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Tyr Tyr Asn Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 133
   <211> 163
   <212> PRT
   <213> Artificial
<220>
   <223> variant cleaving RAG2
<400> 133
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Gln
   20 25 30
   Arg Lys Phe Lys His Gln Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Ala Asp Ser Gly Ser Val Ser Asp Tyr Arg Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 134
   <211> 163
   <212> PRT
   <213> Artificial
<220>
   <223> Variant cleaving RAG2
<400> 134
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Asn Lys Phe Lys His Gln Leu Ala Leu Ala Phe Asn Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Tyr Tyr Asn Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 135
   <211> 163
   <212> PRT
   <213> Artificial
<220>
   <223> Variant cleaving RAG2
<400> 135
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Gly Gln Ser
               20                  25                  30
   Tyr Lys Phe Lys His His Leu Ser Leu Ala Phe Asn Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Tyr Tyr Gln Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 136
   <211> 163
   <212> PRT
   <213> Artificial
<220>
   <223> Variant cleaving RAG2
<400> 136
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Gly Gln Ser
               20                  25                  30
   Tyr Lys Phe Lys His His Leu Ser Leu Ala Phe Arg Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Glu Tyr Val Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 137
   <211> 163
   <212> PRT
   <213> Artificial
<220>
   <223> Variant cleaving RAG2
<400> 137
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Gln Pro Asn Gln Ser
               20                  25                  30
   Tyr Lys Phe Lys His Arg Leu Lys Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Tyr Tyr Lys Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 138
   <211> 163
   <212> PRT
   <213> Artificial
<220>
   <223> Variant cleaving RAG2
<400> 138
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Arg Gln Gly
               20                  25                  30
   Tyr Lys Phe Lys His Gln Leu Ser Leu Ala Phe Gln Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Ser Gly Ser Val Ser Arg Tyr Thr Leu Ser Lys
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 139
   <211> 163
   <212> PRT
   <213> Artificial
<220>
   <223> Variant cleaving RAG2
<400> 139
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Gly Gln Ser
               20                  25                  30
   Tyr Lys Phe Lys His His Leu Ser Leu Ala Phe Arg Val Thr Gln Lys
           35                  40                  45
   Thr Gln Arg Arg Trp Phe Leu Asp Lys Leu Val Asp Glu Ile Gly Val
       50                  55                  60
   Gly Tyr Val Asn Asp Ser Gly Ser Val Ser Asn Tyr Asn Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 140
   <211> 163
   <212> PRT
   <213> Artificial
<220>
   <223> variant cleaving RAG2
<400> 140
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Thr
   20 25 30
   His Lys Phe Lys His Gln Leu Ser Leu Ala Phe Glu Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 141
   <211> 163
   <212> PRT
   <213> Artificial
<220>
   <223> Variant cleaving RAG2
<400> 141
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Tyr Lys Phe Lys His Gln Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Ser Gly Ser Val Ser Lys Tyr Val Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 142
   <211> 163
   <212> PRT
   <213> Artificial
<220>
   <223> Variant cleaving RAG2
<400> 142
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   His Lys Phe Lys His Ala Leu Ser Leu Ala Phe Lys Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Gln Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 143
   <211> 163
   <212> PRT
   <213> Artificial
<220>
   <223> Variant cleaving RAG2
<400> 143
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Gly Gln Ser
   20 25 30
   Tyr Lys Phe Lys His Gly Leu Ser Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Lys Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 144
   <211> 163
   <212> PRT
   <213> Artificial
<220>
   <223> Variant cleaving RAG2
<400> 144
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Arg Lys Phe Lys His Ala Leu Gln Leu Ala Phe Tyr Val Thr Gln Lys
           35                  40                  45
   Thr Gln Arg Arg Trp Phe Leu Asp Lys Leu Val Asp Glu Ile Gly Val
       50                  55                  60
   Gly Tyr Val Ser Asp Ser Gly Ser Val Ser Ser Tyr Asp Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 145
   <211> 163
   <212> PRT
   <213> Artificial
<220>
   <223> Variant cleaving RAG2
<400> 145
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Tyr Lys Phe Lys His Gln Leu Ser Leu Ala Phe Lys Val Thr Gln Lys
           35                  40                  45
   Thr Gln Arg Arg Trp Phe Leu Asp Lys Leu Val Asp Glu Ile Gly Val
       50                  55                  60
   Gly Tyr Val His Asp Glu Gly Ser Val Ser Asp Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 146
   <211> 163
   <212> PRT
   <213> Artificial
<220>
   <223> Variant cleaving RAG2
<400> 146
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Ser Lys Phe Lys His Arg Leu His Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Arg Tyr Gln Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 147
   <211> 163
   <212> PRT
   <213> Artificial
<220>
   <223> variant cleaving RAG2
<400> 147
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Ser Lys Phe Lys His Arg Leu Glu Leu Ala Phe Gln Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Arg Tyr Gln Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 148
   <211> 22
   <212> DNA
   <213> Artificial
<220>
   <223> Target RAG1
<400> 148
   cagcctgctg agcaaggtaa ca 22
<210> 149
   <211> 22
   <212> DNA
   <213> Artificial
<220>
   <223> Target RAG1
<400> 149
   ttgaataatt caaatgatac aa 22
<210> 150
   <211> 22
   <212> DNA
   <213> Artificial
<220>
   <223> Target RAG1
<400> 150
   tctgaaccat aagtagtttt ag 22
<210> 151
   <211> 22
   <212> DNA
   <213> Artificial
<220>
   <223> Target RAG1
<400> 151
   tgttctcagg tacctcagcc ag 22
<210> 152
   <211> 22
   <212> DNA
   <213> Artificial
<220>
   <223> Target RAG1
<400> 152
   cccaccttgg gactcagttc tg 22
<210> 153
   <211> 22
   <212> DNA
   <213> Artificial
<220>
   <223> Target RAG1
<400> 153
   ctggacaagg ctgatggtca ga 22
<210> 154
   <211> 22
   <212> DNA
   <213> Artificial
<220>
   <223> Target RAG1
<400> 154
   cgatccaccc cactgagttc tg 22
<210> 155
   <211> 22
   <212> DNA
   <213> Artificial
<220>
   <223> Target RAG1
<400> 155
   caagcccgtc agcgcaagag aa 22
<210> 156
   <211> 22
   <212> DNA
   <213> Artificial
<220>
   <223> Target RAG1
<400> 156
   cttcccagag cactttgtga aa 22
<210> 157
   <211> 22
   <212> DNA
   <213> Artificial
<220>
   <223> Target RAG1
<400> 157
   cattctggct gaccctgtgg ag 22
<210> 158
   <211> 22
   <212> DNA
   <213> Artificial
<220>
   <223> Target RAG1
<400> 158
   cctactgacc tggagagtcc ag 22
<210> 159
   <211> 22
   <212> DNA
   <213> Artificial
<220>
   <223> Target RAG1
<400> 159
   tgaaatgtcc agcaaaagag tg 22
<210> 160
   <211> 22
   <212> DNA
   <213> Artificial
<220>
   <223> Target RAG1
<400> 160
   ctggctctga gggcgaggaa tg 22
<210> 161
   <211> 22
   <212> DNA
   <213> Artificial
<220>
   <223> Target RAG1
<400> 161
   tgagctggag gccatcatgc ag 22
<210> 162
   <211> 22
   <212> DNA
   <213> Artificial
<220>
   <223> Target RAG1
<400> 162
   caggactgtg aaagccatca ca 22
<210> 163
   <211> 22
   <212> DNA
   <213> Artificial
<220>
   <223> Target RAG1
<400> 163
   ttgcatgccc ttcggaatgc tg 22
<210> 164
   <211> 22
   <212> DNA
   <213> Artificial
<220>
   <223> Target RAG1
<400> 164
   ttacccagtg gacaccattg ca 22
<210> 165
   <211> 22
   <212> DNA
   <213> Artificial
<220>
   <223> Target RAG1
<400> 165
   tggccccttc actgtggtgg tg 22
<210> 166
   <211> 22
   <212> DNA
   <213> Artificial
<220>
   <223> Target RAG1
<400> 166
   tggaatggga gacgtgagtg ag 22
<210> 167
   <211> 22
   <212> DNA
   <213> Artificial
<220>
   <223> Target RAG1
<400> 167
   caagccattg tgccttatgc tg 22
<210> 168
   <211> 22
   <212> DNA
   <213> Artificial
<220>
   <223> Target RAG1
<400> 168
   cgagacgctg actgccatcc tg 22

<210> 169
   <211> 22
   <212> DNA
   <213> Artificial
<220>
   <223> Target RAG1
<400> 169
   tcctctcatt gctgagaggg ag 22
<210> 170
   <211> 22
   <212> DNA
   <213> Artificial
<220>
   <223> Target RAG1
<400> 170
   cgttatgagg tctggcgttc ca 22
<210> 171
   <211> 22
   <212> DNA
   <213> Artificial
<220>
   <223> Target RAG1
<400> 171
   ttctacaaga tcttccagct ag 22
<210> 172
   <211> 22
   <212> DNA
   <213> Artificial
<220>
   <223> Target RAG1
<400> 172
   ctggacaagc atctccggaa ga 22
<210> 173
   <211> 22
   <212> DNA
   <213> Artificial
<220>
   <223> Target RAG1
<400> 173
   cagaatccct ctgccagtac ag 22
<210> 174
   <211> 22
   <212> DNA
   <213> Artificial
<220>
   <223> Target RAG1
<400> 174
   cagtccaaat gctatgagat gg 22
<210> 175
   <211> 20
   <212> DNA
   <213> Artificial
<220>
   <223> Target RAG1
<400> 175
   caaatgctat gagatggaag 20
<210> 176
   <211> 22
   <212> DNA
   <213> Artificial
<220>
   <223> Target RAG1
<400> 176
   tttaccatga accctcaggc aa 22
<210> 177
   <211> 22
   <212> DNA
   <213> Artificial
<220>
   <223> Target RAG1
<400> 177
   caagcttagg ggacccatta gg 22
<210> 178
   <211> 22
   <212> DNA
   <213> Artificial
<220>
   <223> Target RAG2
<400> 178
   taatacctgg tttagcggca aa 22
<210> 179
   <211> 22
   <212> DNA
   <213> Artificial
<220>
   <223> Target RAG2
<400> 179
   tggcctaaga caggaaggaa ga 22
<210> 180
   <211> 22
   <212> DNA
   <213> Artificial
<220>
   <223> Target RAG2
<400> 180
   tactctggag caatcaagaa aa 22
<210> 181
   <211> 22
   <212> DNA
   <213> Artificial
<220>
   <223> Target RAG2
<400> 181
   tactatgagt cctttcatta ta 22
<210> 182
   <211> 22
   <212> DNA
   <213> Artificial
<220>
   <223> Target RAG2
<400> 182
   ttgtatatat ttattggtcc ta 22
<210> 183
   <211> 22
   <212> DNA
   <213> Artificial
<220>
   <223> Target RAG2
<400> 183
   tcagctgaag aacaggatct ta 22
<210> 184
   <211> 22
   <212> DNA
   <213> Artificial
<220>
   <223> Target RAG2
<400> 184
   tgaaactatg gaagagatac aa 22
<210> 185
   <211> 22
   <212> DNA
   <213> Artificial
<220>
   <223> Target RAG2
<400> 185
   ccttatgtct tgcccaagaa aa 22
<210> 186
   <211> 22
   <212> DNA
   <213> Artificial
<220>
   <223> Target RAG2
<400> 186
   cttgccttgt atctcaataa ca 22
<210> 187
   <211> 22
   <212> DNA
   <213> Artificial
<220>
   <223> Target RAG2
<400> 187
   tcagatgcct tccctcatgt ag 22
<210> 188
   <211> 22
   <212> DNA
   <213> Artificial
<220>
   <223> Target RAG2
<400> 188
   ccagataatt gttgaaagat ca 22
<210> 189
   <211> 22
   <212> DNA
   <213> Artificial
<220>
   <223> Target RAG2
<400> 189
   tactaccagc accctcatca ta 22
<210> 190
   <211> 22
   <212> DNA
   <213> Artificial
<220>
   <223> Target RAG2
<400> 190
   ctgacctgat tcccatatcc ta 22
<210> 191
   <211> 22
   <212> DNA
   <213> Artificial
<220>
   <223> Target RAG2
<400> 191
   ctatatgcaa atccctgttc ta 22
<210> 192
   <211> 22
   <212> DNA
   <213> Artificial
<220>
   <223> Target RAG2
<400> 192
   ttacccaaaa gttcttggac tg 22
<210> 193
   <211> 22
   <212> DNA
   <213> Artificial
<220>
   <223> Target RAG2
<400> 193
   cactccaaca gaagcagttg tg 22
<210> 194
   <211> 22
   <212> DNA
   <213> Artificial
<220>
   <223> Target RAG2
<400> 194
   tggaatggca gtaaaggttc tg 22
<210> 195
   <211> 22
   <212> DNA
   <213> Artificial
<220>
   <223> Target RAG2
<400> 195
   tcagacaaaa atctacgtac ca 22
<210> 196
   <211> 22
   <212> DNA
   <213> Artificial
<220>
   <223> Target RAG2
<400> 196
   ttgcacattc aaaggcagct tg 22
<210> 197
   <211> 22
   <212> DNA
   <213> Artificial
<220>
   <223> Target RAG2
<400> 197
   tgatcttccc ctgggtagcc ca 22
<210> 198
   <211> 22
   <212> DNA
   <213> Artificial
<220>
   <223> Target RAG2
<400> 198
   tggaactgtt tttcttggca ta 22
<210> 199
   <211> 22
   <212> DNA
   <213> Artificial
<220>
   <223> Target RAG2
<400> 199
   tgagacaggc tactggatta ca 22
<210> 200
   <211> 22
   <212> DNA
   <213> Artificial
<220>
   <223> Target RAG2
<400> 200
   taaaatcata acattgattt ta 22
<210> 201
   <211> 22
   <212> DNA
   <213> Artificial
<220>
   <223> Target RAG2
<400> 201
   tctgatctga ttttttattc aa 22
<210> 202
   <211> 22
   <212> DNA
   <213> Artificial
<220>
   <223> Target RAG2
<400> 202
   ttaaattgat tattttgtgc aa 22
<210> 203
   <211> 501
   <212> DNA
   <213> Artificial
<220>
   <223> cDNA I-CreI N75 scaffold
<400> 203
   atggccaata ccaaatataa caaagagttc ctgctgtacc tggccggctt tgtggacggt 60
   gacggtagca tcatcgctca gattaaacca aaccagtctt ataagtttaa acatcagcta 120
   agcttgacct ttcaggtgac tcaaaagacc cagcgccgtt ggtttctgga caaactagtg 180
   gatgaaattg gcgttggtta cgtacgtgat cgcggatccg tttccaacta catcttaagc 240
   gaaatcaagc cgctgcacaa cttcctgact caactgcagc cgtttctgaa actgaaacag 300
   aaacaggcaa acctggttct gaaaattatc gaacagctgc cgtctgcaaa agaatccccg 360
   gacaaattcc tggaagtttg tacctgggtg gatcagattg cagctctgaa cgattctaag 420
   acgcgtaaaa ccacttctga aaccgttcgt gctgtgctgg acagcctgag cgagaagaag 480
   aaatcctccc cggcggccga c 501
<210> 204
   <211> 68
   <212> DNA
   <213> Artificial
<220>
   <223> Ulib4rev
<400> 204
   ctgaaaggtc aagcttagmb batgtttaaa cttmbbagac tgmbbtggtt taatctgagc 60
   gatgatgc 68
<210> 205
   <211> 68
   <212> DNA
   <213> Artificial
<220>
   <223> Ulib5rev
<400> 205
   ctgaaaggtc aagcttagmb batgtttaaa cttataagac tgmbbtggmb baatctgagc 60
   gatgatgc 68
<210> 206
   <211> 18
   <212> DNA
   <213> Artificial
<220>
   <223> Ulib456for
<400> 206
   ctaagcttga cctttcag 18
<210> 207
   <211> 24
   <212> DNA
   <213> Artificial
<220>
   <223> Target
<400> 207
   tcgggacgtc gtacgacgtc ccga 24
<210> 208
   <211> 24
   <212> DNA
   <213> Artificial
<220>
   <223> Derivative of target RAG1.10
<400> 208
   tcgttacgtc gtacgacgta acga 24
<210> 209
   <211> 24
   <212> DNA
   <213> Artificial
<220>
   <223> Derivative of target RAG1.10
<400> 209
   tctggacgtc gtacgacgtc caga 24
<210> 210
   <211> 24
   <212> DNA
   <213> Artificial
<220>
   <223> Derivative of target RAG1.10
<400> 210
   tcaaaaccag gtacctggtt ttga 24
<210> 211
   <211> 24
   <212> DNA
   <213> Artificial
<220>
   <223> Derivative of target RAG1.10
<400> 211
   tcaaaacgag gtacctcgtt ttga 24
<210> 212
   <211> 22
   <212> DNA
   <213> Artificial
<220>
   <223> Target RAG1.10.2
<400> 212
   tgttctcagg tacctgagaa cg 22
<210> 213
   <211> 22
   <212> DNA
   <213> Artificial
<220>
   <223> Target RAG1.10.3
<400> 213
   ttggctgagg tacctcagcc ag 22
<210> 214
   <211> 24
   <212> DNA
   <213> Artificial
<220>
   <223> Oligo
<400> 214
   tcgaaacgtc gtacgacgtt tcga 24
<210> 215
   <211> 24
   <212> DNA
   <213> Artificial
<220>
   <223> Oligo
<400> 215
   tctgtacgtc gtacgacgta caga 24
<210> 216
   <211> 24
   <212> DNA
   <213> Artificial
<220>
   <223> Oligo
<400> 216
   tcaaaactat gtacatagtt ttga 24
<210> 217
   <211> 24
   <212> DNA
   <213> Artificial
<220>
   <223> Oligo
<400> 217
   tcaaaacctc gtacgaggtt ttga 24
<210> 218
   <211> 22
   <212> DNA
   <213> Artificial
<220>
   <223> Target RAG2.8.2
<400> 218
   tgaaactatg tacgagatac aa 22
<210> 219
   <211> 22
   <212> DNA
   <213> Artificial
<220>
   <223> Target RAG2.8.3
<400> 219
   tgaaactatg tacatagttt ca 22
<210> 220
   <211> 22
   <212> DNA
   <213> Artificial
<220>
   <223> Target RAG2.8.4
<400> 220
   ttgtatctcg tacgagatac aa 22
<210> 221
   <211> 24
   <212> DNA
   <213> Artificial
<220>
   <223> I-CreI natural target
<400> 221
   tcaaaacgtc gtgagacagt ttgg 24
<210> 222
   <211> 49
   <212> DNA
   <213> Artificial
<220>
   <223> Target oligo
<220>
   <221> misc_feature
   <222> (15)..(17) g, or t
   <223> n is a, c, g, or t
<220>
   <221> misc_feature
   <222> (32)..(34)
   <223> n is a, c, g, or t
<400> 222
   ggcatacaag tttcnnnacg tcgtacgacg tnnngacaat cgtctgtca 49
<210> 223
   <211> 26
   <212> DNA
   <213> Artificial
<220>
   <223> Gal-10F
<400> 223
   gcaactttag tgctgacaca tacagg 26
<210> 224
   <211> 24
   <212> DNA
   <213> Artificial
<220>
   <223> Gal-10R
<400> 224
   acaaccttga ttgcagactt gacc 24
<210> 225
   <211> 50
   <212> DNA
   <213> Artificial
<220>
   <223> Oligo
<400> 225
   tggcatacaa gttttgttct caggtacctg agaacaacaa tcgtctgtca 50
<210> 226
   <211> 15
   <212> DNA
   <213> Artificial
<220>
   <223> Oligo
<220>
   <221> misc_feature
   <222> (4)..(6)
   <223> n is a, c, g, or t
<400> 226
   ctannnttga ccttt 15
<210> 227
   <211> 15
   <212> DNA
   <213> Artificial
<220>
   <223> Oligo
<220>
   <221> misc_feature
   <222> (10)..(12)
   <223> n is a, c, g, or t
<400> 227
   aaaggtcaan nntag 15
<210> 228
   <211> 59
   <212> DNA
   <213> Artificial
<220>
   <223> oligo
<400> 228
   gcataaatta ctatacttct atagacacgc aaacacaaat acacagcggc cttgccacc 59
<210> 229
   <211> 48
   <212> DNA
   <213> Artificial
<220>
   <223> Oligo
<400> 229
   ggctcgagga gctcgtctag aggatcgctc gagttatcag tcggccgc 48
<210> 230
   <211> 33
   <212> DNA
   <213> Artificial sequence
<220>
   <223> G19SRev primer
<400> 230
   gatgatgcta ccgtcagagt ccacaaagcc ggc 33
<210> 231
   <211> 33
   <212> DNA
   <213> artificial sequence
<220>
   <223> G19SFor primer
<400> 231
   gccggctttg tggactctga cggtagcatc atc 33
<210> 232
   <211> 84
   <212> DNA
   <213> artificial sequence
<220>
   <223> CCM2For primer
<400> 232
   aagcagagct ctctggctaa ctagagaacc cactgcttac tggcttatcg accatggcca 60
   ataccaaata taacaaagag ttcc 84
<210> 233
   <211> 38
   <212> DNA
   <213> artificial sequence
<220>
   <223> CCMRevBis primer
<400> 233
   ctgctctaga ttagtcggcc gccggggagg atttcttc 38
<210> 234
   <211> 163
   <212> PRT
   <213> Chlamydomonas reinhardtii
<400> 234
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Tyr Lys Phe Lys His Gln Leu Ser Leu Ala Phe Gln Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Arg Gly Ser Val Ser Asp Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 235
   <211> 167
   <212> PRT
   <213> artificial sequence
<220>
   <223> I-CreI N75 scaffold protein
<400> 235
   Met Ala Asn Thr Lys Tyr Asn Lys Glu Phe Leu Leu Tyr Leu Ala Gly
   1 5 10 15
   Phe Val Asp Gly Asp Gly Ser Ile Ile Ala Gln Ile Lys Pro Asn Gln
   20 25 30
   Ser Tyr Lys Phe Lys His Gln Leu Ser Leu Thr Phe Gln Val Thr Gln
   35 40 45
   Lys Thr Gln Arg Arg Trp Phe Leu Asp Lys Leu Val Asp Glu Ile Gly
   50 55 60
   Val Gly Tyr Val Arg Asp Arg Gly Ser Val Ser Asn Tyr Ile Leu Ser
   65 70 75 80
   Glu Ile Lys Pro Leu His Asn Phe Leu Thr Gln Leu Gln Pro Phe Leu
   85 90 95
   Lys Leu Lys Gln Lys Gln Ala Asn Leu Val Leu Lys Ile Ile Glu Gln
   100 105 110
   Leu Pro Ser Ala Lys Glu Ser Pro Asp Lys Phe Leu Glu Val Cys Thr
   115 120 125
   Trp Val Asp Gln Ile Ala Ala Leu Asn Asp Ser Lys Thr Arg Lys Thr
   130 135 140
   Thr Ser Glu Thr Val Arg Ala Val Leu Asp Ser Leu Ser Glu Lys Lys
   145 150 155 160
   Lys Ser Ser Pro Ala Ala Asp
   165
<210> 236
   <211> 163
   <212> PRT
   <213> artificial sequence
<220>
   <223> I-CreI variant cleaving RAG2.8.3
<400> 7
<210> 237
   <211> 163
   <212> PRT
   <213> artificial sequence
<220>
   <223> I-CreI variant cleaving RAG2.8.3
<400> 237
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Arg Lys Phe Lys His Gln Leu Gln Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Asn Gly Ser Val Ser Asn Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Val Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 238
   <211> 163
   <212> PRT
   <213> artificial sequence
<220>
   <223> I-CreI variant cleaving RAG2.8.4
<400> 238
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Asn Pro Asn Gln Ser
               20                  25                  30
   Ser Lys Phe Lys His Arg Leu Lys Leu Ala Phe Arg Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Tyr Tyr Asn Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 239
   <211> 163
   <212> PRT
   <213> artificial sequence
<220>
   <223> I-CreI variant cleaving RAG2.8.4
<400> 239
   Met Asn Thr Lys Tyr Asp Lys Glu Phe Leu Leu Tyr Leu Ala Gly Phe
   1               5                   10                  15
   Val Asp Gly Asp Gly Ser Ile Ile Ala Gln Ile Asn Pro Asn Gln Ser
               20                  25                  30
   Ser Lys Phe Lys His Arg Leu Lys Leu Ala Phe Arg Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Asn Tyr Thr Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Arg Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 240
   <211> 163
   <212> PRT
   <213> artificial sequence
<220>
   <223> I-creI variant cleaving RAG2.8.4
<400> 240
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Asn Pro Asn Gln Ser
   20 25 30
   Ser Lys Phe Lys His Arg Leu Lys Leu Ala Phe Arg Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Asn Tyr Thr Leu Ser Glu
   65 70 75 80
   Ile Lys Pro Leu His Asn Phe Leu Thr Gln Leu Gln Pro Phe Leu Glu
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 241
   <211> 163
   <212> PRT
   <213> artificial sequence
<220>
   <223> I-creI variant cleaving RAG2.8.4
<400> 241
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Gln Pro Asn Gln Ser
   20 25 30
   Ser Lys Phe Lys His Arg Leu Lys Leu Ala Phe Arg Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Asn Tyr Thr Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Gly Ser Pro Asp Lys Phe Leu Glu Val Cys Thr Trp
   115 120 125
   Val Asp Gln Ile Ala Ala Leu Asn Asp Ser Arg Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 242
   <211> 163
   <212> PRT
   <213> artificial sequence
<220>
   <223> I-CreI variant cleaving RAG2.8.4
<400> 242
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Asn Pro Asn Gln Ser
   20 25 30
   Ser Lys Phe Lys His Arg Leu Lys Leu Ala Phe Arg Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Asn Tyr Thr Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Ala Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 243
   <211> 163
   <212> PRT
   <213> artificial sequence
<220>
   <223> I-CreI variant cleaving RAG2.8.4
<400> 243
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Asn Pro Asn Gln Ser
   20 25 30
   Ser Lys Phe Lys His Arg Leu Lys Leu Ala Leu Arg Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Asn Tyr Thr Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro

<210> 244
   <211> 163
   <212> PRT
   <213> artificial sequence
<220>
   <223> I-CreI variant cleaving RAG2.8.4
<400> 244
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Asn Pro Asn Gln Ser
   20 25 30
   Ser Lys Phe Lys His Arg Leu Lys Leu Ala Phe Arg Val Thr Gln Lys
   35 40 45
   Ala Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Asn Leu Ser Glu
   65 70 75 80
   Ile Lys Pro Leu His Asn Leu Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 245
   <211> 163
   <212> PRT
   <213> artificial sequence
<220>
   <223> I-CreI variant cleaving RAG2.8.4
<400> 245
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Asn Pro Asn Gln Ser
               20                  25                  30
   Ser Lys Phe Lys His Arg Leu Lys Leu Ala Phe Arg Val Thr Gln Lys
           35                  40                  45
   Thr Gln Arg Arg Trp Leu Leu Asp Lys Leu Val Asp Glu Ile Gly Val
       50                  55                  60
   Gly Tyr Val Tyr Asp Ser Gly Ser Val Ser Asn Tyr Thr Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 246
   <211> 163
   <212> PRT
   <213> artificial sequence
<220>
   <223> I-CreI variant cleaving RAG2.8.4
<400> 246
   Met Asn Thr Asn Tyr Asn Lys Glu Phe Leu Leu Tyr Leu Ala Gly Phe
   1               5                   10                  15
   Val Asp Gly Asp Gly Ser Ile Ile Ala Gln Ile Asn Pro Asn Gln Ser
               20                  25                  30
   Ser Lys Phe Lys His Arg Leu Lys Leu Ala Phe Arg Val Thr Gln Lys
           35                  40                  45
   Thr Arg Arg Arg Trp Phe Leu Asp Lys Leu Val Asp Glu Ile Gly Val
   50 55 60
   Gly Tyr Val Tyr Asp Ser Gly Ser Val Ser Asn Tyr Thr Leu Ser Glu
   65 70 75 80
   Ile Lys Pro Leu His Asn Leu Leu Thr Gln Leu Gln Pro Phe Leu Arg
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 247
   <211> 163
   <212> PRT
   <213> artificial sequence
<220>
   <223> I-creI variant cleaving RAG2.8.4
<400> 247
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Asn Pro Asn Gln Ser
   20 25 30
   Ser Lys Phe Lys His Arg Leu Lys Leu Ala Leu Arg Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Asn Tyr Thr Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Val Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 248
   <211> 163
   <212> PRT
   <213> artificial sequence
<220>
   <223> I-CreI variant cleaving RAG1.10.2
<400> 248
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Arg Gln Ser
   20 25 30
   Asn Lys Phe Lys His Gln Leu Ser Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Lys Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Gly Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 249
   <211> 163
   <212> PRT
   <213> artificial sequence
<220>
   <223> I-CreI variant cleaving RAG1.10.2
<400> 249
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Arg Gln Ser
               20                  25                  30
   Asn Lys Phe Lys His Gln Leu Ser Leu Ala Phe Ala Val Thr Gln Lys
           35                  40                  45
   Thr Gln Arg Arg Trp Phe Leu Asp Lys Leu Val Asp Glu Ile Gly Val
       50                  55                  60
   Gly Tyr Val Tyr Asp Ser Gly Ser Val Ser Tyr Tyr Lys Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Arg Ile Ile Trp Arg Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Gly Ser Leu Ser Glu Lys Lys Lys
   145                 150                 155                 160
   Ser Ser Pro
<210> 250
   <211> 163
   <212> PRT
   <213> artificial sequence
<220>
   <223> I-CreI variant cleaving RAG1.10.2
<400> 250
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Arg Gln Ser
   20 25 30
   Asn Lys Phe Lys His Gln Leu Ser Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Asp Tyr Arg Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 251
   <211> 163
   <212> PRT
   <213> artificial sequence
<220>
   <223> I-creI variant cleaving RAG1.10.2
<400> 251
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Arg Gln Ser
   20 25 30
   Asn Thr Phe Lys His Gln Leu Ser Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Lys Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Lys Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 252
   <211> 163
   <212> PRT
   <213> artificial sequence
<220>
   <223> I-CreI variant cleaving RAG1.10.2
<400> 252
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Arg Gln Ser
   20 25 30
   Asn Lys Phe Lys His Gln Leu Ser Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Lys Leu Ser Glu
   65 70 75 80
   Ile Lys Pro Leu His Asn Phe Leu Thr Gln Leu Gln Pro Phe Leu Lys
   85 90 95
   Leu Lys Gln Arg Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 253
   <211> 163
   <212> PRT
   <213> artificial sequence
<220>
   <223> I-CreI variant cleaving RAG1.10.2
<400> 253
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Arg Gln Ser
   20 25 30
   Asn Lys Phe Lys His Gln Leu Ser Leu Ala Phe Ala Val Thr Gln Lys
   35 40 45
   Thr Gln Arg Arg Trp Phe Leu Asp Lys Leu Val Asp Glu Ile Gly Val
   50 55 60
   Gly Tyr Val Tyr Asp Ser Gly Ser Val Ser Tyr Tyr Lys Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Thr Val Leu Asp Ser Leu Ser Glu Lys Lys Lys
   145 150 155 160
   Ser Ser Pro